# EUROPÄISCHE PATENTANMELDUNG

(11) **EP 3 910 338 A2**
(43) Veröffentlichungstag der Anmeldung: **17.11.2021**
(21) Anmeldenummer: 21163757.4
(22) Anmeldetag: 10.02.2015
(51) Int. Cl.: G01N 33/564

(54) **MARKERSEQUENZEN ZUR DIAGNOSE UND STRATIFIZIERUNG VON SLE PATIENTEN**

(30) Priorität: 10.02.2014 EP 14154557; 22.07.2014 EP 14178090
(62) Teilanmeldung aus: 15709103.4
(71) Anmelder: Oncimmune Germany GmbH, 44227 Dortmund (DE)
(72) Erfinder: LÜKING, Angelika, 44892 Bochum (DE); SCHULZ-KNAPPE, Peter, 30966 Hemmingen (DE); THEEK, Carmen, 58313 Herdecke (DE); BUDDE, Petra, 44229 Dortmund (DE); TELAAR, Anna, 44225 Dortmund (DE)
(74) Vertreter: Boult Wade Tennant LLP

(57) **Zusammenfassung**

Die vorliegende Erfindung betrifft Verfahren zur Identifizierung von Markern für Systemischer Lupus erythematodes (SLE) und die mit Hilfe dieses Verfahrens identifizierten Marker, die zwischen SLE und anderen Autoimmunerkrankungen einerseits und zwischen verschiedenen SLE Subgruppen andererseits differenzieren können. Weiterhin betrifft die Erfindung Panel, Diagnostika und Test-Kits, die diese Marker umfassen sowie deren Verwendung und Anwendung, beispielsweise zur Diagnose, Prognose und Therapiesteuerung bei SLE. Ferner Verfahren zum Screenen und zur Validierung von Wirkstoffen für die Anwendung bei SLE Subgruppen.

## Beschreibung

Die vorliegende Erfindung betrifft Verfahren zur Identifizierung von Markern für Systemischer Lupus erythematodes (SLE) und die mit Hilfe dieses Verfahrens identifizierten Marker, die zwischen SLE und anderen Autoimmunerkrankungen einerseits und zwischen verschiedenen SLE Subgruppen andererseits differenzieren können. Weiterhin betrifft die Erfindung Panel von Markern für SLE, Diagnostika und Test Kits für SLE, die diese Marker umfassen sowie deren Verwendung und Anwendung, beispielsweise zur Diagnose, Prognose und Therapiesteuerung bei SLE. Ferner Verfahren zum Screenen und zur Validierung von Wirkstoffen für die Anwendung bei SLE Subgruppen.

Der Systemische Lupus erythematodes (SLE) auch Schmetterlingsflechte, ist eine seltene Autoimmunerkrankung. Beim Lupus erythematodes ist das körpereigene Immunsystem fehlreguliert. Es richtet sich nicht nur gegen Bakterien, Viren und Krebszellen, sondern auch gegen gesunde körpereigene Zellen. Dadurch werden Organe und Organsysteme, z. B. die Haut, geschädigt.

In der klinischen Praxis wird SLE aufgrund einer Kombination aus klinischen und immunologischen Parametern diagnostiziert. Dabei spielen antinukleäre Autoantikörper (ANA) und anti-Doppelstrang DNA (anti-dsDNA) Autoantikörper eine wichtige Rolle. Jedoch ist der ANA-Test nicht spezifisch für SLE, da auch andere Autoimmunerkrankungen und bis zu 20% der Gesunden positiv getestet werden. Daher wird zunehmend die Autoreaktivität gegen extrahierbare Nukleäre Antigene (extractable nuclear antigens, ENA) als rekombinante oder gereinigte Einzelantigene getestet, z.B. gegen Sm-Protein, U1-RNP, Rho52/SS-A und Ro60/SS-B. Diese Antigene als auch zugehörige Autoantikörper sind jedoch nicht ausreichend, um alle SLE Patienten insbesondere in einer frühen Phase der Erkrankung zweifelsfrei zu diagnostizieren. Beispielsweise sind anti-dsDNA Antikörper zwar hochspezifisch für SLE und können in etwa 70% der Patienten nachgewiesen werden. Allerdings korreliert der Titer der anti-dsDNA Antikörper mit der Krankheitsaktivität in einigen aber nicht in allen Patienten. Dies hat zur Folge, dass SLE häufig erst Monate oder Jahre nach Auftreten der ersten Symptome diagnostiziert wird. Ein weiteres Problem der derzeit angewendeten diagnostischen Verfahren ist, dass die Tauglichkeit der bisher untersuchten Autoantigene zur Diagnose von Organbeteiligungen und Komplikationen umstritten sind und zum Teil widersprüchliche Daten veröffentlicht wurden.

Daher besteht ein großer Bedarf, neue Marker zur Diagnose und Differentialdiagnose von SLE bereitzustellen.

Markersequenzen zur Diagnose von SLE sind in WO 2012/049225 A2 offenbart. Diese Markersequenzen wurden durch ein Verfahren gefunden, bei dem Serumproben von SLE Patienten und solche von Gesunden vergleichend untersucht und die Ergebnisse statistisch ausgewertet wurden. Die in WO 2012/049225 A2 beschriebenen Markersequenzen sind zur Diagnose von SLE geeignet jedoch nicht ausreichend im Hinblick auf die Abgrenzung von anderen Autoimmunerkrankungen und zur Identifizierung von SLE Subgruppen.

Es besteht deshalb weiterhin Bedarf an Markern für SLE, insbesondere zur Abgrenzung von SLE von anderen Autoimmunerkrankungen.

Diese Aufgabe wurde erfindungsgemäß dadurch gelöst, dass ein differentielles, eine Vielzahl von Schritten umfassendes Verfahren entwickelt wurde, bei dem Serumproben von Gesunden und Patienten mit verschiedenen Autoimmunerkrankungen vergleichend im Hinblick auf ihre Reaktivität mit einer Vielzahl von potentiellen Antigenen untersucht und diese Ergebnisse statistisch ausgewertet wurden. Die Auswahl der Serumproben und die Abfolge der Schritte ermöglichte überraschend die Identifizierung hochspezifischer Marker für SLE, die auch geeignet sind, SLE Subgruppen zu identifizieren und Komplikationen wie Lupus Nephritis zu diagnostizieren sowie eine Differentialdiagnose im Hinblick auf andere Autoimmunerkrankungen wie rheumatoide Arthritis (RA), Systemische Sklerodermie (SSc), Spondylitis ankylosans oder Morbus Bechterew (SPA) und auch im Hinblick auf Personen, die eine frühe/early RA mit einer Erkrankung von weniger als zwei Jahren aufweisen ("Patienten mit früher RA").

Gegenstand der vorliegenden Erfindung ist ein Verfahren zur Identifizierung von Markern für Systemischer Lupus erythematodes (SLE) umfassend die Schritte
a) Serumproben von SLE Patienten werden mit mehr als 5000 an (Luminex-)Beads gekoppelten Antigenen in Kontakt gebracht, die Bindung der einzelnen Antigene an Proteine, insbesondere Autoantikörper, im Serum der SLE Patienten durch Immunfluoreszenzassay gemessen und die mittlere Fluoreszenzintensität (MFI = median fluorescence intensity) für jedes einzelne Antigen bestimmt;
b) Serumproben von Patienten mit rheumatoider Arthritis (RA) werden mit denselben an (Luminex-)Beads gekoppelten Antigenen in Kontakt gebracht, die Bindung der einzelnen Antigene an Proteine, insbesondere Autoantikörper, im Serum der RA Patienten durch Immunfluoreszenzassay gemessen und daraus die mittlere Fluoreszenzintensität (MFI) für jedes einzelne Antigen bestimmt;
c) Serumproben von Gesunden werden mit denselben an (Luminex-) Beads gekoppelten Antigenen in Kontakt gebracht, die Bindung der einzelnen Antigene an Proteine, insbesondere Autoantikörper, im Serum von Gesunden durch Immunfluoreszenzassay gemessen und daraus die mittlere Fluoreszenzintensität (MFI) für jedes einzelne Antigen bestimmt;
d) die MFI Daten aus a), b) und c) werden statistisch mittels univariater Analyse ausgewertet und dadurch Markerkandidat Antigene identifiziert, mit denen SLE Patienten von RA Patienten und von Gesunden differenziert werden können;
e) Serumproben von Patienten mit früher RA werden mit den an (Luminex-)Beads gekoppelten Markerkandidat Antigenen, die in d) identifiziert werden, in Kontakt gebracht, die Bindung von Markerkandidat Antigenen an Proteine, insbesondere Autoantikörper, im Serum von Patienten mit früher RA durch Immunfluoreszenzassay gemessen und daraus die mittlere Fluoreszenzintensität (MFI) für jedes Markerkandidat Antigen bestimmt;
f) Serumproben von Patienten mit Systemischer Sklerodermie (SSc Patienten) werden mit den an (Luminex-)Beads gekoppelten Markerkandidat Antigenen, die in d) identifiziert wurden, in Kontakt gebracht, die Bindung von Markerkandidat Antigenen an Proteine, insbesondere Autoantikörper, im Serum von SSc Patienten durch Immunfluoreszenzassay gemessen und daraus die mittlere Fluoreszenzintensität (MFI) für jedes Markerkandidat Antigen bestimmt;
g) Serumproben von Patienten mit Spondylitis ankylosans oder Morbus Bechterew (SPA Patienten) werden mit den an (Luminex-)Beads gekoppelten Markerkandidat Antigenen, die in d) identifiziert wurden, in Kontakt gebracht, die Bindung von Markerkandidat Antigenen an Proteine, insbesondere Autoantikörper, im Serum von SPA Patienten durch Immunfluoreszenzassay gemessen und daraus die mittlere Fluoreszenzintensität (MFI) für jedes Markerkandidat Antigen bestimmt;
h) die MFI Daten aus e), f) und g) werden statistisch mittels univariater Analyse ausgewertet und wenn ein Schwellenwert von 3 Standardabweichungen über dem Mittelwert der Gesundproben nicht erreicht wird, wird dadurch ein spezifischer Marker für SLE identifiziert, wobei die Marker ausgewählt werden aus den Sequenzen SEQ ID No. 1 bis 1587, Homologen der Sequenzen SEQ ID No. 1 bis 1587 mit mindestens 95 % Homologie, Teilsequenzen von SEQ ID No. 1 bis 1587, Teilsequenzen der Homologen von SEQ ID No. 1 bis 1587 mit mindestens 95 % Homologie.

Der Begriff "Systemischer Lupus erythematodes (SLE)" betrifft eine systemische Autoimmunerkrankung aus der Gruppe der Kollagenosen. Besonders charakteristisch für SLE (systemischer Lupus erythematodes) ist das so genannte Schmetterlingserythem. Die Diagnosekriterien für SLE sind:
1. Schmetterlingserythem, 2. Diskoide Hautveränderungen, 3. Lichtempfindlichkeit, 4. Schleimhautul zera (im Allgemeinen schmerzlos), 5. Arthritis in mindestens zwei Gelenken, 6. Serositis (Pleuritis od. Perikarditis), 7. Nierenbeteiligung (Proteinurie >0,5 g/d od. Zylinder), 8. ZNS-Beteiligung (Krämpfe od. Psychose), 9. Hämatologische Befunde (hämolyt. Anämie, Leukood. Thrombopenie), 10. Immunologische Befunde (Anti-dsDNA-Antikörper, Anti-Sm-Antikörper, AntikardiolipinAntikörper), 11. antinukleäre Antikörper ohne Einnahme Lupus erythematodes auslösender Medikamente. Auswertung: Bei vier (drei) positiven Befunden gilt die Diagnose als sicher (wahrscheinlich) (Definition z.B. nach Pschyrembel, de Gruyter, 261. Auflage (2007), Berlin).

Eine Ausführungsform der Erfindung betrifft Verfahren zur Identifizierung von Markern für SLE, die zur Diagnose und Differentialdiagnose von SLE geeignet sind, insbesondere zur Abgrenzung von anderen Autoimmunerkrankungen, vorzugsweise zur Abgrenzung von anderen rheumatischen Erkrankungen, besonders bevorzugt zur Abgrenzung von RA, SSc, SPA. Diese Marker sind auch zur Abgrenzung von Patienten mit früher RA geeignet. Diese erfindungsgemäßen Marker für SLE sind Gegenstand der Gruppe 1 der Antigene in Tabelle 2, die zur Diagnose von SLE verwendet werden können. Für die Generierung dieser Marker werden basierend auf den univariaten Ergebnissen Markerkandidat Antigene ausgewählt, die einen adjustierten p-Wert für den nichtparametrischen Mittelwertvergleich zwischen Gruppen von <0,05 aufweisen, gleichzeitig einen Fold Change von >1,5 und zusätzlich eine aus der ROC-Analyse resultierende AUC von >0,75. Außerdem werden die ENA-4 Antigene ausgewählt. Für diesen Pool ausgewählter Markerkandidaten Antigene wird vorzugsweise im Rahmen einer getesteten Kreuzvalidierung auch ein L1-penalisiertes logistisches Regressionsmodell aufgestellt. Markerkandidat Antigene, die im Rahmen der Modellbildung nicht berücksichtigt werden, werden aus der weiteren Betrachtung entfernt. Dadurch werden die Marker für SLE ausgewählt aus den Sequenzen (Gruppe 1)
SEQ ID No. 1 bis 24, 134, 168, 214, 368 bis 370 SEQ ID No. 530 bis 553, 663, 697, 743, 897 bis 899 und SEQ ID No. 1059 bis 1082, 1192, 1226, 1272, 1426 bis 1428,
Homologen von SEQ ID No. 1 bis 24, 134, 168, 214, 368 bis 370 SEQ ID No. 530 bis 553, 663, 697, 743, 897 bis 899 und SEQ ID No. 1059 bis 1082, 1192, 1226, 1272, 1426 bis 1428 mit mindestens 95 % Homologie, Teilsequenzen von SEQ ID No. 1 bis 24, 134, 168, 214, 368 bis 370 SEQ ID No. 530 bis 553, 663, 697, 743, 897 bis 899 und SEQ ID No. 1059 bis 1082, 1192, 1226, 1272, 1426 bis 1428 und Teilsequenzen der Homologen von SEQ ID No. 1 bis 24, 134, 168, 214, 368 bis 370 SEQ ID No. 530 bis 553, 663, 697, 743, 897 bis 899 und SEQ ID No. 1059 bis 1082, 1192, 1226, 1272, 1426 bis 1428 mit mindestens 95 % Homologie erhalten.

Eine andere Ausführungsform betrifft Verfahren zur Identifizierung von Markern für die Subgruppe der SLE Patienten mit der Komplikation *Lupus Nephritis* umfassend den Vergleich der Autoantikörperprofile von SLE Patienten mit Lupus Nephritis mit denen von SLE Patienten ohne Lupus Nephritis. Marker, die mittels dieser Ausführungsform des Verfahrens gefunden werden, sind beispielsweise in Tabelle 2, in den Gruppen 2 und Gruppe 5 genannt. Dies sind beispielsweise Verfahren, wobei die Marker für die Subgruppe der SLE Patienten mit der Komplikation Lupus Nephritis ausgewählt werden aus den Sequenzen
SEQ ID No. 25 bis 54, 215, 216, 217, 218, 228, 233, 241, 245, 247, 249, 258, 288, 289, 301, 309, 315, 316, 324, 330, 331, 337, 339, 348, 350, 359, 362, 363,
SEQ ID No. 554 bis 583, 744, 745, 746, 747, 757, 762, 770, 774, 776, 778, 787, 817, 818, 830, 838, 844, 845, 853, 859, 860, 866, 868, 877, 879, 888, 891, 892 und
SEQ ID No. 1083 bis 1112, 1273, 1274, 1275, 1276, 1286, 1291, 1299, 1303, 1305, 1307, 1316, 1346, 1347, 1359, 1367, 1373, 1374, 1382, 1388, 1389, 1395, 1397, 1406, 1408, 1417, 1420, 1421,
Homologen von SEQ ID No. SEQ ID No. 25 bis 54, 215, 216, 217, 218, 228, 233, 241, 245, 247, 249, 258, 288, 289, 301, 309, 315, 316, 324, 330, 331, 337, 339, 348, 350, 359, 362, 363, SEQ ID No. 554 bis 583, 744, 745, 746, 747, 757, 762, 770, 774, 776, 778, 787, 817, 818, 830, 838, 844, 845, 853, 859, 860, 866, 868, 877, 879, 888, 891, 892 und SEQ ID No. 1083 bis 1112, 1273, 1274, 1275, 1276, 1286, 1291, 1299, 1303, 1305, 1307, 1316, 1346, 1347, 1359, 1367, 1373, 1374, 1382, 1388, 1389, 1395, 1397, 1406, 1408, 1417, 1420, 1421 mit mindestens 95 % Homologie, Teilsequenzen von SEQ ID No. 25 bis 54, 215, 216, 217, 218, 228, 233, 241, 245, 247, 249, 258, 288, 289, 301, 309, 315, 316, 324, 330, 331, 337, 339, 348, 350, 359, 362, 363, SEQ ID No. 554 bis 583, 744, 745, 746, 747, 757, 762, 770, 774, 776, 778, 787, 817, 818, 830, 838, 844, 845, 853, 859, 860, 866, 868, 877, 879, 888, 891, 892 und SEQ ID No. 1083 bis 1112, 1273, 1274, 1275, 1276, 1286, 1291, 1299, 1303, 1305, 1307, 1316, 1346, 1347, 1359, 1367, 1373, 1374, 1382, 1388, 1389, 1395, 1397, 1406, 1408, 1417, 1420, 1421 und Teilsequenzen der Homologen von SEQ ID No. SEQ ID No. 25 bis 54, 215, 216, 217, 218, 228, 233, 241, 245, 247, 249, 258, 288, 289, 301, 309, 315, 316, 324, 330, 331, 337, 339, 348, 350, 359, 362, 363, SEQ ID No. 554 bis 583, 744, 745, 746, 747, 757, 762, 770, 774, 776, 778, 787, 817, 818, 830, 838, 844, 845, 853, 859, 860, 866, 868, 877, 879, 888, 891, 892 und SEQ ID No. 1083 bis 1112, 1273, 1274, 1275, 1276, 1286, 1291, 1299, 1303, 1305, 1307, 1316, 1346, 1347, 1359, 1367, 1373, 1374, 1382, 1388, 1389, 1395, 1397, 1406, 1408, 1417, 1420, 1421 mit mindestens 95 % Homologie.

Eine weitere Ausführungsform betrifft Verfahren, die die statistische Auswertung mittels eines L1-Penalisierten logistischen Regressionsmodels mit fünffacher Kreuzvalidierung und zwanzigfacher Wiederholung und Auswahl der Marker, die mit einer Frequenz von 50 % oder mehr auftreten umfassen. Marker, die mittels dieser Ausführungsform des Verfahrens identifiziert werden können, sind beispielsweise in Tabelle 2, Gruppe 2 genannt. Dies sind beispielsweise Verfahren, wobei die Marker für die Subgruppe der SLE Patienten mit der Komplikation Lupus Nephritis ausgewählt werden aus den Sequenzen
SEQ ID No. 25 bis 54, SEQ ID No. 554 bis 583 und SEQ ID No. 1083 bis 1112,
Homologen von 25 bis 54, SEQ ID No. 554 bis 583 und SEQ ID No. 1083 bis 1112 mit mindestens 95 % Homologie, Teilsequenzen von SEQ ID No. 25 bis 54, SEQ ID No. 554 bis 583 und SEQ ID No. 1083 bis 1112 und Teilsequenzen der Homologen von SEQ ID No. 225 bis 54, SEQ ID No. 554 bis 583 und SEQ ID No. 1083 bis 1112 mit mindestens 95 % Homologie.

In einer weiteren Ausführungsform des Verfahrens werden Marker für definierte Subgruppen von SLE Patienten dadurch identifiziert, dass die Sequenzen SEQ ID No. 1 bis 529 (Klonsequenzen) durch Berechnung des Spearman Rangkorrelationskoeffizienten für den jeweiligen Marker mit einer der Sequenzen SEQ ID No. 1 bis 529 korreliert werden. Auf diese Weise können mit dem erfindungsgemäßen Verfahren beispielsweise die Marker der Gruppen 1, 2 und 3 in Tabelle 2 identifiziert werden. Dies sind beispielsweise solche Verfahren, wobei die Marker, die eine Korrelation der Reaktivitäten in SLE Patienten miteinander zeigen ausgewählt werden aus den Sequenzen (Gruppe 3)
SEQ ID No. 55 bis 111, SEQ ID No. 584 bis 1007 und SEQ ID No. 1113 bis 1169,
Homologen von SEQ ID No. 55 bis 111, SEQ ID No. 584 bis 1007 und SEQ ID No. 1113 bis 1169 mit mindestens 95 % Homologie, Teilsequenzen von SEQ ID SEQ ID No. 55 bis 111, SEQ ID No. 584 bis 1007 und SEQ ID No. 1113 bis 1169 und Teilsequenzen der Homologen von SEQ ID No. 55 bis 111, SEQ ID No. 584 bis 1007 und SEQ ID No. 1113 bis 1169 mit mindestens 95 % Homologie und aus den Sequenzen (Gruppe 2)
SEQ ID No. 25 bis 54, SEQ ID No. 554 bis 583 und SEQ ID No. 1083 bis 1112,
Homologen von SEQ ID No. 25 bis 54, SEQ ID No. 554 bis 583 und SEQ ID No. 1083 bis 1112 mit mindestens 95 % Homologie, Teilsequenzen von SEQ ID No. 25 bis 54, SEQ ID No. 554 bis 583 und SEQ ID No. 1083 bis 1112 und Teilsequenzen der Homologen von SEQ ID No. 25 bis 54, SEQ ID No. 554 bis 583 und SEQ ID No. 1083 bis 1112 mit mindestens 95 % Homologie und aus den Sequenzen (Gruppe 1)
SEQ ID No. 1 bis 24, 134, 168, 214, 368 bis 370 SEQ ID No. 530 bis 553, 663, 697, 743, 897 bis 899 und SEQ ID No. 1059 bis 1082, 1192, 1226, 1272, 1426 bis 1428,
Homologen von SEQ ID No. 1 bis 24, 134, 168, 214, 368 bis 370 SEQ ID No. 530 bis 553, 663, 697, 743, 897 bis 899 und SEQ ID No. 1059 bis 1082, 1192, 1226, 1272, 1426 bis 1428 mit mindestens 95 % Homologie, Teilsequenzen von SEQ ID No. 1 bis 24, 134, 168, 214, 368 bis 370 SEQ ID No. 530 bis 553, 663, 697, 743, 897 bis 899 und SEQ ID No. 1059 bis 1082, 1192, 1226, 1272, 1426 bis 1428 und Teilsequenzen der Homologen von SEQ ID No. 1 bis 24, 134, 168, 214, 368 bis 370 SEQ ID No. 530 bis 553, 663, 697, 743, 897 bis 899 und SEQ ID No. 1059 bis 1082, 1192, 1226, 1272, 1426 bis 1428 mit mindestens 95 % Homologie.

Eine Ausführungsform der Erfindung betrifft Verfahren zur Identifizierung von Markern für die Subgruppe der ENA-4 negativen SLE Patienten. Diese Ausführungsform des Verfahrens umfasst beispielsweise das Testen der Serumproben von SLE Patienten auf die Abwesenheit von Autoantikörpern gegen die extrahierbaren nukleären Antigene Sm-Protein, U1-RNP, Rho52/SS-A und Ro60/SS-B. Dadurch können beispielsweise die Marker der Gruppe 4, Tabelle 2 identifiziert werden. Dies sind beispielsweise Verfahren, wobei die Marker für ENA-4 negative SLE Patienten ausgewählt werden aus den Sequenzen (Gruppe 4)
SEQ ID No. 112 bis 214 und 279, SEQ ID No. 641 bis 743 und 808 und SEQ ID No. 1170 bis 1272 und 1337,
Homologen von SEQ ID No. 112 bis 214 und 279, SEQ ID No. 641 bis 743 und 808 und SEQ ID No. 1170 bis 1272 und 1337 mit mindestens 95 % Homologie, Teilsequenzen von SEQ ID No. 112 bis 214 und 279, SEQ ID No. 641 bis 743 und 808 und SEQ ID No. 1170 bis 1272 und 1337 und Teilsequenzen der Homologen von SEQ ID No. 112 bis 214 und 279, SEQ ID No. 641 bis 743 und 808 und SEQ ID No. 1170 bis 1272 und 1337 mit mindestens 95 % Homologie.

Eine Ausführungsform der Erfindung betrifft Verfahren umfassend die Auswahl von Markern, die einen adjustierten p-Wert für den nichtparametrischen Mittelwertvergleich zwischen Gruppen von kleiner 0,05 aufweisen, gleichzeitig einen Fold Change von größer 1,5 und eine aus der ROC-Analyse resultierende AUC von größer 0,75 haben. Dadurch können beispielsweise die Marker der Gruppen 1, 4, 6 identifiziert werden. Die entsprechenden Berechnungen für Panel von Markern sind in Tabelle 5 angegeben, wobei die entsprechende Markerzusammensetzung in den Paneln (Anordnungen) Tabelle 4 entnommen werden kann. Dies sind beispielsweise Verfahren, wobei die Marker ausgewählt werden aus den Sequenzen (Gruppe 6)
SEQ ID No. 219 bis 227, 229 bis 232, 234 bis 240, 242, 243, 244, 246, 248, 250 bis 257, 259 bis 278, 280 bis 287, 290 bis 300, 302 bis 308, 310 bis 314, 317 bis 323, 325 bis 329, 332 bis 336, 338, 340 bis 347, 349, 351 bis 358, 360, 361, 364 bis 367,
SEQ ID No. 748 bis 756, 758 bis 761, 763 bis 769, 771, 772, 773, 775, 777, 779 bis 786, 788 bis 807, 809 bis 816, 819 bis 829, 831 bis 837, 839 bis 843, 846 bis 852, 853 bis 857, 861 bis 865, 867, 869 bis 876, 878, 880 bis 887, 889, 890, 893 bis 896 und
SEQ ID No. 1277 bis 1285, 1287 bis 1290, 1292 bis 1298, 1300, 1301, 1302, 1304, 1306, 1308 bis 1315, 1317 bis 1336, 1338 bis 1345, 1348 bis 1358, 1360 bis 1366, 1368 bis 1372, 1375 bis 1381, 1382 bis 1386, 1390 bis 1394, 1396, 1398 bis 1405, 1407, 1409 bis 1416, 1418, 1420, 1422 bis 1425,
Homologen von SEQ ID No. 219 bis 227, 229 bis 232, 234 bis 240, 242, 243, 244, 246, 248, 250 bis 257, 259 bis 278, 280 bis 287, 290 bis 300, 302 bis 308, 310 bis 314, 317 bis 323, 325 bis 329, 332 bis 336, 338, 340 bis 347, 349, 351 bis 358, 360, 361, 364 bis 367, SEQ ID No. 748 bis 756, 758 bis 761, 763 bis 769, 771, 772, 773, 775, 777, 779 bis 786, 788 bis 807, 809 bis 816, 819 bis 829, 831 bis 837, 839 bis 843, 846 bis 852, 853 bis 857, 861 bis 865, 867, 869 bis 876, 878, 880 bis 887, 889, 890, 893 bis 896, SEQ ID No. 1277 bis 1285, 1287 bis 1290, 1292 bis 1298, 1300, 1301, 1302, 1304, 1306, 1308 bis 1315, 1317 bis 1336, 1338 bis 1345, 1348 bis 1358, 1360 bis 1366, 1368 bis 1372, 1375 bis 1381, 1382 bis 1386, 1390 bis 1394, 1396, 1398 bis 1405, 1407, 1409 bis 1416, 1418, 1420, 1422 bis 1425 mit mindestens 95 % Homologie, Teilsequenzen von SEQ ID No. 219 bis 227, 229 bis 232, 234 bis 240, 242, 243, 244, 246, 248, 250 bis 257, 259 bis 278, 280 bis 287, 290 bis 300, 302 bis 308, 310 bis 314, 317 bis 323, 325 bis 329, 332 bis 336, 338, 340 bis 347, 349, 351 bis 358, 360, 361, 364 bis 367, SEQ ID No. 748 bis 756, 758 bis 761, 763 bis 769, 771, 772, 773, 775, 777, 779 bis 786, 788 bis 807, 809 bis 816, 819 bis 829, 831 bis 837, 839 bis 843, 846 bis 852, 853 bis 857, 861 bis 865, 867, 869 bis 876, 878, 880 bis 887, 889, 890, 893 bis 896, SEQ ID No. 1277 bis 1285, 1287 bis 1290, 1292 bis 1298, 1300, 1301, 1302, 1304, 1306, 1308 bis 1315, 1317 bis 1336, 1338 bis 1345, 1348 bis 1358, 1360 bis 1366, 1368 bis 1372, 1375 bis 1381, 1382 bis 1386, 1390 bis 1394, 1396, 1398 bis 1405, 1407, 1409 bis 1416, 1418, 1420, 1422 bis 1425 und Teilsequenzen der Homologen von SEQ ID No. 219 bis 227, 229 bis 232, 234 bis 240, 242, 243, 244, 246, 248, 250 bis 257, 259 bis 278, 280 bis 287, 290 bis 300, 302 bis 308, 310 bis 314, 317 bis 323, 325 bis 329, 332 bis 336, 338, 340 bis 347, 349, 351 bis 358, 360, 361, 364 bis 367, SEQ ID No. 748 bis 756, 758 bis 761, 763 bis 769, 771, 772, 773, 775, 777, 779 bis 786, 788 bis 807, 809 bis 816, 819 bis 829, 831 bis 837, 839 bis 843, 846 bis 852, 853 bis 857, 861 bis 865, 867, 869 bis 876, 878, 880 bis 887, 889, 890, 893 bis 896, SEQ ID No. 1277 bis 1285, 1287 bis 1290, 1292 bis 1298, 1300, 1301, 1302, 1304, 1306, 1308 bis 1315, 1317 bis 1336, 1338 bis 1345, 1348 bis 1358, 1360 bis 1366, 1368 bis 1372, 1375 bis 1381, 1382 bis 1386, 1390 bis 1394, 1396, 1398 bis 1405, 1407, 1409 bis 1416, 1418, 1420, 1422 bis 1425 mit mindestens 95 % Homologie.

Gruppe 7 in Tabelle 2 enthält weitere 85 statistisch signifikante Antigene aus den erfindungsgemäßen Verfahren; Marker ausgewählt aus den Sequenzen SEQ ID No. 368 bis 452, SEQ ID No. 897 bis 981, SEQ ID No. 1426 bis 1510, Homologen von SEQ ID No. 368 bis 452, SEQ ID No. 897 bis 981, SEQ ID No. 1426 bis 1510 mit mindestens 95 % Homologie, Teilsequenzen von SEQ ID No. 368 bis 452, SEQ ID No. 897 bis 981, SEQ ID No. 1426 bis 1510 und Teilsequenzen der Homologen von SEQ ID No. 368 bis 452, SEQ ID No. 897 bis 981, SEQ ID No. 1426 bis 1510 mit mindestens 95 % Homologie, die für die Diagnose und Differentialdiagnose von SLE gegenüber Gesund und anderen Autoimmunerkrankungen herangezogen werden können. Antigene aus der Gruppe 7 wurden ebenfalls für die Berechnung von Biomarkerkombinationen verwendet.

Gruppe 8 besteht aus weiteren statistisch signifikanten Antigenen aus den erfindungsgemäßen Verfahren; Marker ausgewählt aus den Sequenzen SEQ ID No. 453 bis 529, SEQ ID No. 982 bis 1058, SEQ ID No. 1511 bis 1587, Homologen von SEQ ID No. 453 bis 529, SEQ ID No. 982 bis 1058, SEQ ID No. 1511 bis 1587 mit mindestens 95 % Homologie, Teilsequenzen von SEQ ID No. 453 bis 529, SEQ ID No. 982 bis 1058, SEQ ID No. 1511 bis 1587 und Teilsequenzen der Homologen von SEQ ID No. 453 bis 529, SEQ ID No. 982 bis 1058, SEQ ID No. 1511 bis 1587 mit mindestens 95 % Homologie, für die Autoantikörper in SLE-Patienten detektiert und identifiziert wurden.

Gegenstand der Erfindung sind auch die einzelnen mit dem erfindungsgemäßen Verfahren identifizierten Marker für SLE. Gegenstand des Verfahrens sind Marker für SLE ausgewählt aus den Sequenzen SEQ ID No. 1 bis 1587, Homologen der Sequenzen SEQ ID No. 1 bis 1587 mit mindestens 95 % Homologie, Teilsequenzen von SEQ ID No. 1 bis 1587 und Teilsequenzen der Homologen von SEQ ID No. 1 bis 1587 mit mindestens 95 % Homologie. Gegenstand des Verfahrens sind die Marker der Gruppen 1, 2, 3, 4, 5, 6, 7, 8 in Tabelle 2 und wobei die jeweiligen Gruppen die Marker der in Tabelle 2 aufgeführten Klonsequenzen, die korrespondierenden RNA Sequenzen, die korrespondierenden Proteinsequenzen, die betreffenden Homologen mit einer Homologie von mindestens 95 % und die betreffenden Teilsequenzen umfassen. Gegenstand der Erfindung ist ein Marker für SLE ausgewählt aus den Sequenzen SEQ ID No. 1 bis 529 (Klonsequenzen), SEQ ID No. 530 bis 1058 (RNA Sequenzen), SEQ ID No. 1059 bis 1587 (Proteinsequenzen). Die erfindungsgemäßen Marker und die dazugehörigen Nukleinsäuresequenzen sind Gegenstand der Tabelle 2 (SEQ ID No. der betreffenden Klonsequenzen ist angegeben) und können durch den jeweils zitierten Datenbankeintrag, z.B. unter http://www.ncbi.nlm.nih.gov/, mittels ihrer GeneID (Tabelle 2) eindeutig identifiziert werden. Die Sequenzen SEQ ID No. 1 - 1587 sind im anliegenden Sequenzprotokoll angegeben, wobei SEQ ID No. 1 - 529 Klonsequenzen (cDNA), SEQ ID No. 530 - 1058 RNA Sequenzen und SEQ ID No. 1059 - 1587 Proteinsequenzen sind.

Gegenstand der Erfindung sind ebenfalls die durch die Sequenzen SEQ ID No. 1 bis 1058 kodierten Proteine, die durch Homologe der Sequenzen SEQ ID No. 1 bis 1058 mit mindestens 95 % Homologie zu den Sequenzen SEQ ID No. 1 bis 1058 kodierten Proteine, die durch Teilsequenzen von SEQ ID No. 1 bis 1058 kodierten Proteine, die durch Homologe der Teilsequenzen von SEQ ID No. 1 bis 1058 mit mindestens 95 % Homologie in den Teilsequenzen kodierten Proteine. In einer bevorzugten Ausführungsform sind dies die Proteine SEQ ID No. 1059 bis 1587, Homologe der Proteine mit der Sequenz SEQ ID No. SEQ ID No. 1059 bis 1587 mit mindestens 95 % Homologie, Teilsequenzen von SEQ ID No. 1059 bis 1587, Homologe der Teilsequenzen von SEQ ID No. SEQ ID No. 1059 bis 1587 mit mindestens 95 % Homologie.

Gegenstand der Erfindung ist auch ein Panel von Markern (auch Anordnung von Markern genannt) umfassend mindestens zwei verschiedene Marker für SLE, die unabhängig voneinander ausgewählt aus den Sequenzen SEQ ID No. 1 bis 1587, Homologen der Sequenzen SEQ ID No. 1 bis 1587 mit mindestens 95 % Homologie, Teilsequenzen von SEQ ID No. 1 bis 1587 und Teilsequenzen der Homologen von SEQ ID No. 1 bis 1587 mit mindestens 95 % Homologie. Ein Panel von Markern für SLE kann 2 bis 20 oder mehr, beispielsweise 3, 4, 5, 6, 7, 8, 9, 10, 11, 12, 13, 14, 15, 16, 17, 18, 19, 20, 25, 30, 40, 50, 100 oder mehr verschiedene Marker für SLE und gegebenenfalls weitere Marker umfassen, wobei die Marker von SLE unabhängig voneinander ausgewählt aus den Sequenzen SEQ ID No. 1 bis 1587, Homologen der Sequenzen SEQ ID No. 1 bis 1587 mit mindestens 95 % Homologie, Teilsequenzen von SEQ ID No. 1 bis 1587 und Teilsequenzen der Homologen von SEQ ID No. 1 bis 1587 mit mindestens 95 % Homologie sowie die durch die Sequenzen kodierten Proteine.

Aufgrund der hohen klinischen und serologischen Heterogenität der SLE Erkrankung ist es schwierig, mit nur einem Biomarker SLE eindeutig zu diagnostizieren. Daher ist es oft erforderlich, möglichst unkorrelierte Autoantigene zu sogenannten Paneln von Markern (Biomarker Panel für SLE) zu kombinieren. Beispielsweise im Rahmen der individualisierten Medizin können entsprechende Panel von Markern für SLE für einzelne Patienten oder Patientengruppen individuell für den betreffenden SLE Subtyp (Subgruppe) zusammengestellt werden. Deshalb ist es auch notwendig, eine Vielzahl von potentiellen Markern für SLE zur Verfügung zu haben, um entsprechende Subgruppen bzw. Subtypen spezifische Marker für SLE für den jeweils individuellen Fall auszuwählen. Ein entsprechendes Panel kann beispielsweise in Form einer Anordnung, eines Arrays oder auch eines oder mehrerer Beads, vorzugsweise Luminex Beads, ausgestaltet sein. Gegenstand der Erfindung ist somit eine Anordnung umfassend eine oder mehrere erfindungsgemäße Marker, ein Proteinarray umfassend eine oder mehrere erfindungsgemäße Marker, ein Bead (Kügelchen oder Plättchen) umfassend eine oder mehrere erfindungsgemäße Marker. Beispiele für SLE Panels (SLE Anordnungen) sind in Tabelle 4 gegeben.

Gegenstand der Erfindung ist auch ein Diagnostikum oder ein Test-Kit umfassend mindestens einen Marker für SLE ausgewählt aus den Sequenzen SEQ ID No. 1 bis 1587, Homologen der Sequenzen SEQ ID No. 1 bis 1587 mit mindestens 95 % Homologie, Teilsequenzen von SEQ ID No. 1 bis 1587 und Teilsequenzen der Homologen von SEQ ID No. 1 bis 1587 mit mindestens 95 % Homologie sowie die durch die Sequenzen kodierten Proteine. Ein entsprechendes Diagnostikum oder ein entsprechender Test-Kit können auch ein Panel von Markern für SLE umfassen und ggf. weitere Hilfs- und Zusatzstoffe.

Gegenstand der Erfindung ist auch die Verwendung eines oder mehrerer Marker für SLE ausgewählt aus den Sequenzen SEQ ID No. 1 bis 1587, Homologen der Sequenzen SEQ ID No. 1 bis 1587 mit mindestens 95 % Homologie, Teilsequenzen von SEQ ID No. 1 bis 1587 und Teilsequenzen der Homologen von SEQ ID No. 1 bis 1587 mit mindestens 95 % Homologie sowie die durch die Sequenzen kodierten Proteine, eines Marker Panels für SLE, eines Diagnostikums oder eines Test-Kits zur Identifizierung von Subgruppen von SLE Patienten, zur Diagnose von SLE, zur Differentialdiagnose (d.h. zur Abgrenzung von anderen Autoimmunerkrankungen oder anderen rheumatischen Erkrankungen), zur Prognose bei SLE, zur Therapiesteuerung bei SLE, zur Wirkstoffauswahl bei SLE, zur Therapieüberwachung bei SLE, zur Nachsorge bei SLE.

Gegenstand der Erfindung ist auch die Verwendung eines oder mehrerer der Marker für SLE ausgewählt aus den Sequenzen SEQ ID No. 1 bis 1587, Homologen der Sequenzen SEQ ID No. 1 bis 1587 mit mindestens 95 % Homologie, Teilsequenzen von SEQ ID No. 1 bis 1587 und Teilsequenzen der Homologen von SEQ ID No. 1 bis 1587 mit mindestens 95 % Homologie sowie die durch die Sequenzen kodierten Proteine zur Unterscheidung von SLE von RA und / oder anderen Autoimmunerkrankungen, beispielsweise von SSc und/oder SPA und/oder RA und/oder früher RA.

Gegenstand der Erfindung ist auch die Verwendung eines oder mehrerer Marker für SLE ausgewählt aus den Sequenzen SEQ ID No. 112 bis 214 und 279, SEQ ID No. 641 bis 743 und 808 und SEQ ID No. 1170 bis 1272 und 1337, Homologen von SEQ ID No. 112 bis 214 und 279, SEQ ID No. 641 bis 743 und 808 und SEQ ID No. 1170 bis 1272 und 1337 mit mindestens 95 % Homologie, Teilsequenzen von SEQ ID No. 112 bis 214 und 279, SEQ ID No. 641 bis 743 und 808 und SEQ ID No. 1170 bis 1272 und 1337 und Teilsequenzen der Homologen von SEQ ID No. 112 bis 214 und 279, SEQ ID No. 641 bis 743 und 808 und SEQ ID No. 1170 bis 1272 und 1337 mit mindestens 95 % Homologie sowie der durch die Sequenzen kodierten Proteine zur Diagnose von SLE bei ENA-4 negativen SLE Patienten.

Gegenstand der Erfindung ist auch die Verwendung von einem oder mehreren Markern für SLE ausgewählt aus den Sequenzen SEQ ID No. 25 bis 54, SEQ ID No. 554 bis 583 und SEQ ID No. 1083 bis 1112, Homologen von SEQ ID No. 25 bis 54, SEQ ID No. 554 bis 583 und SEQ ID No. 1083 bis 1112 mit mindestens 95 % Homologie, Teilsequenzen von SEQ ID No. 25 bis 54, SEQ ID No. 554 bis 583 und SEQ ID No. 1083 bis 1112 und Teilsequenzen der Homologen von SEQ ID No. 25 bis 54, SEQ ID No. 554 bis 583 und SEQ ID No. 1083 bis 1112 mit mindestens 95 % Homologie sowie der durch die Sequenzen kodierten Proteine zur Diagnose und Differentialdiagnose von Lupus Nephritis bei SLE Patienten. Lupus Nephritis ist eine häufige und schwerwiegende Komplikation von SLE. Bei vollständigem Versagen der Nierenfunktion ist eine Therapie mit Dialyse notwendig. Zur Vermeidung von Spätschäden ist es daher wichtig eine Nierenbeteilung frühzeitig zu erkennen und zu behandeln. Dies ist ebenfalls von besonderer Bedeutung für die Wirkstoffentwicklung für SLE im Allgemeinen, also auch für die Entwicklung von Wirkstoffen für Patienten mit Lupus Nephritis. Bisher stehen noch keine Biomarker zur Verfügung, die Lupus Nephritis in allen Patienten diagnostizieren können.

Gegenstand der Erfindung sind ebenfalls Marker für SLE und Lupus Nephritis ausgewählt aus den Sequenzen SEQ ID No. 25 bis 54, SEQ ID No. 554 bis 583 und SEQ ID No. 1083 bis 1112, Homologen von SEQ ID No. 25 bis 54, SEQ ID No. 554 bis 583 und SEQ ID No. 1083 bis 1112 mit mindestens 95 % Homologie, Teilsequenzen von SEQ ID No. 25 bis 54, SEQ ID No. 554 bis 583 und SEQ ID No. 1083 bis 1112 und Teilsequenzen der Homologen von SEQ ID No. 25 bis 54, SEQ ID No. 554 bis 583 und SEQ ID No. 1083 bis 1112 mit mindestens 95 % Homologie sowie der durch die Sequenzen kodierten Proteine.

Daher wurden die Autoantikörperprofile von SLE Patienten mit Lupus Nephritis mit denen ohne Lupus Nephritis verglichen. Nach univariater statistischer Auswertung wurde ein Schwellenwert von p<0.05 und eine 1,5 fach veränderte Reaktivität gegenüber der Kontrollgruppe angelegt.

Gegenstand der Erfindung ist auch ein Verfahren zur Früherkennung, Diagnose, Differentialdiagnose, Prognose, Therapiesteuerung und/oder Nachsorge bei SLE, wobei
a. mindestens einer der Marker für SLE ausgewählt aus den Sequenzen SEQ ID No. 1 bis 1587, den Homologen der Sequenzen SEQ ID No. 1 bis 1587 mit mindestens 95 % Homologie, den Teilsequenzen von SEQ ID No. 1 bis 1587 oder den Teilsequenzen der Homologen von SEQ ID No. 1 bis 1587 mit mindestens 95 % Homologie sowie der durch die Sequenzen kodierten Proteine
b. mit Körperflüssigkeit oder Gewebeauszug eines zu untersuchenden Individuums in Kontakt gebracht wird, und
c. der Nachweis einer Wechselwirkung der Körperflüssigkeit oder des Gewebeauszugs mit dem oder den Markern aus a. erfolgt.

Gegenstand der Erfindung ist auch ein Target zur Therapie von SLE ausgewählt aus den Sequenzen SEQ ID No. 1 bis 1587, den Homologen der Sequenzen SEQ ID No. 1 bis 1587 mit mindestens 95 % Homologie, den Teilsequenzen von SEQ ID No. 1 bis 1587 und den Teilsequenzen der Homologen von SEQ ID No. 1 bis 1587 mit mindestens 95 % Homologie sowie der durch die Sequenzen kodierten Proteine.

Gegenstand der Erfindung ist auch eine Zusammensetzung, insbesondere eine pharmazeutische Zusammensetzung umfassend mindestens eine der Sequenzen SEQ ID No. 1 bis 1587, der Homologen der Sequenzen SEQ ID No. 1 bis 1587 mit mindestens 95 % Homologie, den Teilsequenzen von SEQ ID No. 1 bis 1587 oder den Teilsequenzen der Homologen von SEQ ID No. 1 bis 1587 mit mindestens 95 % Homologie sowie der durch die Sequenzen kodierten Proteine.

Gegenstand der Erfindung ist auch ein Verfahren zum Screening von Wirkstoffen für SLE, wobei
a. mindestens einer der Marker ausgewählt aus den Sequenzen SEQ ID No. 1 bis 1587, den Homologen der Sequenzen SEQ ID No. 1 bis 1587 mit mindestens 95 % Homologie, den Teilsequenzen von SEQ ID No. 1 bis 1587 oder den Teilsequenzen der Homologen von SEQ ID No. 1 bis 1587 mit mindestens 95 % Homologie sowie der durch die Sequenzen kodierten Proteine
b. mit einer zu untersuchenden Substanz in Kontakt gebracht wird und
c. der Nachweis einer Wechselwirkung der zu untersuchenden Substanz mit dem oder den Markern a. erfolgt.

Die große klinische Heterogenität von SLE stellt derzeit sowohl für die Diagnostik als auch für die Wirkstoffentwicklung ein großes Problem dar.

Die Identifizierung von spezifischen Antikörpersignaturen in SLE-Patienten-Subgruppen stellt daher einen wichtigen Schritt für die bessere Definition von Patientengruppen in klinischen Studien dar. So könnten beispielsweise wie unter Beispiel 9 dargestellt spezifische Autoantikörper für Lupus Nephritis dazu verwendet werden, diese Subgruppe für Medikamentenstudien zu rekrutieren.

Derzeit sind eine Vielzahl von neuen Wirkstoffen und therapeutischen Antikörpern in der klinischen Entwicklung: Unter anderem werden therapeutische Antikörper gegen Zelloberflächenrezeptoren von Immunzellen, wie z.B.anti-CD20, anti-CD22, oder gegen proinflammatorische Cytokine, wie z.B. anti-IL6 entwickelt. Daher besteht nun durch die Identifizierung serologisch-definierter Subgruppen von SLE die Möglichkeit, diese mit einer Target - spezifischen Antwort auf ein Medikament zu verknüpfen.

Gegenstand der Erfindung ist auch die Verwendung einer oder mehrerer erfindungsgemäßer Marker für SLE, einer erfindungsgemäßen Anordnung (Panel von Markern für SLE), eines erfindungsgemäßen Proteinarrays, eines erfindungsgemäßen Beads, eines erfindungsgemäßen Diagnostikums oder eines erfindungsgemäßen Test Kits zur individualisierten Diagnostik und/oder Therapie bei einzelnen Patienten, Patientengruppen, Kohorten, Bevölkerungsgruppen, Varianten von SLE, Stadien von SLE.

Gegenstand der Erfindung ist auch die Verwendung einer oder mehrerer erfindungsgemäßer Marker für SLE, einer erfindungsgemäßen Anordnung (Panel von Markern für SLE), eines erfindungsgemäßen Proteinarrays, eines erfindungsgemäßen Beads, eines erfindungsgemäßen Diagnostikums oder eines erfindungsgemäßen Test-Kits zum Nachweis und/oder zur Bestimmung der Menge von einem oder mehreren Autoantikörpern die mit SLE assoziiert sind, beispielsweise in Körperflüssigkeiten wie Serum, Gewebe oder Gewebeauszügen des Patienten.

Gegenstand der Erfindung ist auch die Verwendung einer oder mehrerer erfindungsgemäßer Marker, einer erfindungsgemäßen Anordnung, eines erfindungsgemäßen Proteinarrays, eines erfindungsgemäßen Beads, eines erfindungsgemäßen Diagnostikums oder eines erfindungsgemäßen Test Kits zur Analyse von Autoantikörperprofilen von Patienten, insbesondere zur qualitativen und/oder quantitativen Analyse von Autoantikörpern und/oder zur Überwachung von Veränderungen von Autoantikörperprofilen, die mit SLE assoziiert sind, beispielsweise in Körperflüssigkeiten wie Serum, Gewebe oder Gewebeauszügen des Patienten.

Eine besondere Ausführungsform der Erfindung betrifft Verfahren zur Früherkennung und Diagnose von SLE, wobei der Nachweis einer Wechselwirkung der Körperflüssigkeit oder des Gewebeauszugs mit dem oder den Markern ein SLE assoziiertes Autoantikörperprofil des Patienten oder einer Kohorte oder einer Bevölkerungsgruppe oder eines bestimmten Krankheitsverlaufs (Prognose) oder eines bestimmten Ansprechens auf eine Therapie/Arzneimittel abbildet.

Die Erfindung umfasst damit die Verwendung mindestens eines Markers für SLE ausgewählt aus den Sequenzen SEQ ID No. 1 bis 1587, den Homologen der Sequenzen SEQ ID No. 1 bis 1587 mit mindestens 95 % Homologie, den Teilsequenzen von SEQ ID No. 1 bis 1587 oder den Teilsequenzen der Homologen von SEQ ID No. 1 bis 1587 mit mindestens 95 % Homologie sowie der durch die Sequenzen kodierten Proteine zur Analyse von Autoantikörperprofilen von Patienten, insbesondere zur quantitativen Analyse und/oder zur Überwachung von Veränderungen von Autoantikörperprofilen von SLE Patienten.

Der Nachweis einer Wechselwirkung der Körperflüssigkeit oder des Gewebeauszugs mit der oder den SLE Markern kann beispielsweise durch eine Sonde, insbesondere durch einen Antikörper erfolgen.

In einer bevorzugten Ausführungsform werden mindestens 2, beispielsweise 3, 4, 5, 6, 7, 8, 9, 10, vorzugsweise 15 bis 20 Marker für SLE oder 30 bis 50 oder 100 oder mehr Marker zusammen oder in Kombination verwendet, entweder gleichzeitig oder nacheinander und wobei die Marker für SLE ausgewählt unabhängig voneinander ausgewählt werden aus den Sequenzen SEQ ID No. 1 bis 1587, den Homologen der Sequenzen SEQ ID No. 1 bis 1587 mit mindestens 95 % Homologie, den Teilsequenzen von SEQ ID No. 1 bis 1587 oder den Teilsequenzen der Homologen von SEQ ID No. 1 bis 1587 mit mindestens 95 % Homologie sowie der durch die Sequenzen kodierten Proteine.

Eine besondere Ausführungsform der Erfindung betrifft ein erfindungsgemäßes Verfahren, wobei das Stratifizieren oder die Therapiesteuerung Entscheidungen zur Behandlung und Therapie des Patienten, insbesondere Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf, Ätiologie oder Klassifizierung einer Erkrankung samt Prognose umfasst. Ferner betrifft die Erfindung ein Verfahren zum Stratifizieren, insbesondere zur Risikostratifizierung und / oder Therapiesteuerung eines Patienten mit SLE.

Ferner umfasst ist die Stratifizierung der Patienten mit SLE in neue oder etablierte SLE Subgruppen sowie die sinnvolle Auswahl von Patientengruppen für die klinische Entwicklung von neuen Therapeutika. Der Begriff Therapiesteuerung umfasst ebenfalls die Einteilung von Patienten in Responder und Nicht-Responder bezüglich einer Therapie oder dessen Therapieverlauf.

Die Erfindung betrifft insbesondere auch den Nachweis und die Bestimmung der Menge von mindestens zwei verschiedenen Autoantikörpern bei einem Patienten mittels der erfindungsgemäßen SLE Marker wobei vorzugsweise mindestens zwei verschiedene SLE Marker verwendet werden. Gegenstand der Erfindung ist auch eine erfindungsgemäße Verwendung von einer oder mehreren SLE Markern wobei mindestens 2, beispielsweise 3 bis 5 oder 10, vorzugsweise 30 bis 50 oder 50 bis 100 oder mehr SLE Marker bzw. die betreffenden Autoantikörper an oder von einem zu untersuchenden Patienten bestimmt werden.

Die Erfindung umfasst die SLE Marker auf einem festen Träger, beispielsweise einem Filter, einer Membran, einem kleinen Plättchen oder Kügelchen, beispielsweise einem magnetischen oder Fluorophor-markierten Kügelchen, einem Silizium-Wafer, einem Bead, einem Chip, einem massenspektrometrometrischen Target oder einer Matrix oder ähnlichem. Als Träger sind unterschiedliche Materialien geeignet, die dem Fachmann bekannt sind, beispielsweise Glas, Metall, Kunststoff, Filter, PVDF, Nitrocellulose oder Nylon (z.B. Immobilon P Millipore, Protran Whatman, Hybond N+ Amersham).

Der Träger kann beispielsweise einem Gitter entsprechen, dass die Größenordnung einer Mikrotiterplatte (8-12 Wells Streifen, 96 Wells, 384 Wells oder mehr), eines Silizium-Wafers, eines Chips, eines massenspektrometrischen Targets oder einer Matrix besitzt.

In einer Ausführungsform der Erfindung liegen die Marker für SLE als Klonsequenzen bzw. Klon(e) vor.

Die erfindungsgemäßen Marker können mit bekannten Biomarkern für SLE oder Biomarkern für andere Erkrankungen kombiniert, ergänzt oder erweitert werden. Vorzugsweise sind bei einer solchen Kombination mindestens 50 %, vorzugsweise 60 %, besonders bevorzugt 70 % oder mehr erfindungsgemäße Marker für SLE umfasst.

In einer bevorzugten Ausführungsform erfolgt die Verwendung der SLE Marker als auch die erfindungsgemäßen Verfahren außerhalb des menschlichen oder tierischen Körpers, beispielsweise erfolgt die Diagnose ex vivo / in vitro, vorzugsweise mittels einem Assay, wie untern ausgeführt.

"Diagnose" im Sinne dieser Erfindung bedeutet die positive Feststellung von SLE mit Hilfe der erfindungsgemäßen Marker und die Zuordnung der Patienten bzw. deren Symptomen zu der Erkrankung SLE dar. Der Begriff der Diagnose umfasst die medizinische Diagnostik und diesbezügliche Untersuchungen, insbesondere die in-vitro Diagnostik und Labordiagnostik, ebenfalls Proteomics und Nukleinsäureblots. Weitere Untersuchungen können zur Absicherung und zum Ausschluss anderer Krankheiten vonnöten sein. Daher umfasst der Begriff Diagnose insbesondere die Differentialdiagnose von SLE mittels der erfindungsgemäßen Marker.

"Stratifizieren (auch: Stratifikation) oder Therapiesteuerung" im Sinne dieser Erfindung bedeutet, dass beispielsweise die erfindungsgemäßen Verfahren Entscheidungen zur Behandlung und Therapie des Patienten erlauben, sei es Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf bzw. Ätiologie oder Klassifizierung einer Erkrankung, z.B. in einen neuen oder bestehenden Subtyp oder die Differenzierung von Krankheiten und dessen Patienten. In einer weiteren Ausführungsform der Erfindung umfasst der Begriff "Stratifizierung" insbesondere die Risikostratifizierung mit der Prognose eines "outcome" eines nachteiligen gesundheitlichen Ereignisses.

"Prognose" bedeutet die Vorhersage des Krankheitsverlaufs.

Erfindungsgemäß bedeutet "Therapiesteuerung", beispielsweise Vorhersage und Überwachung des Ansprechens auf ein Arzneimittel oder eine Therapie sowie die Nachsorge.

Im Rahmen dieser Erfindung wird unter "Patient" bzw. "Patientin" ein beliebiger Proband, ein beliebiges Individuum - Mensch oder Säugetier - verstanden, mit der Maßgabe, dass der Proband bzw. das Individuum auf SLE untersucht wird.

Der Begriff Marker für SLE im Sinne dieser Erfindung bedeutet, dass die Nukleinsäure, beispielsweise DNA, insbesondere cDNA oder RNA oder die kodierte Aminosäuresequenz oder das Polypeptid oder Protein signifikant (spezifisch) für SLE und / oder die mit SLE einhergehenden Autoantikörperprofile sind. Erfindungsgemäße Marker sind Nukleinsäuresequenzen und / oder Aminosäuresequenzen gemäß der Definition im anliegenden Sequenzprotokoll (SEQ ID No. 1 bis SEQ ID No. 1587), deren Homologe und Teilsequenzen und wobei auch modifizierte Nukleinsäure- und Aminosäuresequenzen umfasst sind. Dabei bedeutet, Marker für SLE beispielsweise, dass die cDNA oder RNA oder das jeweils daraus erhältliche Polypeptid oder Protein eine Wechselwirkung mit Substanzen aus der Körperflüssigkeit oder Gewebeauszug eines Patienten mit SLE aufweisen (z.B. Antigen (Epitop) / Antikörper (Paratop) Wechselwirkung). In einer besonders bevorzugten Ausführungsform der Erfindung ist der Marker für SLE ein Antigen oder ein Teil eines Antigens oder kodiert für ein Antigen oder für einen Teil eines Antigens.

Die Substanzen aus der Körperflüssigkeit oder Gewebeauszug treten entweder nur oder zumindest verstärkt bei SLE auf beziehungsweise werden exprimiert, wohingegen diese Substanzen bei Patienten ohne SLE oder Gesunden nicht oder zumindest in geringerem Maße (geringere Menge, geringerer Konzentration) vorhanden sind. Marker für SLE können sich andererseits auch dadurch auszeichnen, dass sie eine Wechselwirkung mit Substanzen aus der Körperflüssigkeit oder Gewebeauszug von Patienten mit SLE eingehen, weil diese Substanzen nicht mehr oder zumindest in deutlich geringerer Menge/Konzentration bei SLE auftreten beziehungsweise exprimiert werden, wohingegen diese Substanzen bei Patienten ohne SLE vorhanden oder zumindest in deutlich höherem Maße vorhanden sind. Marker für SLE können auch in gesunden Probanden vorhanden sein, jedoch verändert sich ihre Menge (Konzentration) beispielsweise bei der Entstehung, Etablierung und Therapie von SLE. Ein oder mehrere Marker können auf diese Weise ein Profil von Substanzen aus Körperflüssigkeit und Gewebeauszug abbilden, beispielsweise ein SLE assoziiertes Autoantikörperprofil des betreffenden Patienten. Erfindungsgemäße Marker sind Biomarker für SLE.

Autoantikörperprofile umfassen die Menge an einem oder mehreren Autoantikörpern deren Vorkommen/Expression mit der Entstehung und/oder Etablierung von SLE einhergehen. Autoantikörperprofile umfassen somit einerseits die Zusammensetzung, d.h. es werden beispielsweise ein oder mehrere Autoantikörper nur bei SLE exprimiert, und anderseits die Menge/Konzentration einzelner Autoantikörper, d.h. die Menge/Konzentration einzelner Autoantikörper verändert sich bei der Entstehung und Etablierung von SLE. Diese Veränderungen können mit Hilfe der erfindungsgemäßen Markersequenzen nachgewiesen werden.

In einer besonders bevorzugten Ausführungsform erkennt/bindet der SLE Marker an Autoantikörper, die im Verlauf der Entstehung, Etablierung und Therapie von SLE (verstärkt) vorhanden sind oder in geringerem Maße (oder nicht mehr) vorhanden sind. Autoantikörper werden vom Körper gegen körpereigene Antigene, die beispielsweise bei SLE entstehen, gebildet. Autoantikörper werden von Körper gegen unterschiedliche Substanzen und Pathogene gebildet. Im Rahmen der vorliegenden Erfindung werden insbesondere die Autoantikörper detektiert, die beim Auftreten und im Laufe der Entstehung von SLE gebildet werden und/oder in ihrer Expression hoch- beziehungsweise herunterreguliert werden. Diese Autoantikörper können mit Hilfe der erfindungsgemäßen Verfahren und Marker nachgewiesen werden und ihr Nachweis und ihre Überwachung (z.B. der Menge) kann zur Früherkennung, Diagnose und/oder Therapieüberwachung/Therapiesteuerung sowie zur Prognose und Vorhersage des Risikos des Wiederauftretens von SLE im Rahmen der Nachsorge verwendet werden.

Die Autoantikörperprofile können bereits bei Verwendung eines einzigen SLE Markers ausreichend charakterisiert werden. In anderen Fällen werden zwei oder mehr SLE Marker notwendig sein, um ein Autoantikörperprofil abzubilden, das für SLE spezifisch ist.

In einer Ausführungsform der Erfindung können Autoantikörper mit SLE Markern nachgewiesen werden, die sich von einem anderen Individuum ableiten und die beispielsweise aus einer kommerziellen cDNA-Bank stammen.

In einer anderen Ausführungsform der Erfindung können diese Autoantikörper mit SLE Markern nachgewiesen werden, die sich von dem gleichen Individuum ableiten und die beispielsweise aus einer eigens für den Patienten oder eine Gruppe von Patienten beispielsweise im Rahmen der individualisierten Medizin hergestellten cDNA-Bank stammen. Beispielsweise können Homologe der genannten SLE Marker mit den Sequenzen SEQ ID. No. 1 bis 1587 oder Teilsequenzen davon verwendet werden.

Autoantikörper können bereits viele Jahre vor Auftreten der ersten Krankheitssymptome vom Patienten gebildet werden. Damit wären eine Früherkennung, Diagnose und auch Prognose und vorbeugende Behandlung bzw. Umstellung der Lebensweise und andere Möglichkeiten der Prävention bereits Jahre vor dem sichtbaren Krankheitsausbruch möglich. Die erfindungsgemäßen Vorrichtungen, Mittel und Verfahren ermöglichen somit ein im Vergleich zu bekannten Verfahren sehr frühes Eingreifen, was die Prävention, Behandlungsmöglichkeiten und Folgen von SLE deutlich verbessert.

Da sich die SLE-assoziierten Autoantikörperprofile während der Etablierung und Behandlung/Therapie von SLE verändern, ermöglicht die Erfindung auch den Nachweis und die Überwachung von SLE in jedem Stadium der Entstehung und Behandlung sowie die Überwachung im Rahmen der SLE Nachsorge. Die erfindungsgemäßen Mittel, beispielsweise eine entsprechendes Diagnostikum oder ein Test-Kit erlauben auch eine einfache Handhabung zu Hause durch den Patienten und die kostengünstige routinemäßige Vorsorge zur Früherkennung.

Insbesondere durch die Verwendung von Antigenen als spezifische Markers für SLE, die sich von bereits bekannten Sequenzen, z.B. aus kommerziellen cDNA Banken, ableiten, können Probanden getestet und gegebenenfalls vorhandene SLE assoziierte Autoantikörper in diesen Probanden nachgewiesen werden, auch wenn bei diesen Probanden die entsprechenden Autoantigene (noch) nicht bekannt sind.

Verschiedene Patienten können unterschiedliche SLE assoziierte Autoantikörperprofile aufweisen, beispielsweise unterscheiden sich verschiedene Kohorten oder Bevölkerungsgruppen. Jeder Patient kann dabei ein oder mehrere verschiedene SLE assoziierte Autoantikörper im Laufe der Entstehung von SLE und des Fortschreitens der SLE Erkrankung, d.h. ebenfalls verschiedene Autoantikörperprofile, bilden. Außerdem kann sich die Zusammensetzung und/oder die Menge der gebildeten Autoantikörper im Laufe der SLE Entstehung und des Fortschreitens der Krankheit verändern, so dass eine quantitative Auswertung notwendig wird. Auch die Therapie/Behandlung von SLE führt zu Veränderungen in der Zusammensetzung und/oder der Menge an SLE assoziierten Autoantikörpern. Die große Auswahl an erfindungsgemäßen SLE Markern, die mit dieser Erfindung bereitgestellt werden, ermöglicht die individuelle Zusammenstellung von SLE Markern in einer Anordnung, einem Panel für einzelne Patienten, Gruppen von Patienten, bestimmte Kohorten, Bevölkerungsgruppen und dergleichen. Im Einzelfall kann deshalb die Verwendung eines SLE Markers ausreichen, während in anderen Fällen mindestens zwei oder mehr SLE Marker zusammen oder in Kombination verwendet werden müssen um ein aussagekräftiges Autoantikörperprofil zu erstellen.

Der Nachweis von SLE assoziierten Autoantikörpern beispielsweise im Serum oder Plasma von Patienten hat gegenüber anderen Biomarkern den Vorteil einer hohen Stabilität und Lagerfähigkeit und einer guten Nachweisbarkeit. Auch unterliegt die Anwesenheit von Autoantikörpern keinem circardianen Rhythmus, so dass die Probennahme unabhängig von Tageszeit, Nahrungsaufnahme und dergleichen ist.

Daneben können die SLE assoziierten Autoantikörper mit Hilfe der korrespondierenden Antigene/Autoantigene in bekannten Assays wie z.B. ELISA oder Western-Blot nachgewiesen werden und auf diese Weise die Ergebnisse überprüft werden.

Im Sinne der Erfindung wird eine Wechselwirkung zwischen dem SLE Marker und dem betreffenden Serum, beispielsweise einem Autoantikörper des Patienten, nachgewiesen. Eine solche Wechselwirkung ist z.B. eine Bindung, insbesondere eine bindende Substanz an mindestens einen Marker für SLE oder für den Fall, dass der Marker für SLE eine Nukleinsäure, beispielsweise eine cDNA ist, die Hybridisierung mit einer geeigneten Substanz unter gewählten Bedingungen, insbesondere stringenten Bedingungen (z.B. wie üblich definiert in J. Sambrook, E.F. Fritsch, T. Maniatis (1989), Molecular cloning: A laboratory manual, 2nd Edition, Cold Spring Habor Laboratory Press, Cold Spring Habor, USA oder Ausubel, "Current Protocols in Molecular Biology", Green Publishing Associates and Wiley Interscience, N.Y. (1989)). Ein Beispiel für stringente Hybridisierungsbedingungen ist: Hybridisierung in 4 x SSC bei 65° C (alternativ in 50% Formamid und 4 X SSC bei 42° C), gefolgt von mehreren Waschschritten in 0,1 x SSC bei 65°C für insgesamt etwa eine Stunde. Ein Beispiel für wenig stringente Hybridisierungsbedingungen ist Hybridisierung in 4 x SSC bei 37° C, gefolgt von mehreren Waschritten in 1 x SSC bei Raumtemperatur. Die Wechselwirkung zwischen der Körperflüssigkeit oder dem Gewebeauszuges eines Patienten und den Markern für SLE ist vorzugsweise eine Protein-Protein Wechselwirkung.

Solche Substanzen, beispielsweise Antigene, Autoantigene, SLE assoziierte Autoantikörper, sind erfindungsgemäß Bestandteil einer Körperflüssigkeit, insbesondere Blut, Vollblut, Blutplasma, Blutserum, Patientenserum, Urin, Cerebrospinalflüssigkeit, Synovialflüssigkeit oder eines Gewebeauszuges des Patienten. Die Erfindung betrifft insbesondere die Verwendung dieser Körperflüssigkeiten und Gewebeauszüge für Früherkennung, Diagnose, Prognose, Therapiesteuerung und Nachsorge.

Die SLE spezifischen Marker verfügen in einer weiteren Ausführungsform der Erfindung über ein Erkennungssignal, welches an die zu bindende Substanz adressiert ist (z.B. Antikörper, Nukleinsäure). Erfindungsgemäß bevorzugt ist für ein Protein das Erkennungssignal Epitop und / oder Paratop und / oder Hapten und für eine cDNA eine Hybridisierungs- oder Bindungsregion.

Umfasst sind auch Homologe der erfindungsgemäßen Marker SEQ ID NO. 1 bis 1587, wie z.B. in den Ansprüchen dargelegt. Homologe im Sinne der Erfindung sind zum einen solche mit Homologie der Amino- bzw. Nukleinsäuresequenz und solche, bei denen die entsprechende Sequenz modifiziert ist, beispielsweise die Proteinvarianten, die zwar die gleiche Aminosäuresequenz aufweisen, sich aber im Hinblick auf die Modifikation, insbesondere die posttranslationale Modifikation, unterscheiden.

Erfindungsgemäß umfasst sind Modifikationen der Nukleinsäuresequenz und der Aminosäuresequenz, beispielsweise Citrullinierung, Acetylierung, Phosphorylierung, Glykosilierung, Methylierung, polyA-Strang-Verlängerung und andere dem Fachmann einschlägig bekannte Modifikationen.

Homologe umfassen auch Sequenz-Homologe der Marker und deren Teilsequenzen. Sequenz Homologe sind beispielsweise Nukleinsäure- und/oder Proteinsequenzen, die eine Identität mit den SLE Markern der Sequenzen SEQ ID No. 1 bis 1587 von mindestens 70 % oder 80 %, vorzugsweise 90 % oder 95 %, besonders bevorzugt 96 % oder 97 % oder mehr, beispielsweise 98 % oder 99 % aufweisen. In einer besonders bevorzugten Ausführungsform der Erfindung beträgt für den Fall, dass es sich bei den SLE Markern um Antigene handelt, die Homologie im Sequenzbereich, in dem die Antigen-Antikörper- beziehungsweise Antigen-Autoantikörper-Wechselwirkung stattfindet, mindestens 95 %, vorzugsweise mindestens 97 %, besonders bevorzugt mindestens 99 %. Erfindungsgemäß umfasst sind beispielsweise Mutationen wie Basenaustauschmutationen, Rastermutationen, Baseneinschubmutationen, Basenverlustmutationen, Punktmutationen, Insertionsmutationen.

Gegenstand der Erfindung sind auch Teilsequenzen der SLE Marker mit der Sequenz SEQ ID No. 1 bis 1587. Teilsequenzen sind solche Nukleinsäure- oder Aminosäuresequenzen, die gegenüber der vollständigen Nukleinsäure oder dem vollständigen Protein/Peptid verkürzt sind. Die Deletion kann sich dabei an dem oder den Ende und/oder innerhalb der Sequenz befinden. Umfasst sind beispielsweise Teilsequenzen, die 50 bis 100 Nukleotide, 70-120 Nukleotide der Sequenz SEQ ID No. 1 bis 1587 aufweisen. Homologe von Teilsequenzen sind ebenfalls erfindungsgemäß umfasst. In einer besonderen Ausführungsform sind die SLE Marker gegenüber den Sequenzen SEQ ID No. 1 bis 1587 so weit verkürzt, dass sie nur noch aus der/den Bindungsstellen für den betreffenden SLE assoziierten Autoantikörper bestehen. Erfindungsgemäß umfasst sind auch SLE Marker, die sich von den Sequenzen SEQ ID No. 1 bis 1587 dadurch unterscheiden, dass sie ein oder mehrere Insertionen beinhalten, wobei die Insertionen beispielsweise 1 bis 100 oder mehr Nukleotide/Aminosäuren, vorzugsweise 5 bis 50, besonders bevorzugt 10 bis 20 Nukleotide/Aminosäuren lang sind und die Sequenzen aber ansonsten identisch oder homolog zu den Sequenzen SEQ ID No. 1 bis 1587 sind. Besonders bevorzugt sind Teilsequenzen, die mindestens 90 %, vorzugsweise mindestens 95 %, besonders bevorzugt mindestens 97 % oder 98 % der Länge der erfindungsgemäßen SLE Marker mit den Sequenzen SEQ ID No. 1 bis 1587 aufweisen.

In einer weiteren Ausführungsform kann der jeweilige SLE Marker in unterschiedlichen Mengen in einem oder mehreren Bereichen in der Anordnung oder auf dem Träger oder in einem Panel repräsentiert sein. Dies erlaubt eine Variation der Sensitivität. Die Bereiche können jeweils eine Gesamtheit von SLE Markern aufweisen, d.h. eine genügende Zahl an verschiedenen SLE Markern, insbesondere 2, 3, 4, 5, 6, 7, 8, 9 oder 10 oder mehr verschiedene. Beispielsweise können 20 bis 50 (numerisch) oder mehr, bevorzugt mehr als 100, besonders bevorzugt 150 oder mehr, beispielsweise 25.000 oder 5000 oder 10000 verschiedene oder gleiche SLE Markersequenzen und gegebenenfalls weitere Nukleinsäuren und/oder Proteine, insbesondere andere Biomarker auf dem Träger oder in dem Panel repräsentiert sein.

Ganz besonders bevorzugt ist ein oder mehrere Panel, wie in den Beispielen dargelegt und zwar ausgewählt aus den Sequenzen, vorzugsweise Proteinsequenzen, bestehend aus mindestens zwei Marker, fünf Marker oder 10 Marker oder mehr ausgewählt aus:
Panel I (P1)
   SEQ ID No. 1, 2, 3, 5, 7, 8, 10, 12, 13, 15, 17, 18, 19, 20, 24,
   SEQ ID No. 530, 531, 532, 534, 536, 537, 539, 541, 542, 544, 546, 547, 548, 549, 553, vorzugsweise
   SEQ ID No. 1059, 1060, 1061, 1063, 1065, 1066, 1068, 1070, 1071, 1073, 1075, 1076, 1077, 1078, 1082, und / oder
Panel II (P2)
   SEQ ID No. 1, 3, 4, 5, 7, 12, 13, 14, 15, 17, 18, 19, 20, 24
   SEQ ID No. 530, 532, 533, 534, 536, 541, 542, 543, 544, 546, 547, 548, 549, 553, vorzugsweise
   SEQ ID No. 1059, 1061, 1062, 1063, 1065, 1070, 1071, 1072, 1073, 1075, 1076, 1077, 1078, 1082, und / oder
Panel III (P3)
   SEQ ID No. 1, 2, 3, 5, 6, 7, 8, 9, 10, 15, 16, 18, 21, 23
   SEQ ID No. 530, 531, 532, 534, 535, 536, 537, 538, 539, 544, 545, 547, 550, 552, vorzugsweise
   SEQ ID No. 1059, 1060, 1061, 1063, 1064, 1065, 1066, 1067, 1068, 1073, 1074, 1076, 1079, 1081, und / oder
Panel IV (P4)
   SEQ ID No. 1, 2, 3, 4, 5, 8, 9, 10, 12, 13, 14, 15, 17, 19, 20
   SEQ ID No. 530, 531, 532, 533, 534, 537, 538, 539, 541, 542, 543, 544, 546, 548, 549, vorzugsweise
   SEQ ID No. 1059, 1060, 1061, 1062, 1063, 1066, 1067, 1068, 1070, 1071, 1072, 1073, 1075, 1077, 1078, und / oder
Panel V
   SEQ ID No. 1, 2, 3, 4, 5, 6, 7, 11, 15, 16, 18, 21, 22, 23, 24
   SEQ ID No. 530, 531, 532, 533, 534, 535, 536, 540, 544, 545, 547, 550, 551, 552, 553, vorzugsweise
   SEQ ID No. 1059, 1060, 1061, 1062, 1063, 1064, 1065, 1069, 1073, 1074, 1076, 1079, 1080, 1081, 1082, und / oder
Panel VI
   SEQ ID No. 2, 5, 6, 7, 8, 10, 13, 18, 19, 22, 168
   SEQ ID No. 531, 534, 535, 536, 537, 539, 542, 547, 548, 551, 697, vorzugsweise
   SEQ ID No. 1060, 1063, 1064, 1065, 1066, 1068, 1071, 1076, 1077, 1080, 1226, und / oder
Panel VII
   SEQ ID No. 1, 2, 5, 6, 7, 8, 9, 10, 13, 15, 19, 22, 24, 134, 168, 214, 368, 369, 370
   SEQ ID No. 530, 531, 534, 535, 536, 537, 538, 539, 542, 544, 548, 551, 553, 663, 697, 743, 897, 898, 899, vorzugsweise
   SEQ ID No. 1059, 1060, 1063, 1064, 1065, 1066, 1067, 1068, 1071, 1073, 1077, 1080, 1082, 1192, 1226, 1272, 1426, 1427, 1428, und / oder
Panel VIII (P8)
   SEQ ID No. 1, 2, 4, 5, 6, 7, 8, 9, 10, 12, 13, 15, 17, 18, 19, 20, 21, 22, 23, 24, 29, 31, 46, 95, 128, 134, 136, 143, 163, 168, 169, 171, 188, 214, 349, 368, 369, 370, 371-392, 424-434
   SEQ ID No. 530, 531, 533, 534, 535, 536, 537, 538, 539, 541, 542, 544, 546, 547, 548, 549, 550, 551, 552, 553, 558, 560, 575, 624, 657, 663, 665, 692, 697, 698, 700, 717, 743, 878, 897, 898, 899, 900-921, 953-963, vorzugsweise
   SEQ ID No. 1059, 1060, 1062, 1063, 1064, 1065, 1066, 1067, 1068, 1070, 1071, 1073, 1075, 1076, 1077, 1078, 1079, 1080, 1081, 1082, 1087, 1089, 1104, 1153, 1186, 1192, 1194, 1221, 1222, 1226, 1227, 1229, 1246, 1272, 1407, 1426, 1427, 1428, 1429-1450, 1482-1492, und / oder
Panel IX (P9)
   SEQ ID No. 1, 2, 3, 4, 5, 6, 7, 8, 9, 10, 11, 12, 13, 14, 15, 16, 17, 18, 19, 20, 21, 22, 23, 24, 29, 31, 33, 41, 46, 48, 74, 95, 105, 108, 114, 115, 116, 128, 132, 134, 136, 143, 163, 168, 169, 171, 188, 214, 349, 368, 369, 370, 371-392, 424-434
   SEQ ID No. 530, 531, 532, 533, 534, 535, 536, 537, 538, 539, 540, 541, 542, 543, 544, 545, 546, 547, 548, 549, 550, 551, 552, 553, 558, 560, 562, 570, 575, 577, 603, 624, 634, 637, 643, 644, 645, 657, 661, 663, 665, 672, 692, 697, 698, 700, 717, 743, 878, 897, 898, 899, 900-921, 953-963, vorzugsweise
   SEQ ID No. 1059, 1060, 1061, 1062, 1063, 1064, 1065, 1066, 1067, 1068, 1069, 1070, 1071, 1072, 1073, 1074, 1075, 1076, 1077, 1078, 1079, 1080, 1081, 1082, 1087, 1089, 1091, 1099, 1104, 1106, 1132, 1153, 1163, 1166, 1172, 1173, 1174, 1186, 1190, 1192, 1194, 1201, 1221, 1226, 1227, 1229, 1246, 1272, 1407, 1426, 1427, 1428, 1429-1450, 1482-1492
oder deren jeweilgen Homologen, Teilsequenzen, wie vorgenannt zu den einzelnen Markersequenzen.

Diese genannten Panels erlauben besonders vorteilhaft die Durchführung der erfindungsgemäßen Verfahren, vgl. Beispiele.

Im Rahmen dieser Erfindung bedeutet "Anordnung" synonym "Array" und synonym "Panel". Sofern dieser "Array" zur Identifizierung von Substanzen an SLE Markern verwendet wird, ist hierunter vorzugsweise ein "Assay" oder ein Bead oder eine diagnostische Vorrichtung oder ein Screening Assay zu verstehen. In einer bevorzugten Ausführungsform ist die Anordnung derart gestaltet, dass die auf der Anordnung repräsentierten Marker in Form eines Gitters auf einem Träger vorliegen. Ferner sind solche Anordnungen bevorzugt, die eine hochdichte (high-density) Anordnung von SLE Markern erlauben. Vorzugsweise werden die Marker gespottet. Solche hochdichte gespotteten Anordnungen sind beispielsweise in der WO 99/57311 und WO 99/57312 offenbart und können vorteilhaft in einem robotergestützten automatisierten High-Throughput Verfahren zur Anwendung kommen.

Im Rahmen dieser Erfindung umfasst der Begriff "Assay" oder Diagnostikum ebenfalls solche Ausführungsformen wie ELISA, Bead-based Assay, Line Assay, Western Blot, immunchromatographische Verfahren (z.B. so genannte Lateral Flow Immunoassays) oder ähnliche immunologische Single- oder Multiplex-Nachweisverfahren.

Ein "Proteinarray" im Sinne dieser Erfindung ist die systematische Anordnung von SLE Markern auf einem festen Träger und wobei der Träger jede beliebige Form und/oder Größe haben kann und wobei der Träger vorzugsweise ein fester Träger ist.

Die SLE Marker der Anordnung/Panel sind auf dem Träger fixiert, vorzugsweise gespottet oder immobilisiert, aufgedruckt oder ähnliches, insbesondere reproduzierbar aufgebracht. Ein oder mehrere SLE Marker können mehrfach in der Gesamtheit aller SLE Marker präsent sein und in unterschiedlichen Mengen bezogen auf einen Spot vorliegen. Ferner können die SLE Marker auf dem Träger standardisiert sein (z.B. mittels serieller Verdünnungsreihen von z.B. Humanglobulinen als interne Kalibratoren zur Datennormalisierung und quantitativen Auswertung). Gegebenenfalls kann auch ein Standard (z.B. ein Gold Standard) auf dem Träger aufgebracht sein.

In einer weiteren Ausführungsform liegen die SLE Marker als Klone vor. Solche Klone können beispielsweise mittels einer erfindungsgemäßen cDNA-Expressionsbibliothek erhalten werden. In einer bevorzugten Ausführungsform werden solche Expressionsbibliotheken mittels Expressionsvektoren aus einer exprimierenden cDNA Bibliothek, umfassend die cDNAs der SLE spezifischen Markersequenzen, erhalten. Diese Expressionsvektoren enthalten vorzugsweise induzierbare Promotoren. Die Induktion der Expression kann z.B. mittels eines Induktors, solche wie IPTG, erfolgen. Geeignete Expressionsvektoren sind beschrieben in Terpe et al. (Terpe T Appl Microbiol Biotechnol. 2003 Jan; 60(5):523-33).

Expressionsbibliotheken sind dem Fachmann bekannt, diese können nach Standardwerken, wie Sambrook et al, "Molecular Cloning, A laboratory handbook, 2nd edition (1989), CSH press, Cold Spring Harbor, New York hergestellt werden. Weiterhin bevorzugt sind solche Expressionsbibliotheken, die gewebespezifisch sind (z.B. humanes Gewebe, insbesondere humane Organe. Ferner sind erfindungsgemäß ebenfalls solche Expressionsbibliotheken mit eingeschlossen, die mittels exontrapping erhalten werden können.

Weiterhin bevorzugt sind Proteinarrays oder entsprechende Expressionsbibliotheken, die keine Redundanz aufweisen (so genannte: Uniclone®-Bibliothek) und nach den Lehren der WO 99/57311 und WO 99/57312 beispielsweise hergestellt werden können. Diese bevorzugten Uniclone- Bibliotheken weisen einen hohen Anteil an nicht-fehlerhaften vollständig exprimierten Proteinen einer cDNA-Expressionsbibliothek auf.

Im Rahmen dieser Erfindung können die Klone ebenfalls nicht abschließend solche sein wie transformierte Bakterien, rekombinante Phagen oder transformierte Zellen von Säugern, Insekten, Pilzen, Hefen oder Pflanzen.

Zusätzlich können die SLE Marker in der jeweiligen Form als Fusionsprotein vorliegen, welches beispielsweise mindestens ein Affinitätsepitop oder "Tag" enthält, wobei das Tag beispielsweise ausgewählt wird aus c-myc, His-Tag, Arg-tag, FLAG, alkalische Phosphatase, V5-Tag, T7-Tag oder Strep-Tag, HAT-tag, NusA, S-tag, SBP-tag, Thioredoxin, DsbA oder das Fusionsprotein beispielsweise ein oder mehrere zusätzliche Domänen aufweist, beispielsweise eine Cellulose-bindende Domäne, grünfluoreszierendes Protein, Maltose bindendes Protein, Calmodulin-bindendes Protein, Glutathione S-transferase oder lacZ.

In einer weiteren Ausführungsform betrifft die Erfindung einen Assay, beispielsweise einen multiplex Assay, einen Bead basierten Assay oder Proteinarray zum Identifizieren und Charakterisieren einer Substanz, beispielsweise eines Hits, einer Leitsubstanz, eines Wirkstoffs für SLE. Dabei wird eine zu untersuchende Substanz eingesetzt. Diese kann ein beliebiges natives oder nicht-natives Biomolekül, ein (synthetisches) chemisches Molekül, ein Naturstoff, eine Mischung oder eine Substanzbibliothek sein. Nachdem die zu untersuchende Substanz einen SLE Marker kontaktiert hat, erfolgt die Auswertung des Bindungserfolges, beispielsweise unter Verwendung mit handelsüblicher Image-Analyse Software (GenePix Pro (Axon Laboratories), Aida (Raytest), ScanArray (Packard Bioscience).

Die Visualisierung erfindungsgemäßer Bindungen, Bindungserfolge, Wechselwirkungen wie Protein-Protein-Wechselwirkungen (z.B. Protein an SLE Marker wie Antigen/Antikörper) oder entsprechende "Mittel zum Nachweis des Bindungserfolges" kann beispielsweise mittels Fluoresenzmarkierung, Biotiniylierung, Radio-Isotopen-Markierung oder kolloidale Gold- oder Latex-Partikel-Markierung in üblicher Weise erfolgen. Ein Nachweis von gebundenen Antikörpern erfolgt vorzugsweise mit Hilfe von sekundären Antikörpern, die mit handelsüblichen Reportermolekülen markiert sind (z.B. Cy-, Alexa , Dyomics, FITC- oder ähnliche Fluoreszenzfarbstoffe, kolloidale Gold- oder Latex-Partikel), oder mit Reporter-Enzymen wie alkalischer Phosphatase, Meerrettichperoxidase, usw. und den entsprechenden colorimetrischen, fluoreszenten oder chemolumineszenten Substraten. Eine Auslesung erfolgt z.B. mittels eines Microarray-Laserscanners, einer CCD-Kamera oder visuell.

In einer weiteren Ausführungsform betrifft die Erfindung ein Arzneimittel oder einen Wirkstoff oder eine Prodrug für SLE entwickelt und erhältlich durch den Einsatz eines erfindungsgemäßen SLE Markers.

Gegenstand der Erfindung ist auch die Verwendung eines SLE Markers ausgewählt aus Sequenzen SEQ ID No. 1 bis 1587 und Teilsequenzen von SEQ ID No. 1 bis 1587 mit mindestens 90 %, vorzugsweise mindestens 95 % der Länge von SEQ ID No. 1 bis 1587 und Homologen von SEQ ID No. 1 bis 1587 und deren Teilsequenzen mit einer Identität von mindestens 95 %, vorzugsweise mindestens 98 % oder mehr zu den entsprechenden Sequenzen und Proteinen/Peptiden kodiert durch die Sequenzen SEQ ID No. 1 bis 1058, kodiert durch deren Teilsequenzen und Homologen als Affinitätsmaterial zur Durchführung einer Apherese oder Blutwäsche für Patienten mit SLE, d.h. Apherese von SLE Autoantikörper. Die Erfindung betrifft somit die Verwendung der erfindungsgemäßen Marker, vorzugsweise in Form einer Anordnung, als Affinitätsmaterial zur Durchführung einer Apherese oder Blutwäsche im weiteren Sinne, wobei Substanzen aus Körperflüssigkeiten eines Patienten mit SLE, wie Blut oder Plasma, an die erfindungsgemäßen Marker binden und folglich der Körperflüssigkeit selektiv entzogen werden können. Die Anwendung in der Blutwäsche ist ein Spezialfall der Verwendung der SLE Marker als Target. Vorrichtungen zur Durchführung einer Blutwäsche, insbesondere Immunapherese, sind dem Fachmann bekannt und kann bespielsweise mittels einer Dialyse durchgeführt werden.

Die nachfolgenden Beispiele und Figuren dienen der Erläuterung der Erfindung ohne die Erfindung jedoch auf die Beispiele einzuschränken.
Figur 1 zeigt einen Vulcano Plot der relativen Antigenreaktivitäten der SLE Patienten im Vergleich zu Gesundkontrollen.
Figur 2 zeigt einen Vulcano Plot der relativen Antigenreaktivitäten der SLE Patienten im Vergleich zu RA Patienten.
Figur 3: Vulcano Plot der Antigenreaktivitäten von SLE Patienten gegen eine vereinigte Gruppe von Patienten mit verschiedenen Autoimmunerkrankungen wie SSc (PSS), SPA, frühere Rheumatoide Arthritis und SPA.
Figur 4 Frequenz der Autoantikörperreaktivitäten ausgewählter Antigen in SLE Patienten und Gesundproben. Es wurde ein Schwellenwert von 3 SD Abweichungen über dem Mittelwert der Gesundprobe angesetzt. Der Schwellenwert für das Antigen SNRNP wurde auf 2SD gesetzt.
Figur 5: Vulcano Plot der Autoantikörperreaktivitäten von ENA-4 negativen SLE Patienten gegen Gesundkontrollen.
Figur 6: Receiver Operating Characteristic-Kurven(ROC)für die Diagnose von SLE im Vergleich zu Gesundproben und AID-Proben
Figur 7: Vulcano Plot SLE Lupus Nephritis gegen SLE ohne Lupus Nephritis
Figur 8: Häufigkeit der Lupus Nephritis Antigene in einem Modell mit geschachtelter Kreuzvalidierung.
Figur 9: Dendogramm der SLE Antigene nach Berechnung des Spearman Rangkorrelationskoeffizienten a) Dendrogramm der bekannten ENA-4 Antigene und b) Dendrogramm von 50 ausgewählten SLE Antigenen.
Figur 10: PPLS-DA Biplot der SLE Patienten und Gesundkontrollen unter Verwendung der SLE Antigene a) PPLS-DA Biplot basierend auf den ENA-4 und ribosomalen Antigenen b) PPLS-DA Biplot basierend auf 50 SLE Antigenen.

### Beispiele

### Beispiel 1: Auswahl der SLE Patienten und Probanden

Auswahl der zu testenden Patientengruppen: Es wurden Blutproben von 129 SLE Patienten, 100 Patienten mit Systemischer Sklerodermie (SSc, PSS), 75 Patienten mit Rheumatoider Arthritis (RA), 537 Patienten mit früher RA (Erkrankungsdauer unter 6 Monaten) und 75 Patienten mit ankylosierende Spondylitis (SPA)/Morbus Bechterew analysiert. Als Kontrollgruppe wurden 343 Blutproben vom Bayrischen Roten Kreuz (BRK) bezogen. Von allen Probanden wurde eine Einverständniserklärung (Informed Consent) der Ethikkommission der klinischen Partner und der Biobank des BRK eingeholt.

**Tabelle 1: Patientenproben und klinische Daten (Testkohorte I)**

| | 1. Screen | | | 2. Screen | | | | | |
|---|---|---|---|---|---|---|---|---|---|
| | SLE | RA | Gesund | SLE | SSc (PSS) | | Early RA (<6 Monate) | SPA | Gesund |
| | | | | | Total | Subtyp | | | |
| Nummer | 129 | 75 | 123 | 100 | 100 | | 537 | 82 | 343 |
| Alter (Jahre) | 39 +/-12 | 56.6 +/-13.2 | 41.3 +/-11 | 39.8 +/-11.9 | 56.9 +/-13.4 | Limitiert n=50 | 56.8 +/-14.3 | 43.7 +/-10.1 | 47.7 +/-11.7 |
| % Weiblich | 86.1 | 72 | 86.2 | 83 | 87 | Diffus n=32 | 62.2 | 15.9 | 58.3 |
| % ANA | 77.5 | N.D. | N.D. | 100 | 95 | Overlap n=9 | N.D. | N.D. | N.D. |
| SLAM | 7.7 +/-5.1 | | | 7.7 +/-5.1 | | | | | |
| SLICC | 1.45 +/-1.8 | | | 1.45 +/-1.8 | | | | | |
| ANA % | | | | | | | | | |
| ENA-4 positive % | 37 | | | 48 | | | | | |
| U1-RNP (% of ENA-4 pos.) | 13 | | | 13 | | | | | |
| Sm(% of ENA-4 pos.) | 8 | | | 8 | | | | | |
| SS-A/Ro52 (% of ENA-4 pos.) | 35 | | | 35 | | | | | |
| SS-B/Ro60( % of ENA-4 pos.) | 10 | | | 10 | | | | | |
| Nierebeteiligung% | 26.4 | | | 34 | | | | | |

### Beispiel 2: Antigen Herstellung

Fünf cDNA Bibliotheken, die aus verschiedenen humanen Geweben (fötales Gehirn, Darm, Lunge, Leber und T Zellen)erzeugt worden waren, wurden für die Produktion der rekombinanten Antigene verwendet. Alle cDNAs wurden in E.coli unter der transkriptionellen Kontrolle des Lactoseinduzierbaren Promotors exprimiert. Die resultierenden Proteine tragen an ihrem Aminoterminus eine zusätzliche Sequenz für einen Hexahistidin-Aufreinigungstag (His6-Tag). Targetantigene, die nicht in der cDNA Biobliothek vorhanden waren, wurden durch chemische Synthese (Life Technologies) erzeugt und in den Expressionsvektor pQE30-NST, der bereits einen Aminoterminalen His6-Tag kodiert, kloniert.

Nach rekombinanter Expression der Proteine wurden diese unter denaturierenden Bedingungen isoliert und mittels Metallaffinitätschromatographie (IMAC) aufgereinigt. Bis zur weiteren Verwendung wurden die Proteine lyophilisiert und bei 20°C gelagert (http://www.lifesciences.sourcebioscience.com).

### Beispiel 3: Herstellung der BBAs

Die Herstellung von BBAs wurde auf ein Mikrotiterplatten-Format adaptiert, so dass 384 Kopplungsreaktionen parallel mit Pipettierautomaten (Starlet, Hamilton Robotics, Evo Freedom 150, Tecan) angesetzt werden konnten. Für die Verwendung von Pipettierautomaten wurden die einzelnen Beadregionen in Kopplungsplatten (96 Well Greiner) und die Antigene in 2D Barcode Gefäße (Thermo Scientific) überführt. Für jede Kopplungsreaktion wurden 0.6 bis 2.5 Millionen Beads und abhängig von Antigen 1 bis 100 µg Protein eingesetzt.

Alle Wasch- und Pipettierschritte der Kopplungsreaktion wurden in Kopplungsplatten, die auf Magneten fixiert wurden, durchgeführt. Die Beads wurden zweimal mit 100 µl LxAP-Puffer (100 mM NaH2PO4, pH 6.2) gewaschen und anschließend in 120 µl LxAP-Puffer aufgenommen. Für die Aktivierung wurden 15 µl 1-Ethyl-3-(3-dimethylaminopropyl)carbodiimid (EDC; 50mg/ml) und 15 µl N-Hydroxysulfosuccinimide (Sulfo-NHS; 50mg/ml) zur Bead-Suspension pipettiert und diese wurde anschließend 20 Minuten auf dem Schüttler (RT, 900 rpm, lichtgeschützt) inkubiert. Die Beads wurden 3x mit 150 µl LxKPT-Puffer gewaschen und anschließend die Proteinlösung zugegeben. Nach zweistündiger Inkubation auf dem Schüttler (RT, 900 rpm, lichtgeschützt) wurden die Beads dreimal mit 150 µl LxWPT-Puffer gewaschen. Zur Blockierung von freien Bindungsstellen wurden 100 µl LxCBSP-Puffer (PBS, 1% BSA, 0.05% ProClin300) zugegeben und 20 min auf dem Schüttler inkubiert (RT, 900 rpm, lichtgeschützt). Anschließend folgte eine Inkubation bei 4-8°C über Nacht. Das BBA wurde durch die Vereinigung von mit Antigen gekoppelten Beads hergestellt und bis zur Anwendung bei 4-8°C lichtgeschützt gelagert.

### Beispiel 4: Qualitätskontrolle der BBAs

Zur Überprüfung der Immobilisierung der Proteine an die jeweiligen Beadregionen wurde eine Kopplungskontrolle durchgeführt. Dabei wurden unterschiedliche Mengen an Beads verwendet (250, 500 und 750 Beads pro Beadregion). Für einen Reaktionsansatz wurden z.B. 500 Beads pro Beadregion in LxCBS Puffer (PBS, 1% BSA) verdünnt und in eine Assayplatte (96 Well half area Microplatte, Greiner) überführt.

Vor jedem Waschschritt wurden die Assayplatte mit den Beads für 2 Minuten auf einen Magneten gestellt und dann der Überstand entfernt. Nach drei Waschschritten wurden die Beads mit 100 µl LxWPT-Puffer (PBS, 0.05% Tween-20) aufgenommen und 10 pg/ml Penta-His-Antikörper (Qiagen) bzw. LxCBS-Puffer (PBS, 1% BSA) pipettiert. Nach einer 45 minütigen Inkubation auf dem Schüttler (RT, 900 rpm, lichtgeschützt) wurde der Überstand entfernt und die Beads mit in zwei Schritten gewaschen. Anschließend wurden jeweils 5 µg/ml Ziege-anti-Maus IgG-PE (*Phycoerythrin*) bzw. Ziege-anti-human IgG-PE (Dianova) als sekundärer Antikörper zum Reaktionsansatz gegeben und für 30 Minuten inkubiert. Nach zwei Waschschritten wurden die Beads mit 100 µl Trägerflüssigkeit (Luminex) versetzt. Das Fluoreszenzsignal der Beads wurde mit Hilfe des FlexMAP3D Instruments detektiert. Dabei wurden zum einen der Beadcount und zum anderen der Median der Fluoreszenzintensität (MFI-Wert) gemessen.

### Beispiel 5: Anwendung von BBAs

Für die Anwendung wurden BBAs mit Seren inkubiert und alle an Antigene gebunden IgG-basierte Autoantikörper wurden mit Hilfe eines sekundären Antikörper detektiert. Um einen hohen Durchsatz an Messungen zu ermöglichen, wurde die Anwendung von BBAs auf ein Mikrotiterplatten-Format adaptiert, so dass entweder ein 8-Kanal (Starlet, Hamilton Robotics) oder ein 96-Kanal (Evo Freedom 150, Tecan) Pipettierautomat verwendet werden konnte. Die zu untersuchenden Seren wurden in 2D Barcode Gefäße überführt und anschließend 1:100 mit Assaypuffer (PBS, 0.5% BSA, 10% E.coli Lysate, 50% Low-Cross Buffer (Candor Technologies)) verdünnt. Um humane Antikörper, die gegen E.coli Proteine gerichtet sind, zu neutralisieren, erfolgte eine Präinkubation der Serenverdünnungen für 20 min. In dieser Zeit wurden 500 Beads pro Beadregion in die Assayplatte verteilt. In die Kopplungsplatte wurden 50 µl verdünntes Serum zu den Beads gegeben und die Reaktionsansätze 18-22 h auf dem Schüttler inkubiert (4-8 °C, 900 rpm, lichtgeschützt) . Nach drei Waschschritte mit jeweils 100 µl LxWPT-Puffer wurden 5 µg/ml des Detektionsantikörpers Ziege-anti-human IgG-PE (Dianova) zu den Reaktionsansätzen gegeben und für 1 h auf dem Schüttler inkubiert (RT, 900 rpm). Anschließenden wurden die Beads dreimal mit 100 µl LxWPT gewaschen und in 100 µl Trägerflüssigkeit (Luminex) aufgenommen. Das Fluoreszenzsignal der Beads wurde mit Hilfe des FlexMAP3D Instruments detektiert. Dabei wurden zum einen der Beadcount und zum anderen der MFI-Wert (Median der Fluoreszenzintensitität) gemessen.

### Beispiel 6: Biostatistische Analyse

Die biostatistische Analyse umfasste univariate und multivariate Methoden um die statistischen Eigenschaften einzelner Antigene sowie von Gruppen von Antigenen zu beschreiben. Für das Auffinden von interessanten Kandidaten für Panels war die entscheidende Eigenschaft eine gute Trennung zwischen den Gruppen von Proben auf Basis der MFI-Werte. Um Antigen-Kandidaten für eine Panel-Generierung zu finden, wurden als Methoden univariates Testen, Receiver Operating Characteristic (ROC) Analysen, Korrelationsprofile, Powered Partial Least Squares Diskriminanzanalyse (PPLS-DA) und Zufallswälder angewendet. Biostatistische Anlysen wurden einer Expertenbewertung unterzogen um finale Antigen-Panels zu definieren.

Vor der statistischen Analyse wurden die MFI-Werte log2-transformiert um die Schiefe in den Verteilungen zu reduzieren. Falls mehr als 20% der Werte fehlten, wurden Antigene von der Analyse ausgeschlossen. Fehlende Werte wurden durch Median-Imputation ersetzt. Eine Quantil-Normalisierung unter Berücksichtigung der Referenzseren wurde durchgeführt, um pro BBA-Set alle gemessenen Proben auf individuellen Platten zu normalisieren.

Neben deskriptiven Standardstatistiken für MFI-Werte wurden mit Hilfe des zweiseitigen Mann-Whitney-U-Tests nichtparametrische Tests durchgeführt um Unterschiede in den Medianwerten der Gruppen zu entdecken. Korrektur des Testniveaus für multiples Testen erfolgte nach der Bonferroni-Holm Prozedur. Außerdem wurde die Benjamini-Hochberg Prozedur inklusive der Bestimmung der False Discovery Rate (FDR, q-Wert) angewendet. Zusätzlich wurden Fold-Change und Effektgröße bestimmt. Um die Klassifikationsgüte zu bewerten wurde eine ROC-Analyse durchgeführt, in deren Rahmen Sensitivität, Spezifität und die Fläche unter der ROC-Kurve (AUC) berechnet wurden, jeweils inklusive der 95% Konfidenzintervalle auf Basis des Bootstrap-Verfahrens. Zur grafischen Darstellung wurden Boxplots und Volcanoplots genutzt. Auf Basis der univariaten Ergebnisse wurde ein Scoring-System implementiert.

Durch die Anwendung einer PPLS-DA wurde versucht, die Korrelation zwischen den Komponenten und der Response-Matrix zu maximieren. Für die finale Klassifizierung wurde eine lineare Diskriminanzanalyse mit den latenten Komponenten als Prädiktoren genutzt. Ein Zufallswald wurde angewendet, in dem binäre Entscheidungsbäume kombiniert werden. Die Entscheidungsbäume wurden gebildet auf Basis mehrerer Bootstrap-Stichproben einer Lernstichprobe und durch zufälliges Auswählen einer Subgruppe von erklärenden Variablen an jedem Knoten. Die Anzahl der Eingangsvariablen, die an jedem Teilungsschritt zufällig gewählt wurde, wurde als die Quadratwurzel der Gesamtzahl der Variablen festgelegt, und die Anzahl der Bäume im Zufallswald wurde auf 1000 gesetzt. Für beide multivariaten Ansätze wurde eine Kreuzvalidierung mit 500 fachem Durchlauf implementiert.

### Beispiel 7: Autoantikörper/ Antigenreaktivitäten differenzieren SLE von Gesundkontrollen, Rheumatoider Arthritis und anderen Autoimmunerkrankungen

In einem ersten Screening wurden die Antigenreaktivitäten von 129 SLE Patienten, 75 RA Patienten, und 134 nach Alter und Geschlecht zugeordnete Gesundkontrollen wurden differentiell getestet. Dazu wurden die Autoantikörperreaktivitäten dieser Blutproben an 5857 an Luminex-Beads gekoppelten Antigenen getestet.

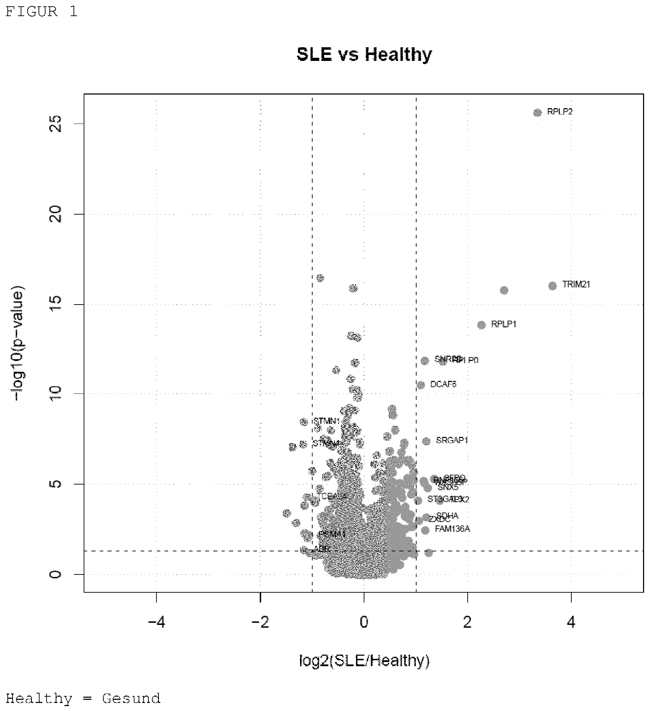

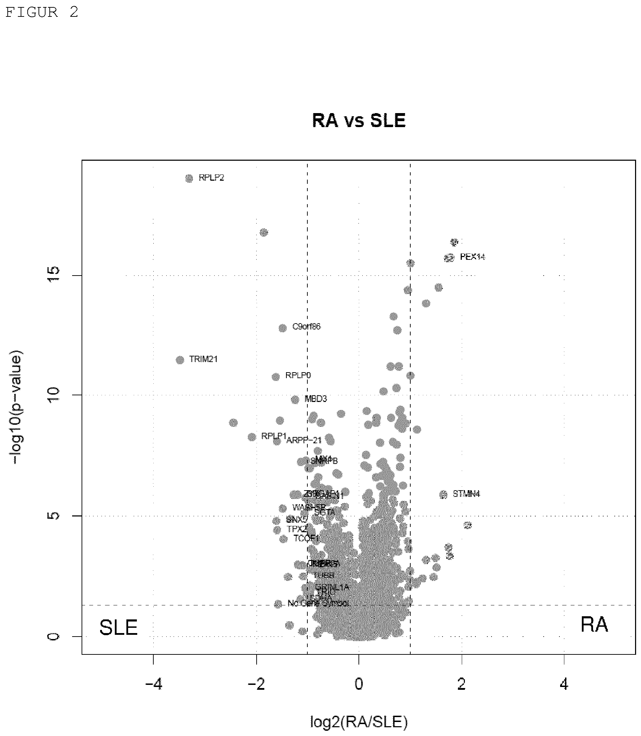

Um Antigene zu identifizieren, mit denen die Gruppe aller SLE Patienten von verschiedenen Kontrollgruppen bestehend aus Gesundproben und Patienten mit RA unterschieden werden kann, wurden univariate statistische Tests durchgeführt. Das Ergebnis der statistischen Tests ist als Vulcano Plot für alle 5857 Antigene dargestellt. Im Vulcano Plot wird auf der x-Achse die relative Änderung der Antigenreaktivtät in SLE Patienten im Vergleich zu Gesundkontrollen (*Figur 1*) und RA Patienten (*Figur 2*) dargestellt. Die Y-Achse gibt den p-Wert des statistischen Tests wieder. Figuren 1 und 2 zeigen, dass spezifische Autoantikörperreaktivitäten gefunden wurden, die in der Gruppe aller SLE erhöht sind und diese sowohl von gesunden Spendern als auch von RA Patienten unterscheiden können.

### Beispiel 8: Autoantikörper/ Antigenreaktivitäten differenzieren SLE von Gesundkontrollen, früher Rheumatoider Arthritis und anderen Autoimmunerkrankungen

In einem zweiten Screening mit 6088 Antigenen wurden die Antigene, die zwischen Gesundkontrollen und Spendern mit rheumatoider Arthritis differenzieren an Patienten mit früher rheumatoider Arthritis, SSc und SPA getestet. Dies ist insbesondere von Bedeutung, da Patienten mit Kollagenosen und Mischkollagenosen ein überlappendes Autoantikörperprofil aufweisen und daher besonders in der frühen Phase schwer zu diagnostizieren sind.

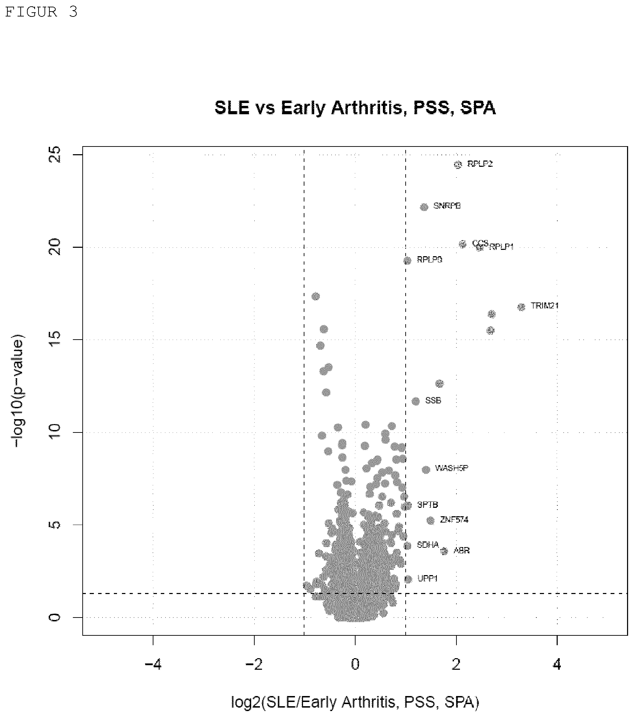

*Figur 3* zeigt einen Vulcano Plot der Antigenreaktivitäten von SLE Patienten gegen eine vereinigte Gruppe von Patienten mit verschiedenen Autoimmunerkrankungen wie SSc, SPA, frühere Rheumatoide Arthritis und SPA.

Nach univariater statistischer Auswertung wurde ein Schwellenwert von p<0.05 und eine 1,5 fach veränderte Reaktivität gegenüber der Kontrollgruppe angelegt. Eine finale Liste von Antigenreaktivitäten über beide Screens wurde erstellt (Tabelle 2).

Um die Frequenz der neuidentifizierten Antigene im Vergleich zu bekannten Antigenen zu analysieren, wurde ein Schwellenwert von 3 Standardabweichungen (SD) über dem Mittelwert der Gesundproben definiert.

Erstaunlicherweise wurden mindestens 4 zusätzliche Antigene identifiziert, deren Frequenz in SLE Patienten über 15% liegt. Dazu gehören TMPO (19%) (SEQ ID No. 18), HNRNPA1 (26%) (SEQ ID No. 5), XRCC5 (15%) (SEQ ID No. 22) und MVP (15%) (SEQ ID No. 7) .

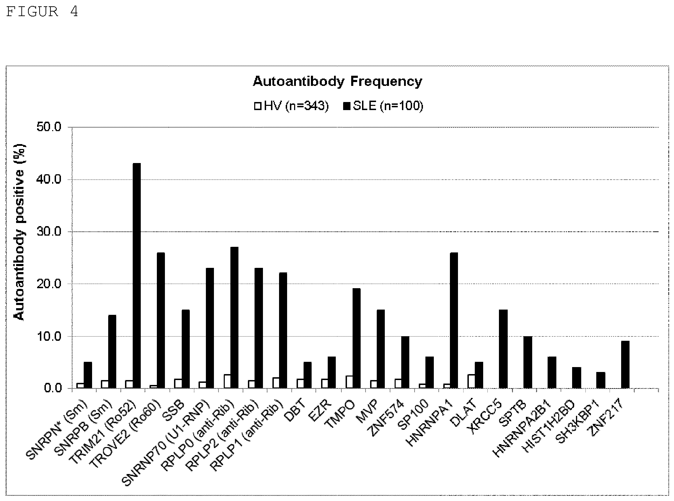

*Figur 4* zeigt die Frequenz von 23 Antigenen im Vergleich zur Gesundkontrolle.

Tabelle 2 fasst die identifizierten Antigenreaktivtäten und verschiedenen Gruppenvergleiche zusammen.

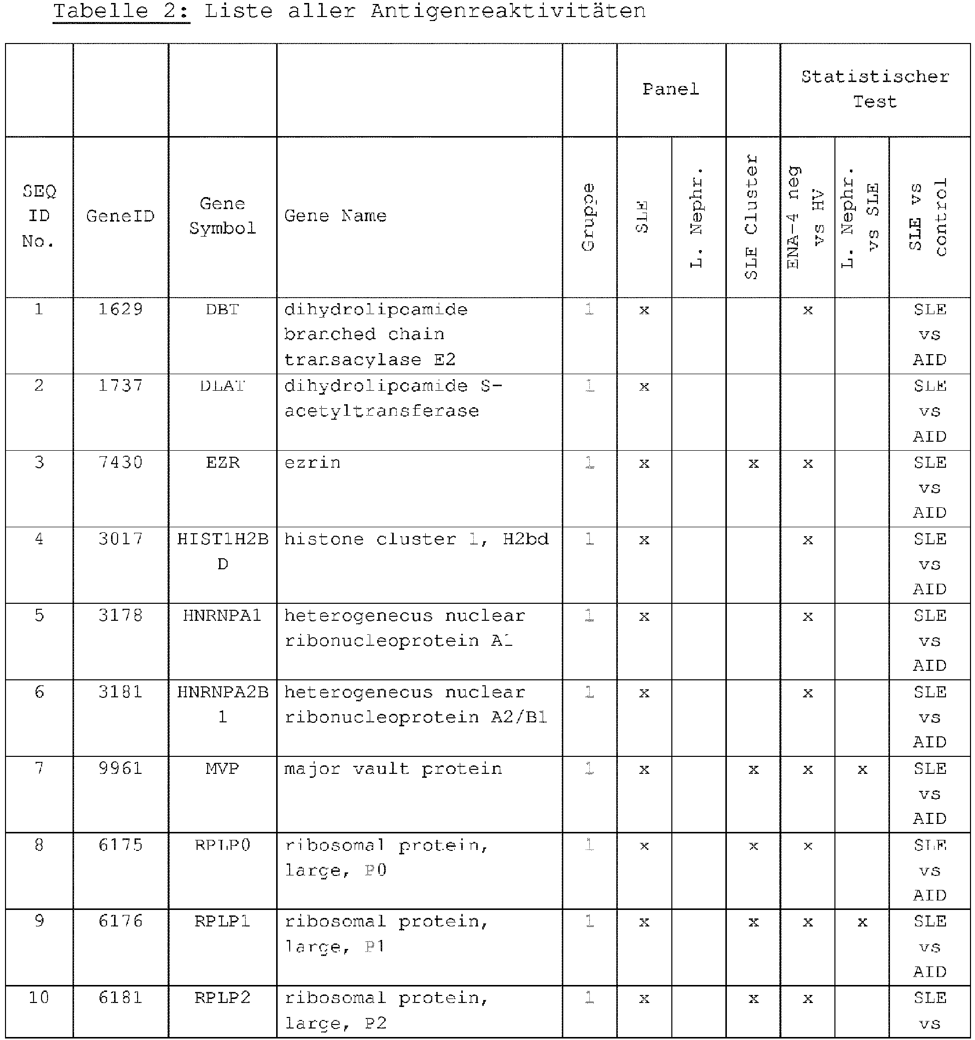

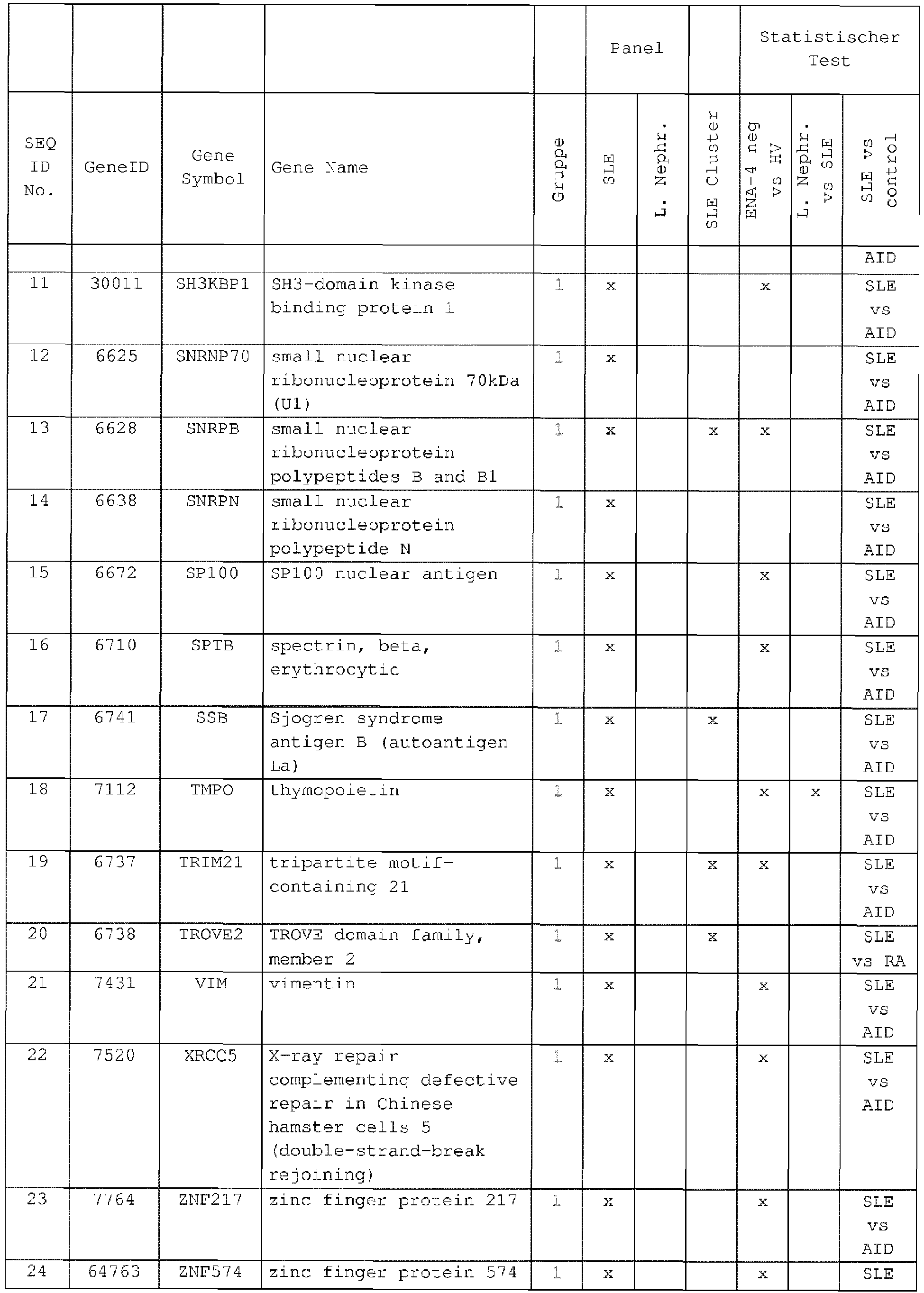

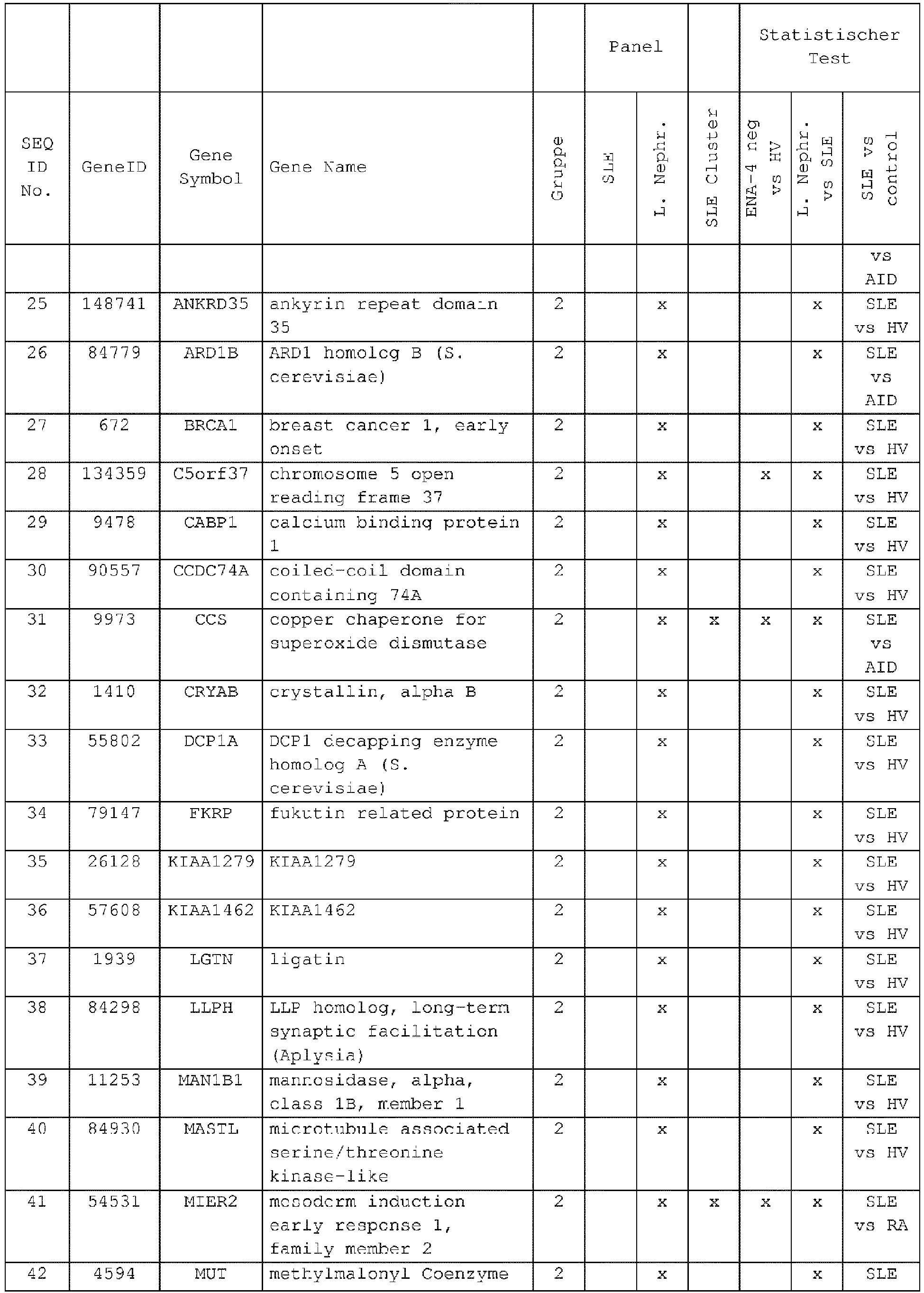

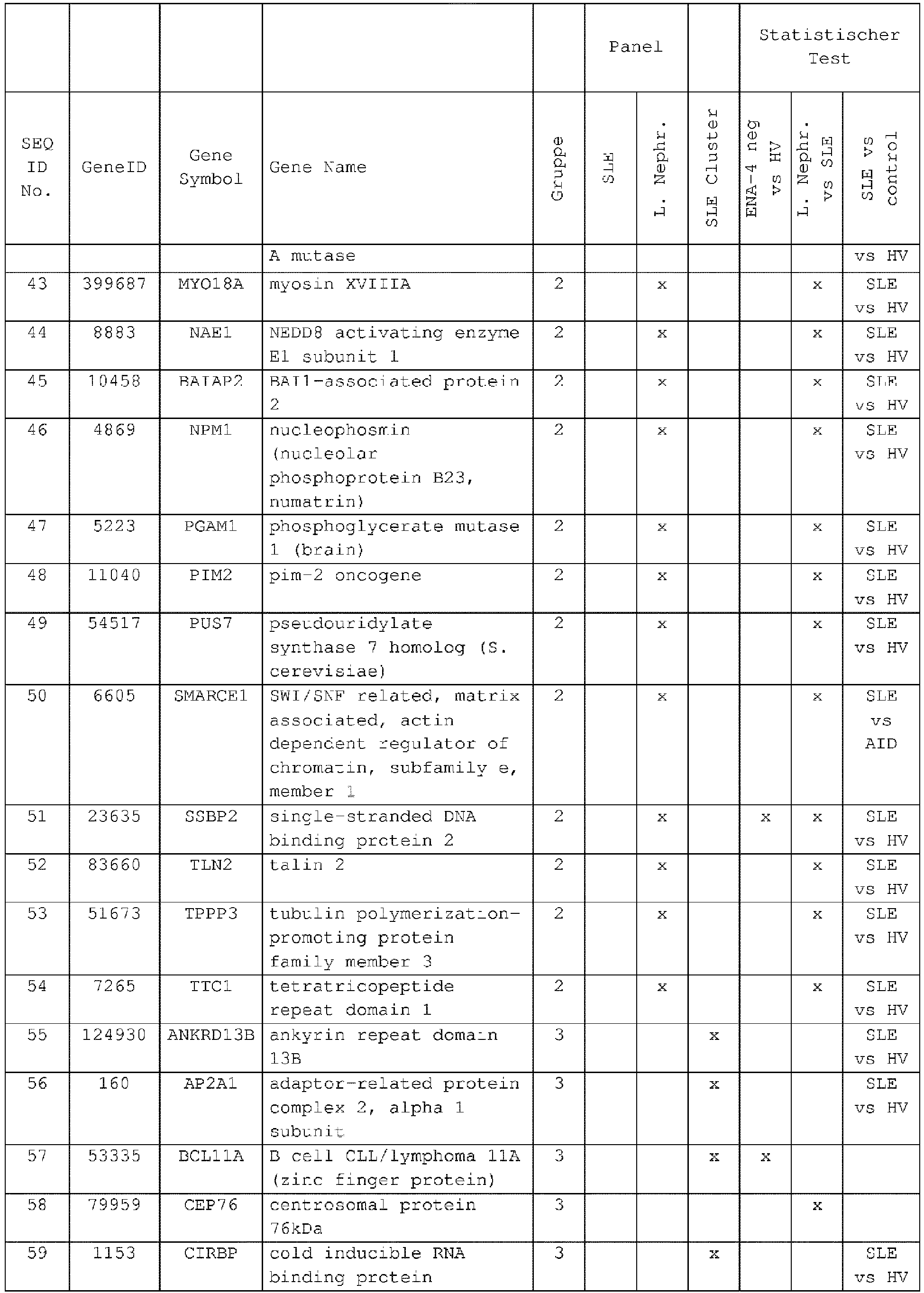

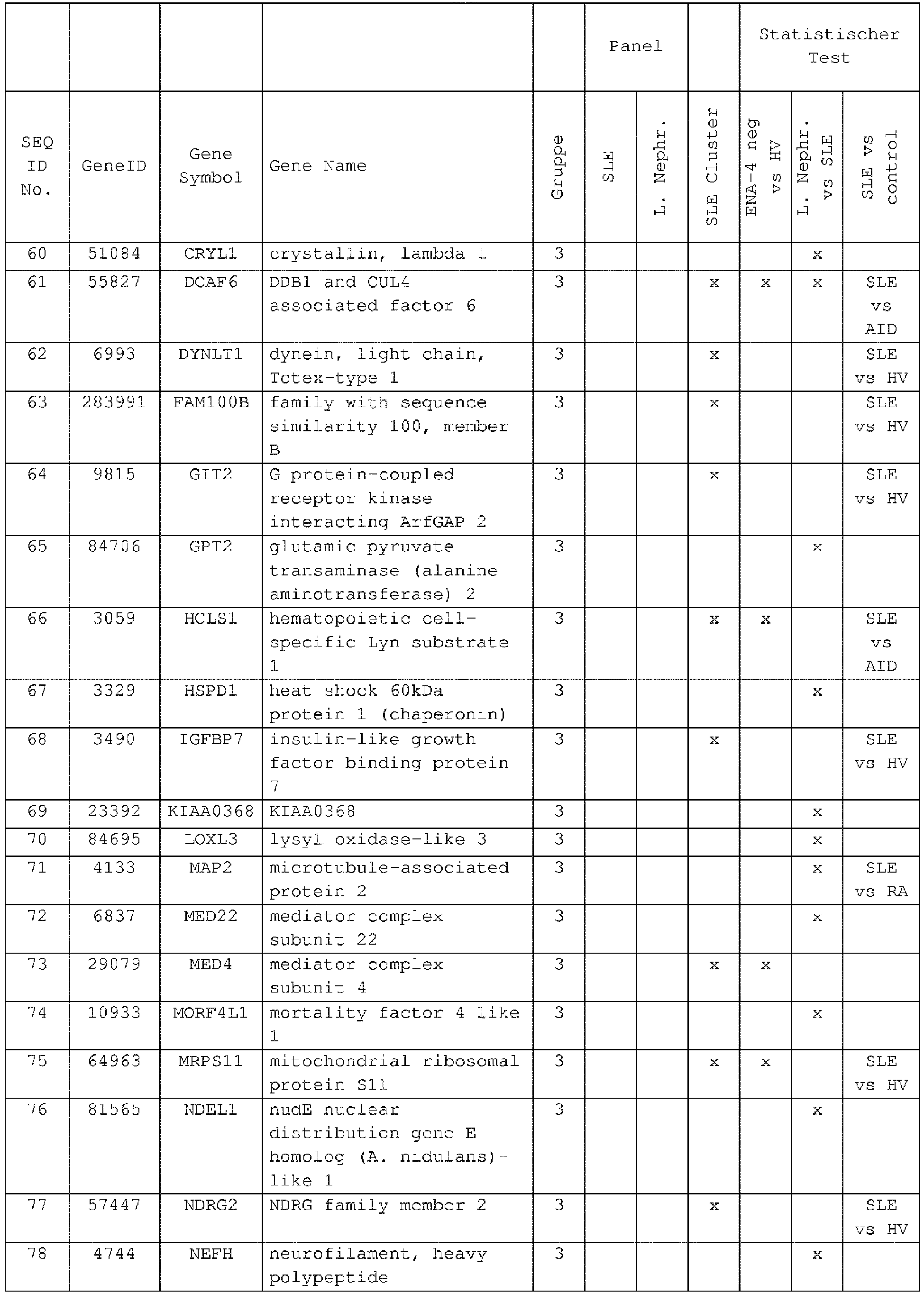

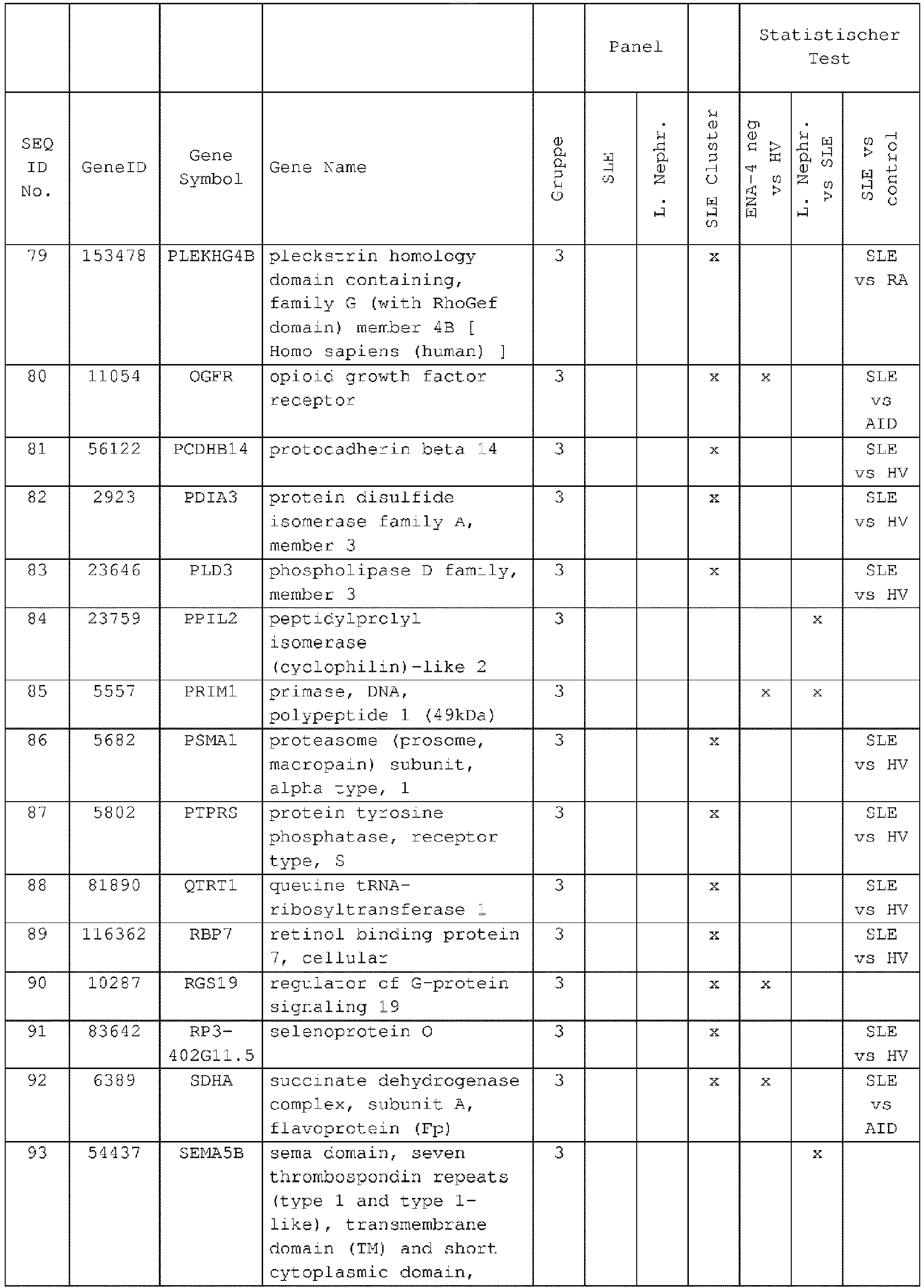

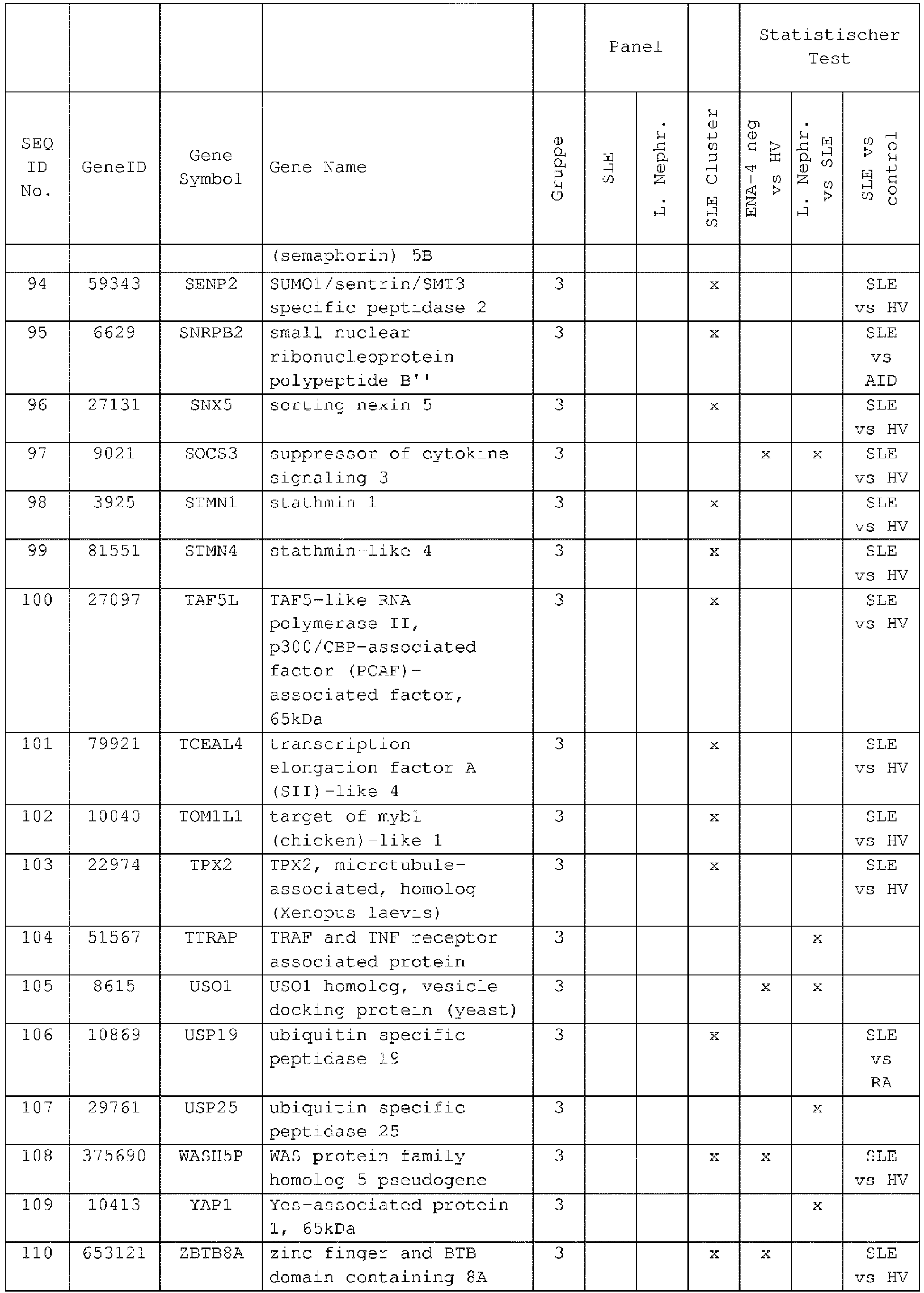

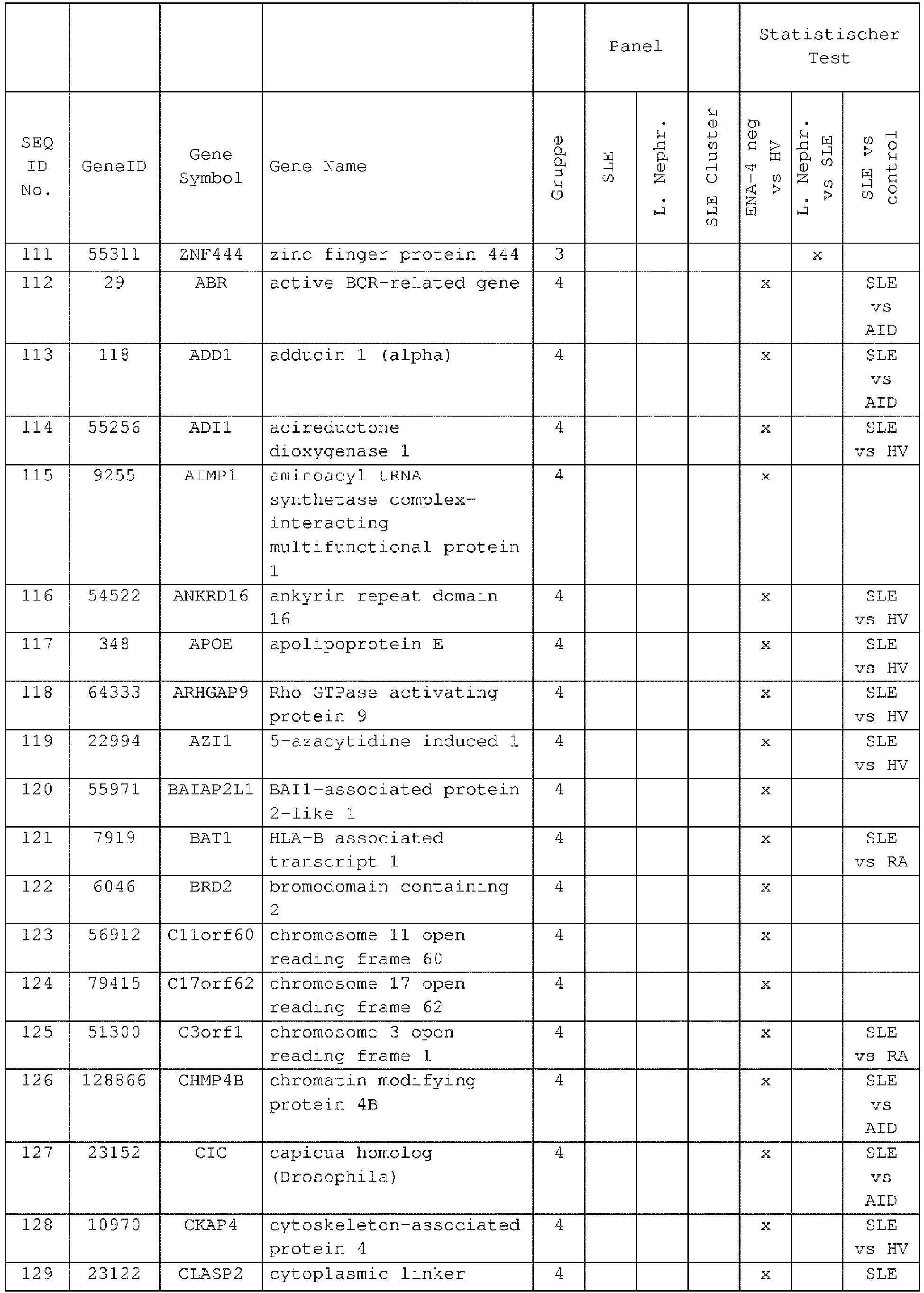

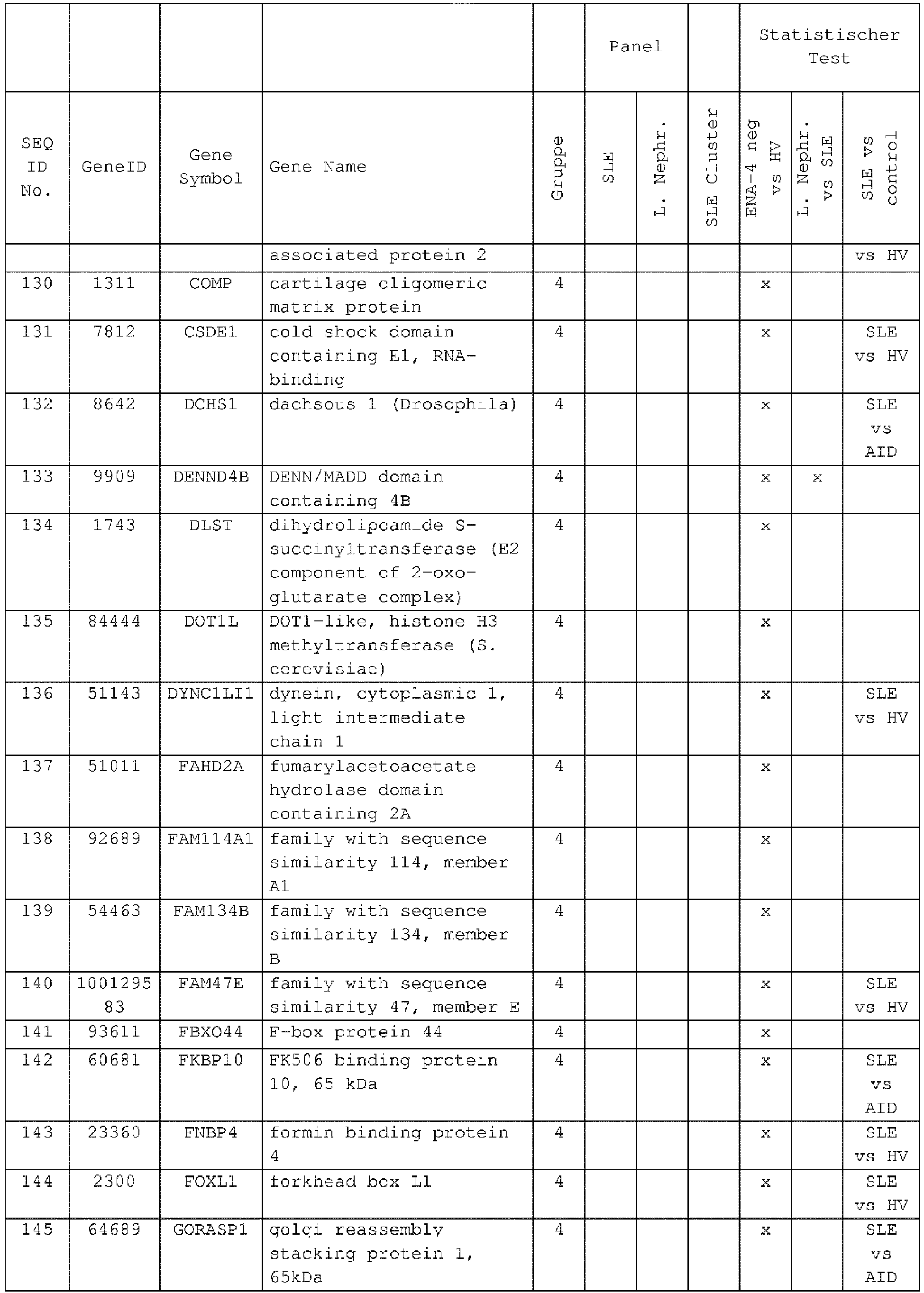

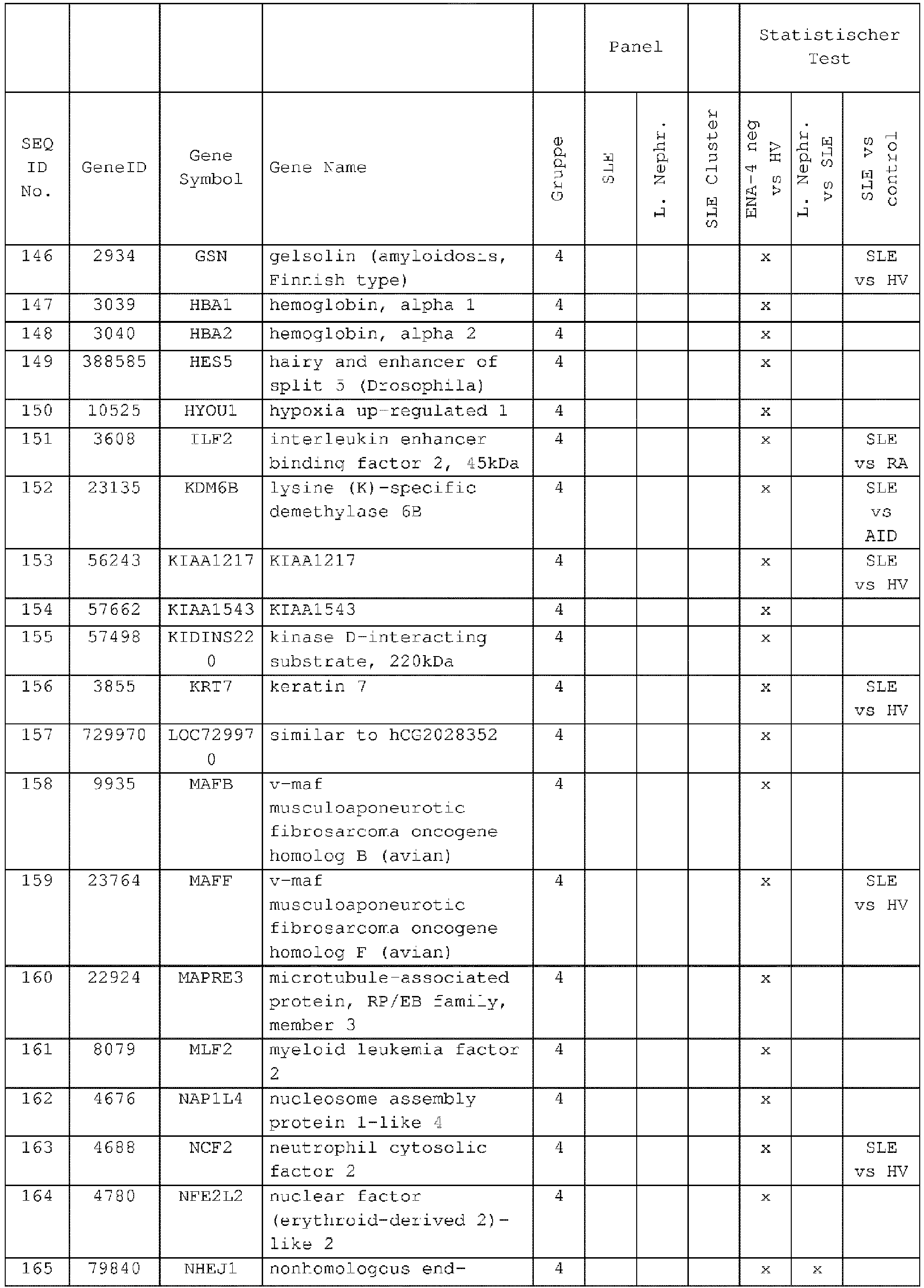

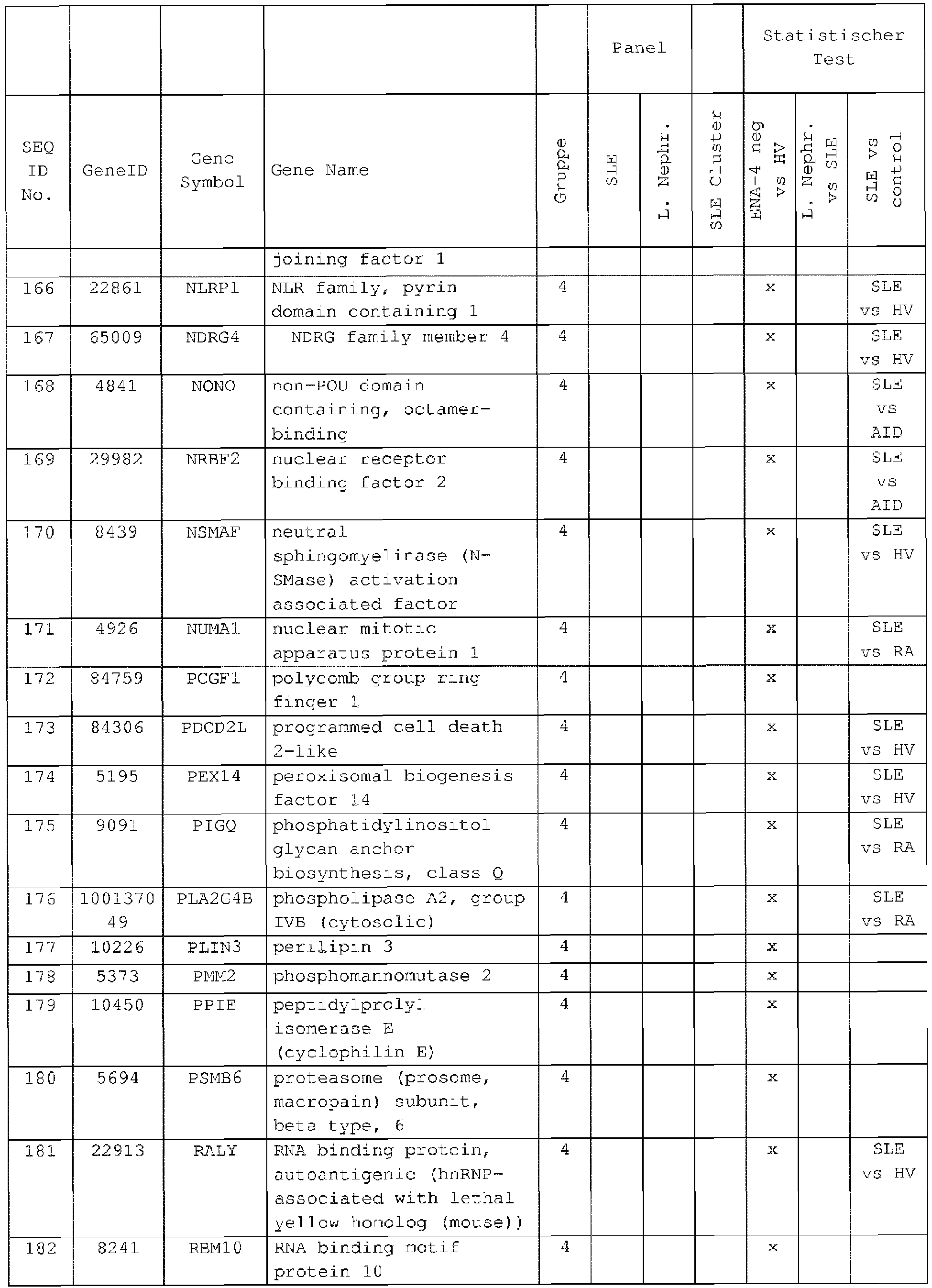

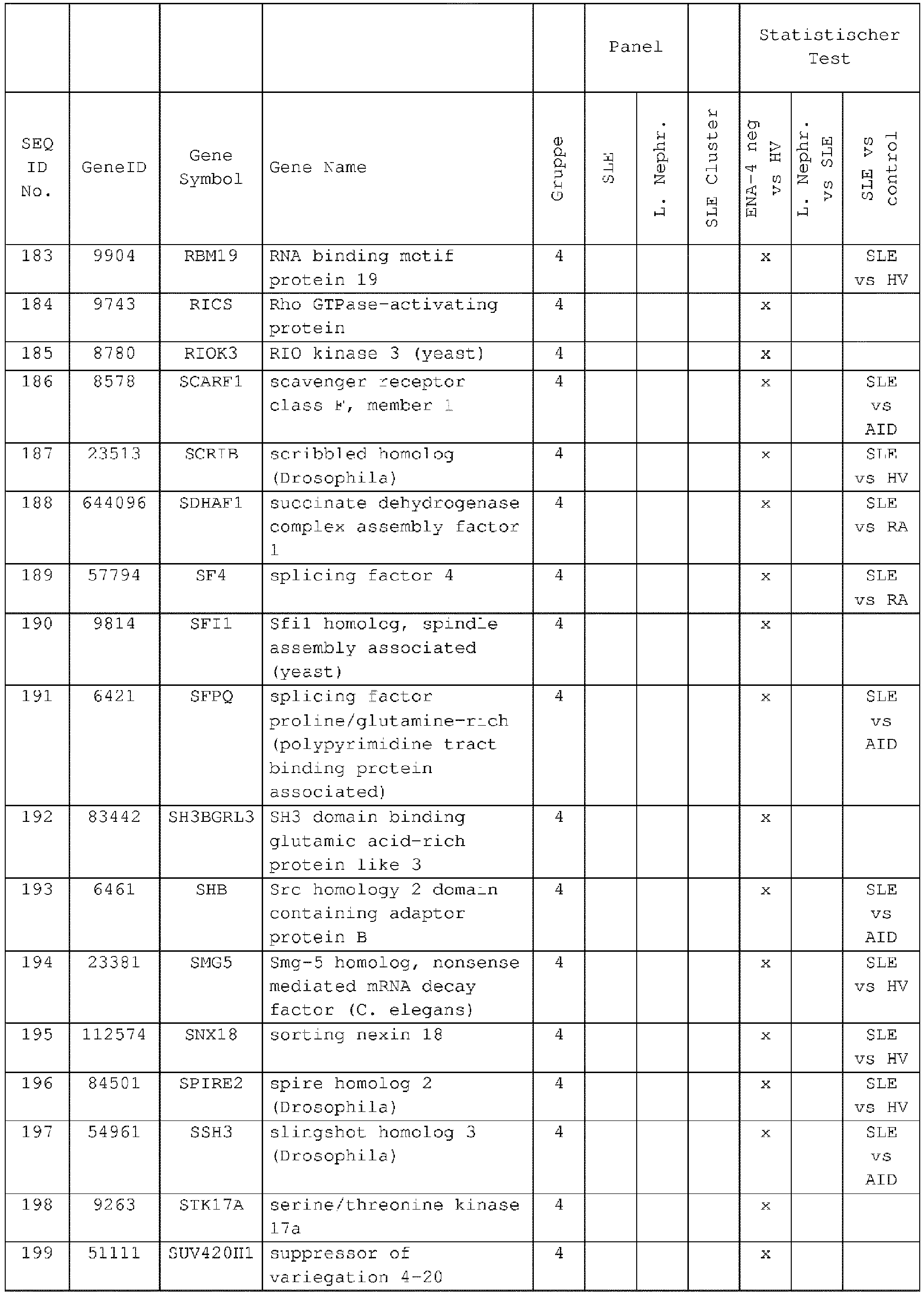

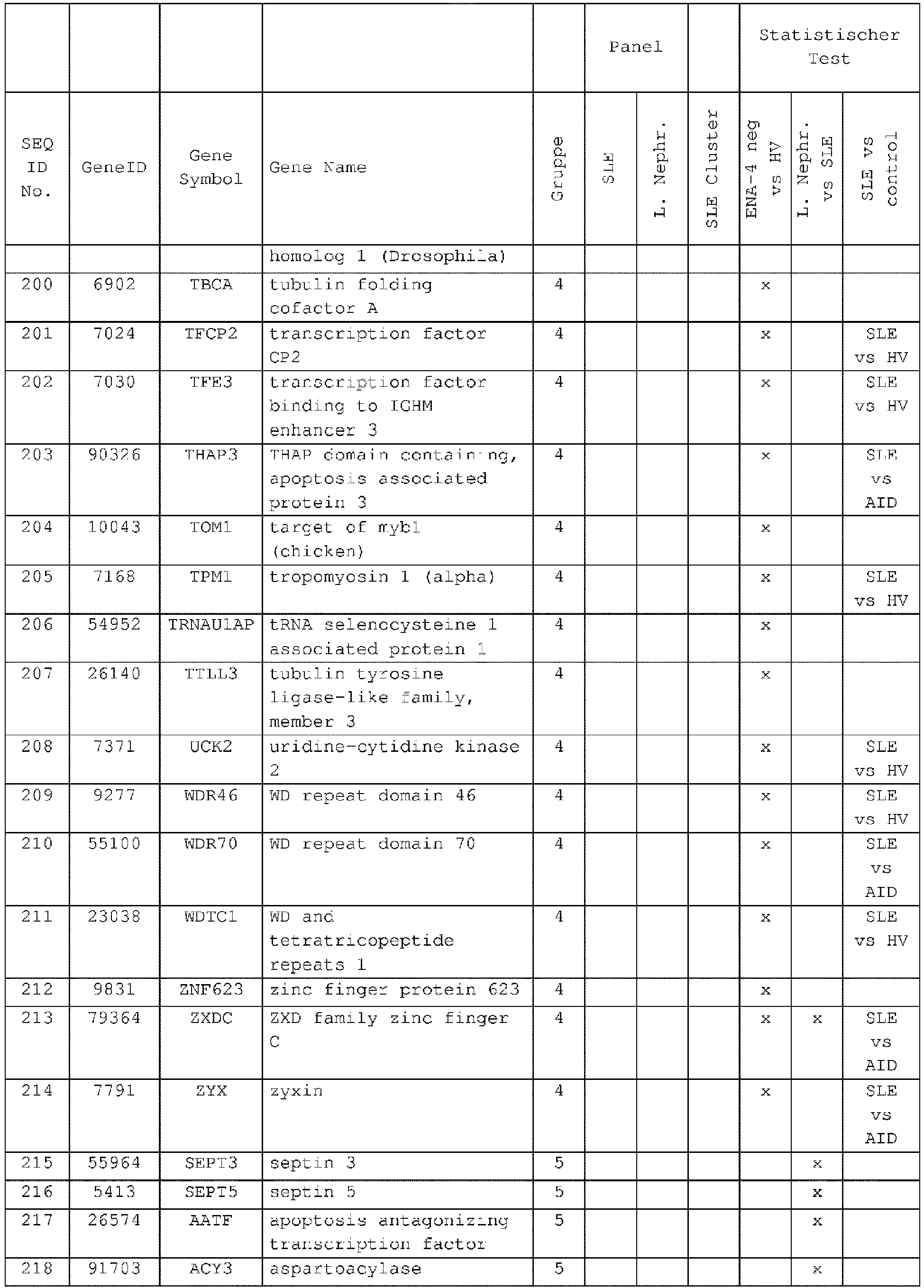

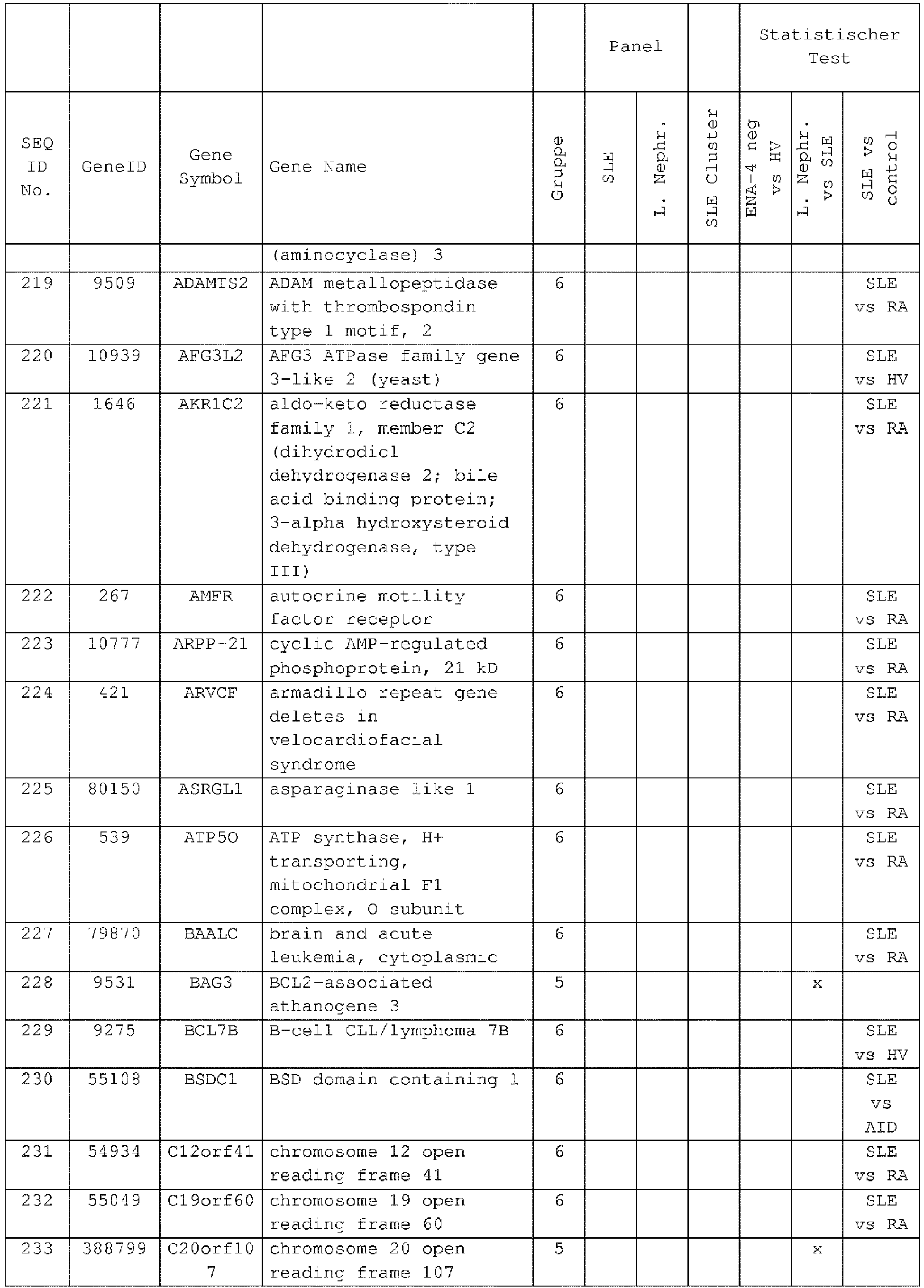

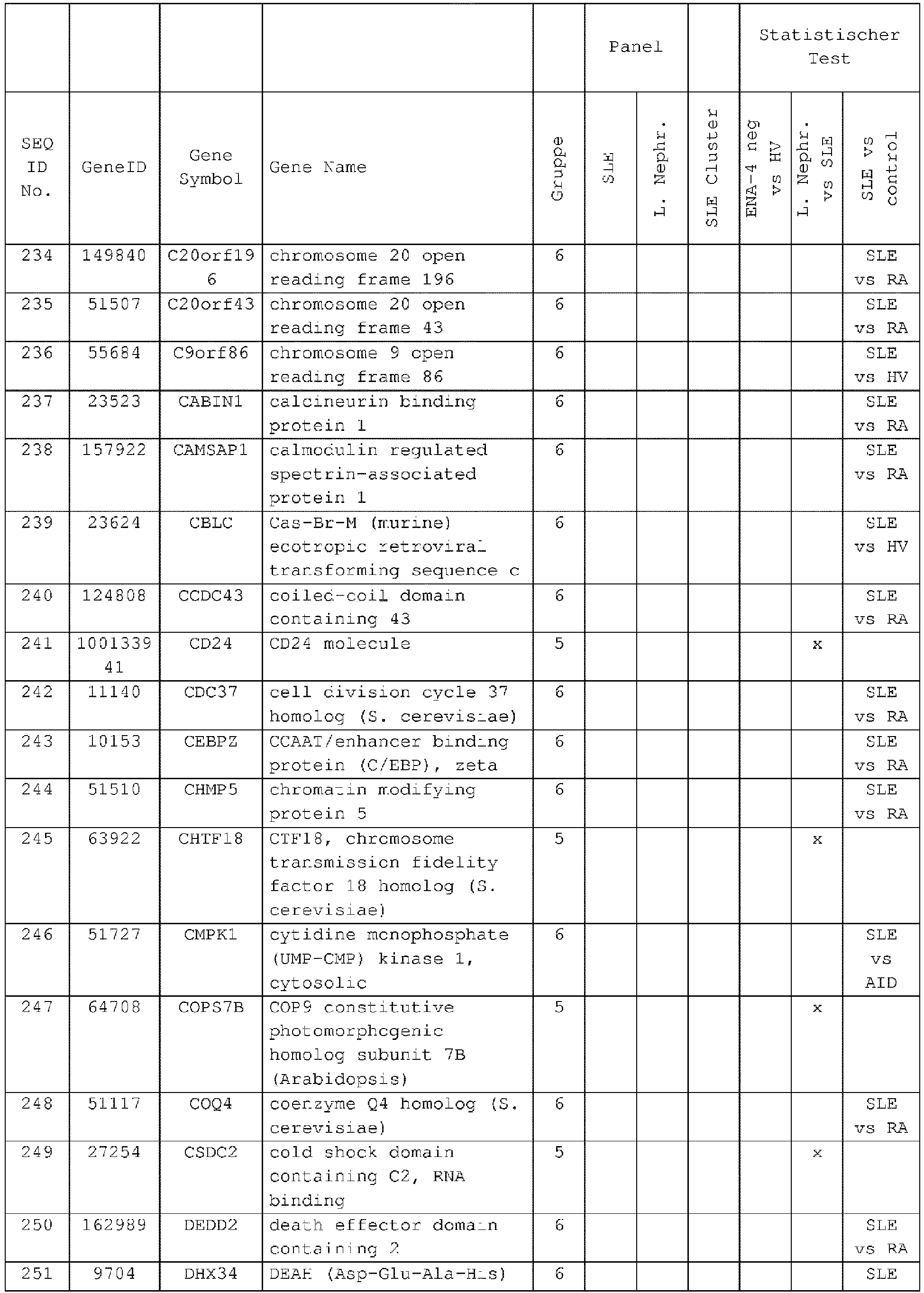

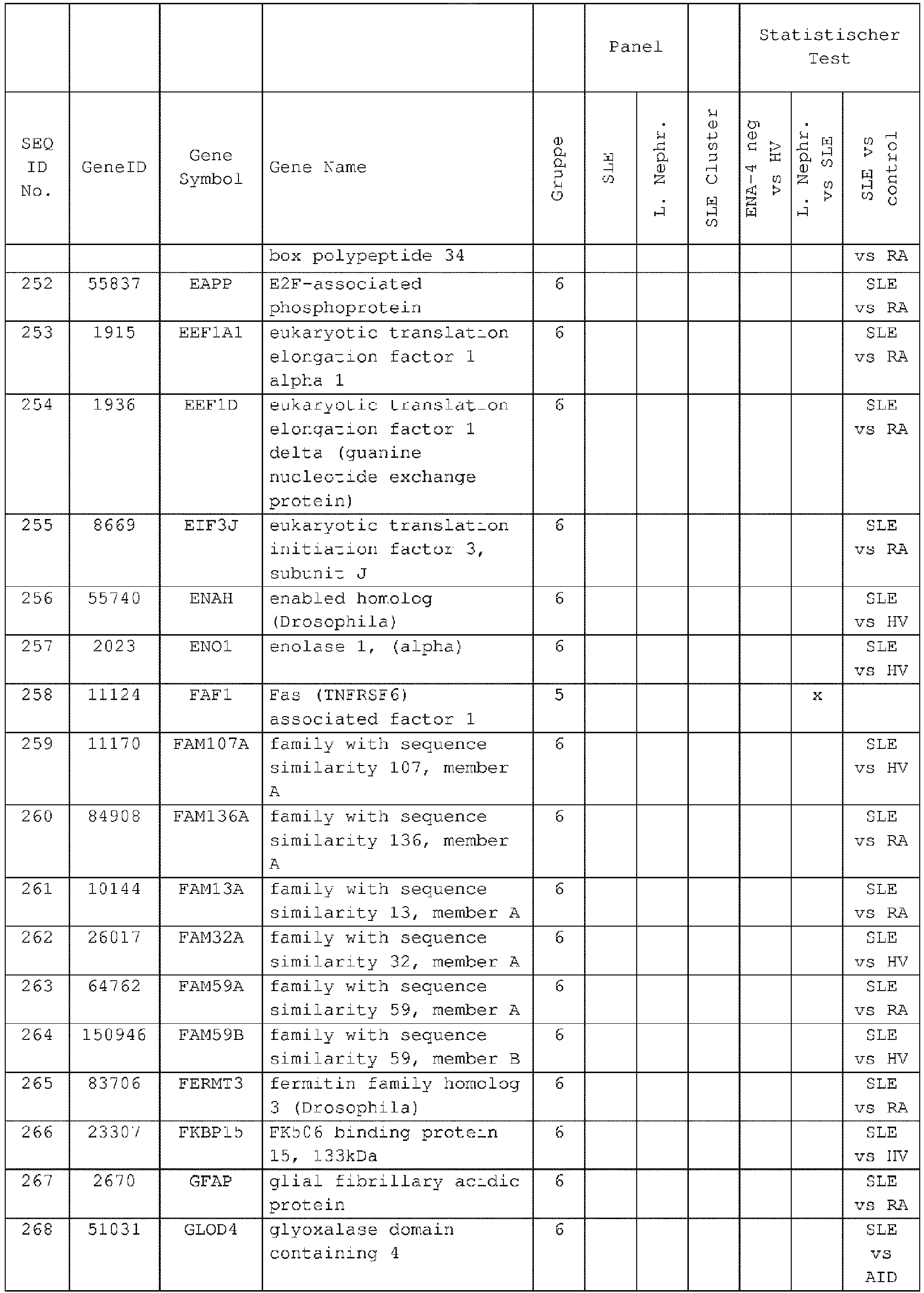

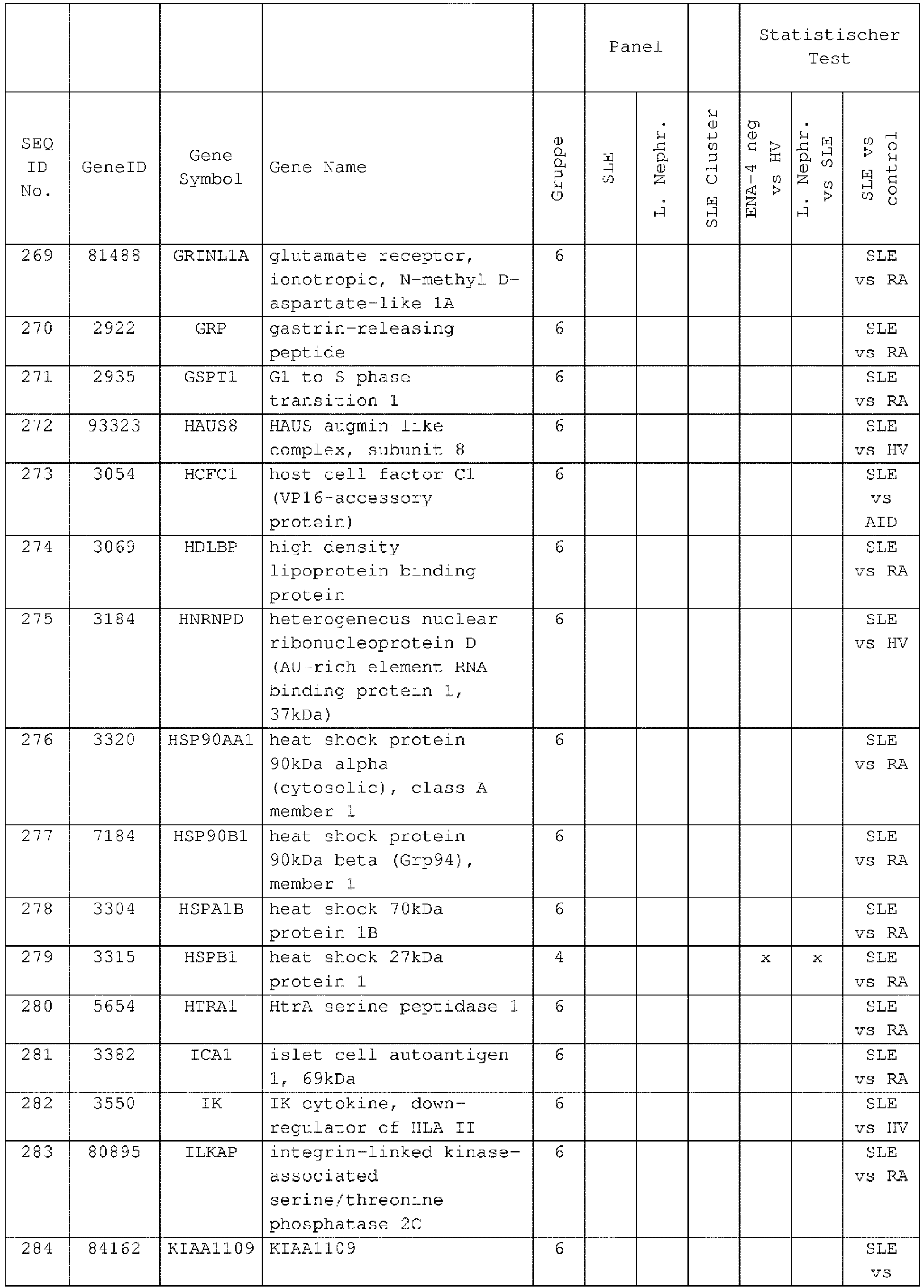

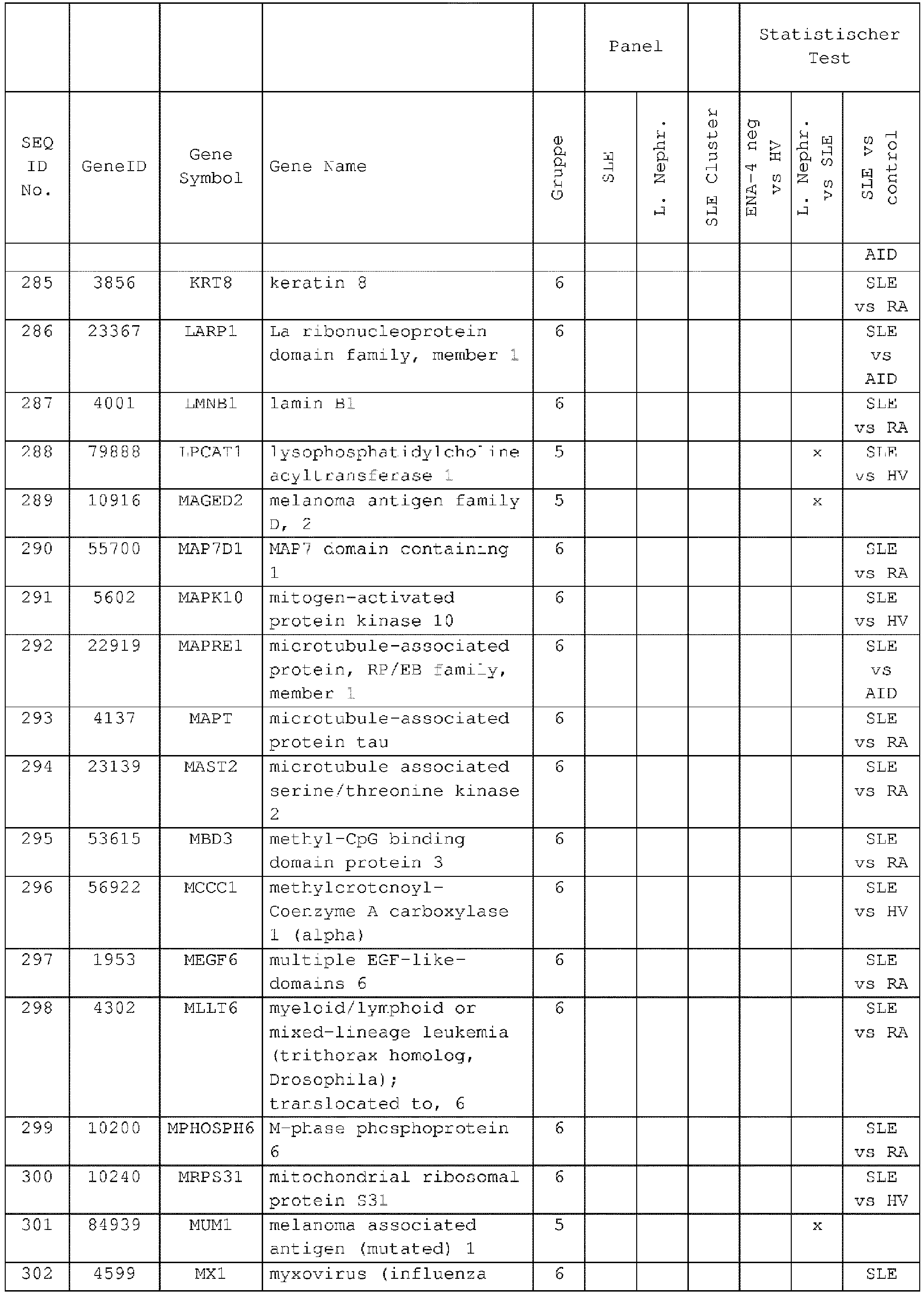

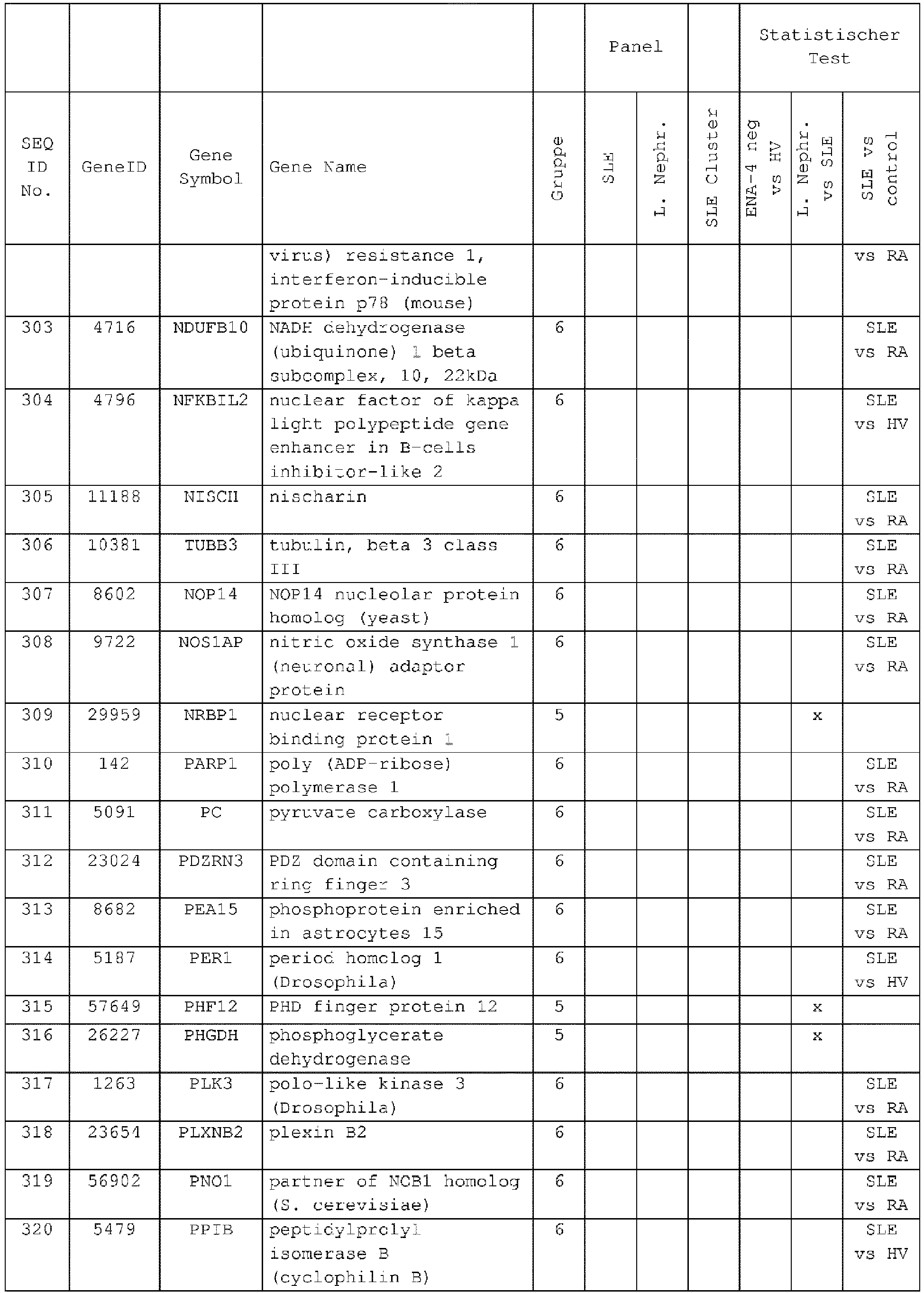

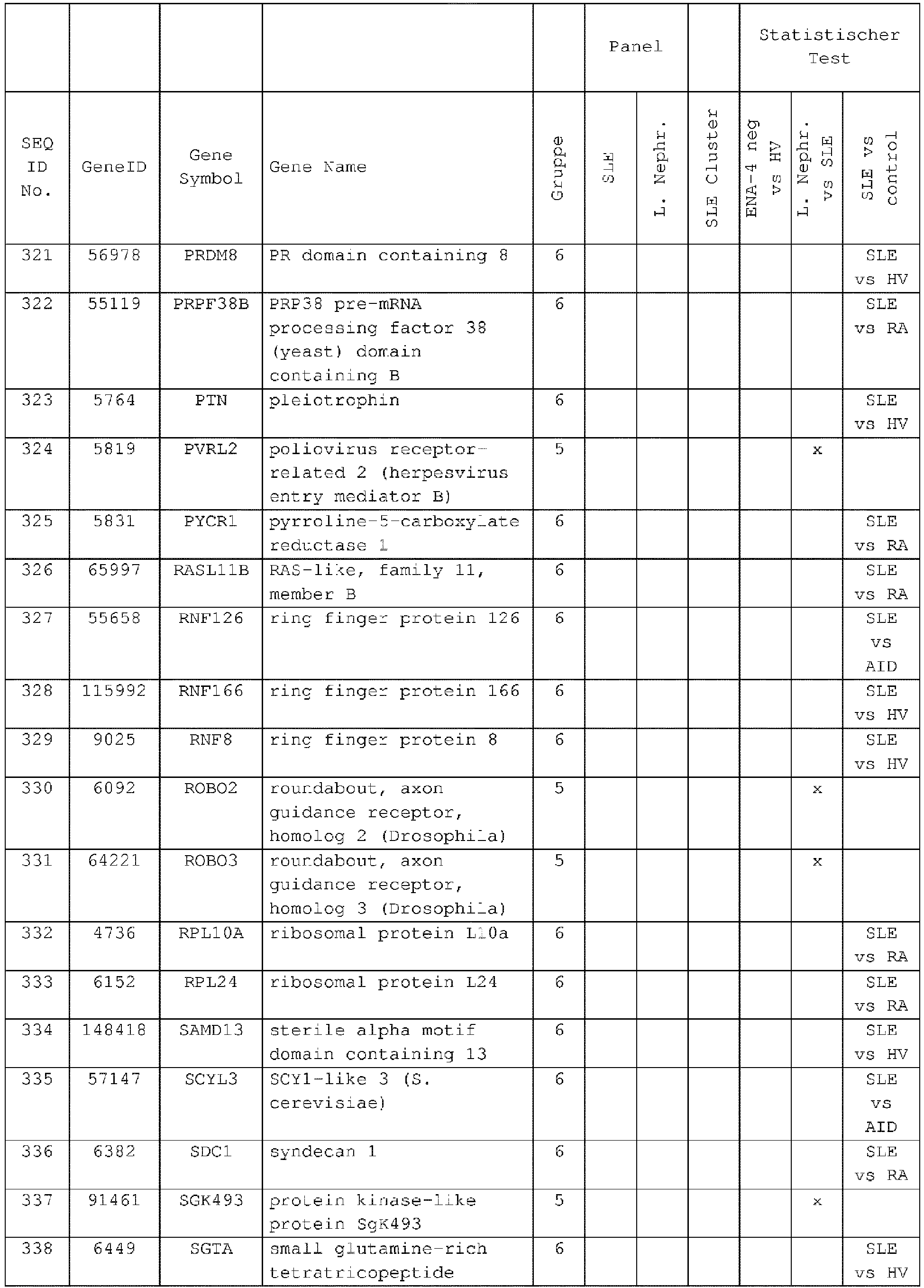

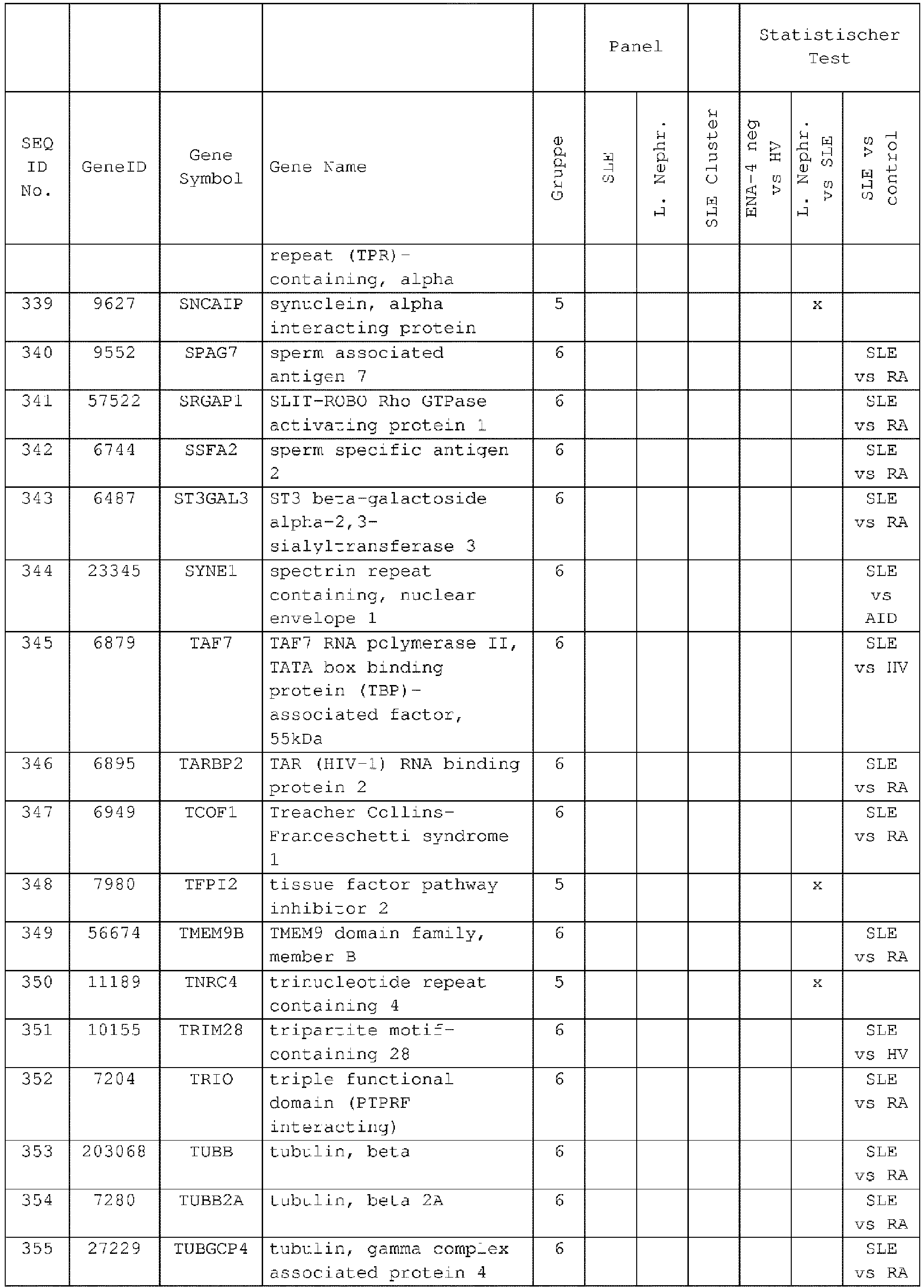

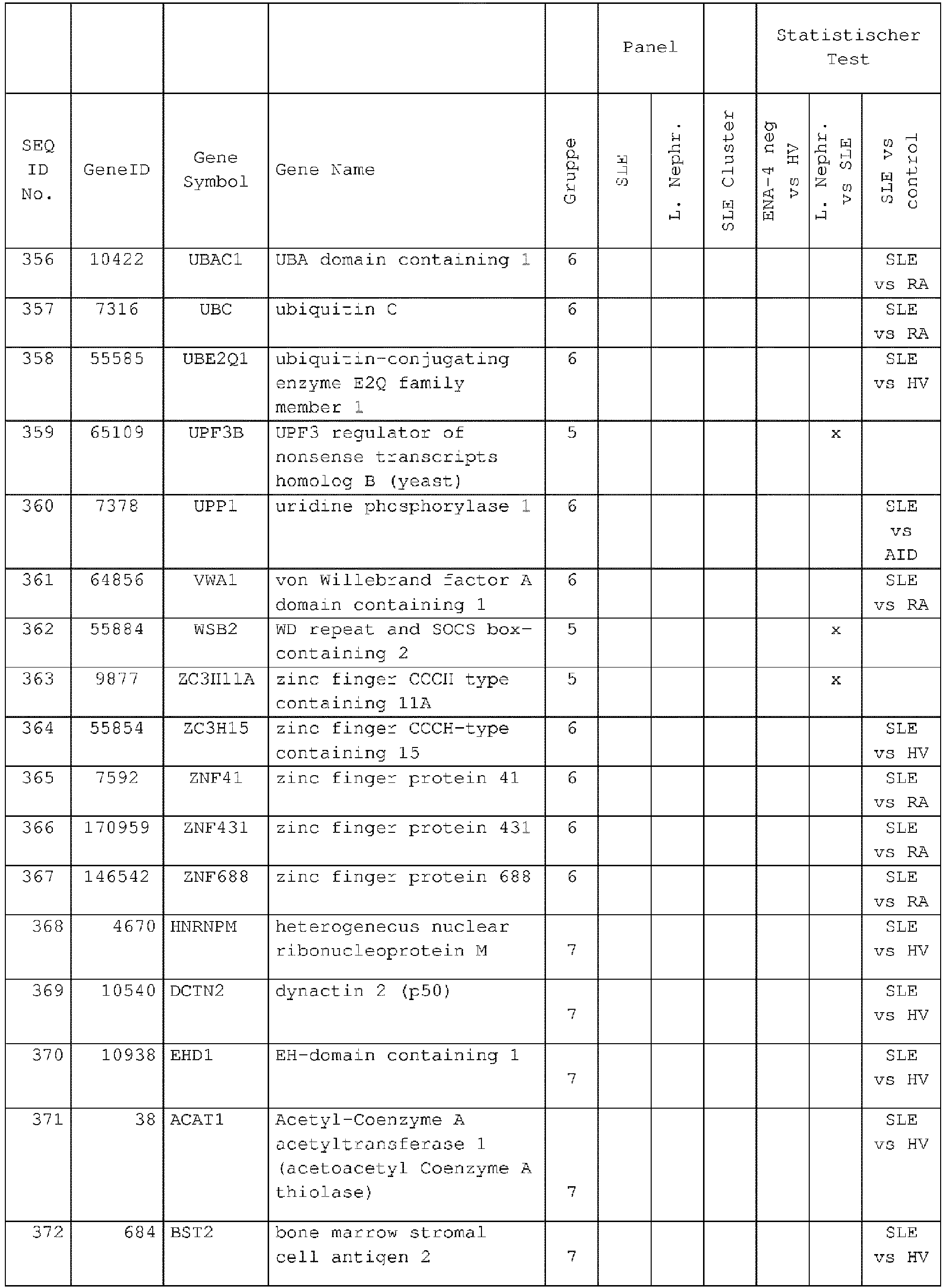

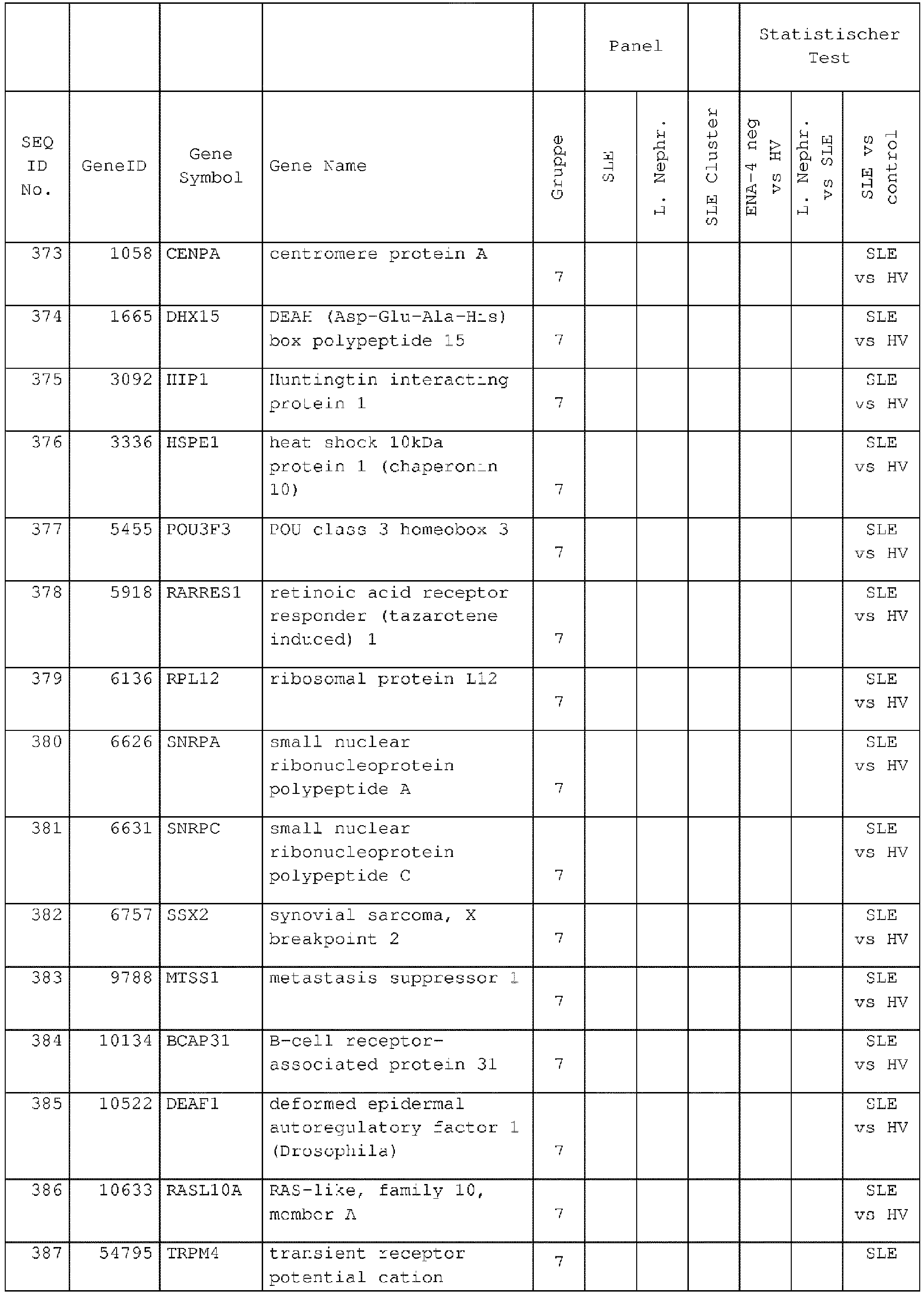

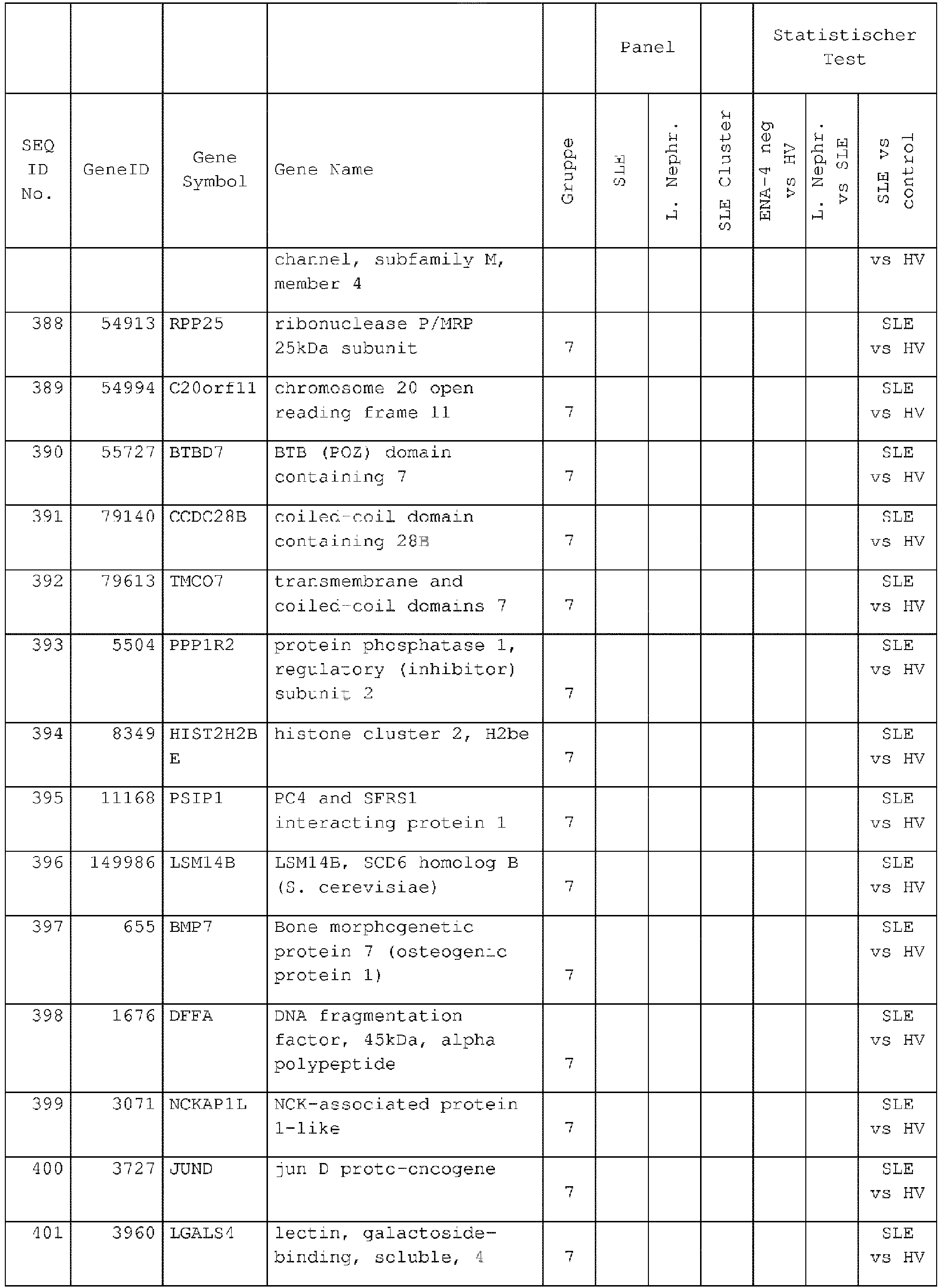

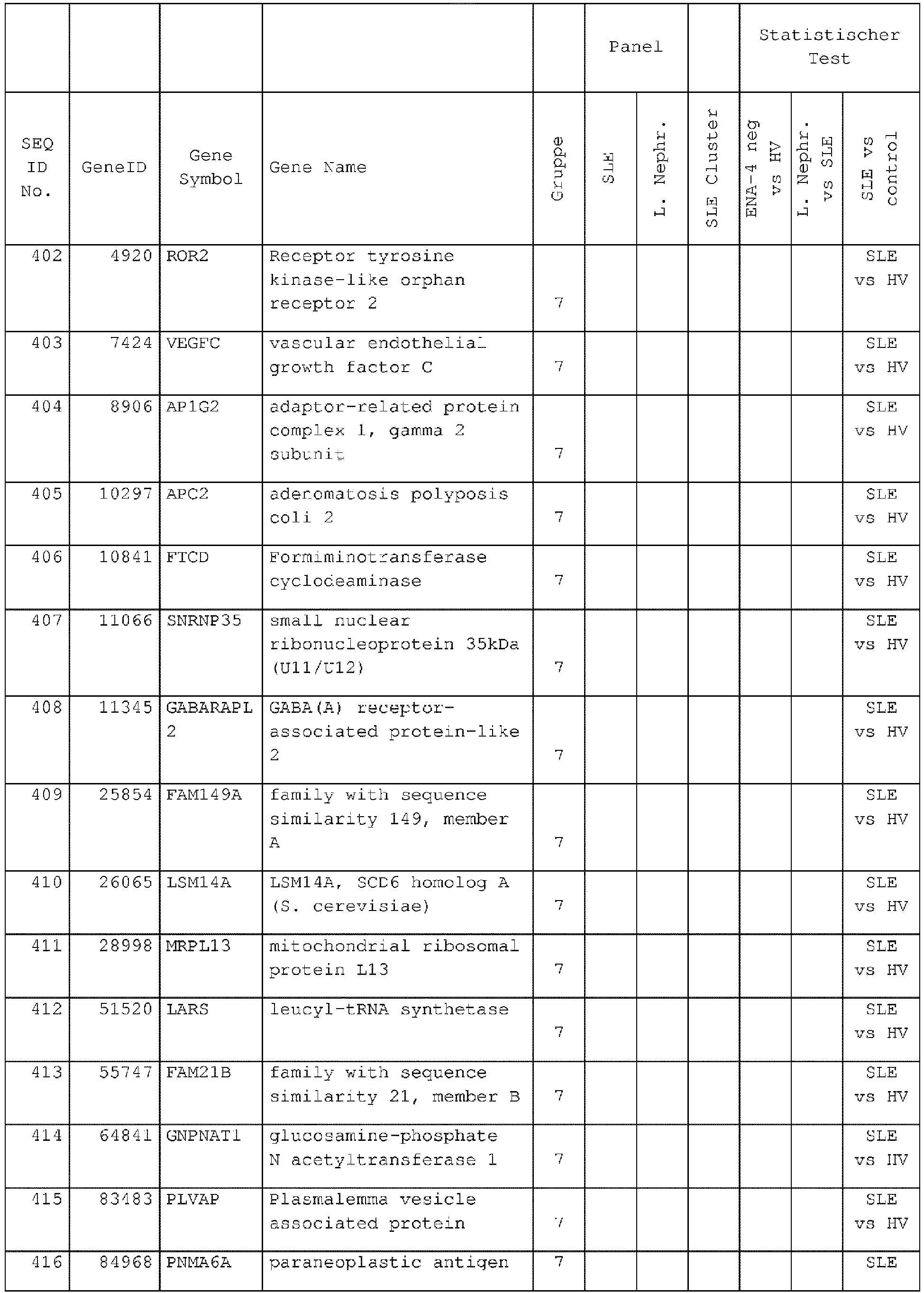

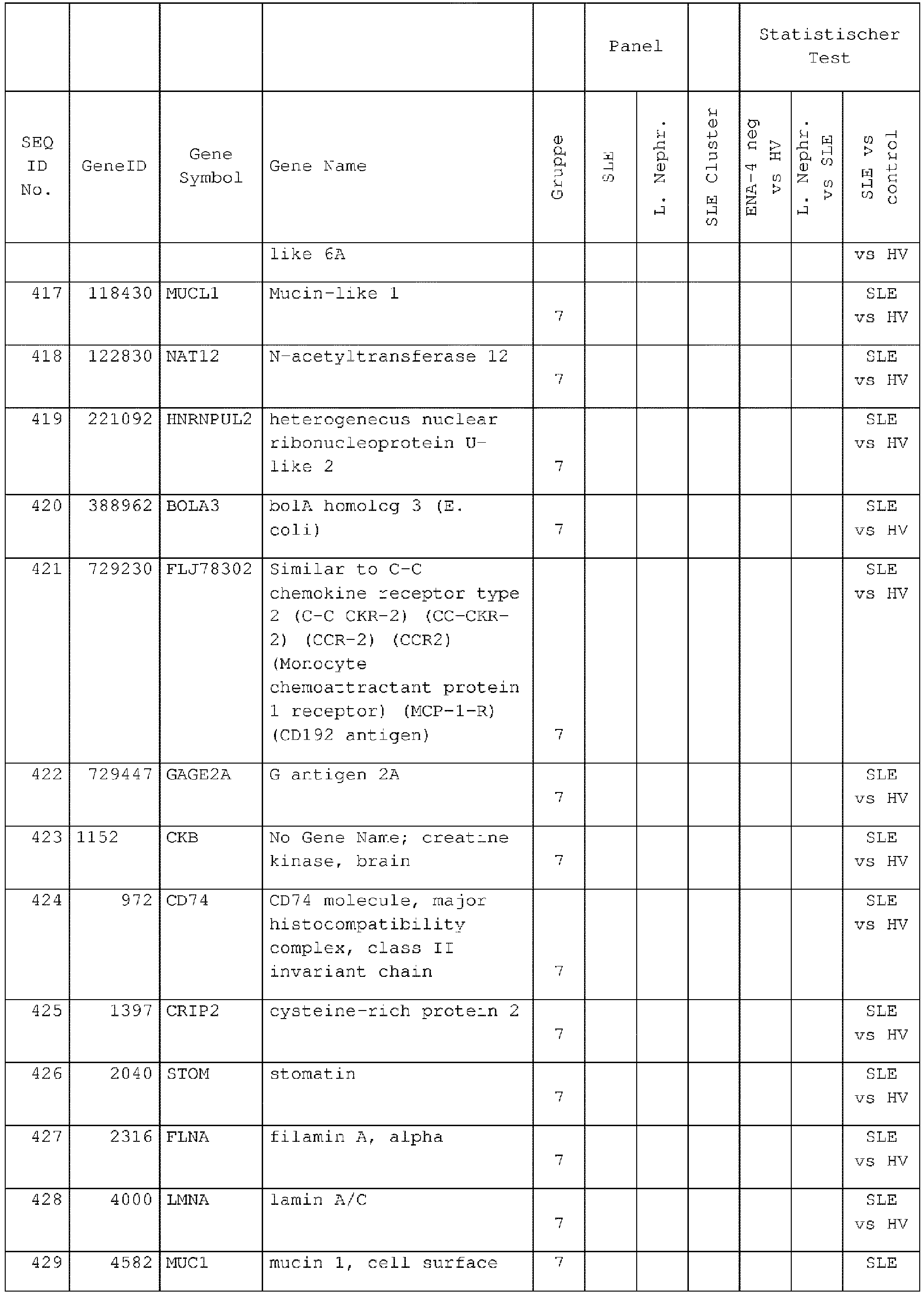

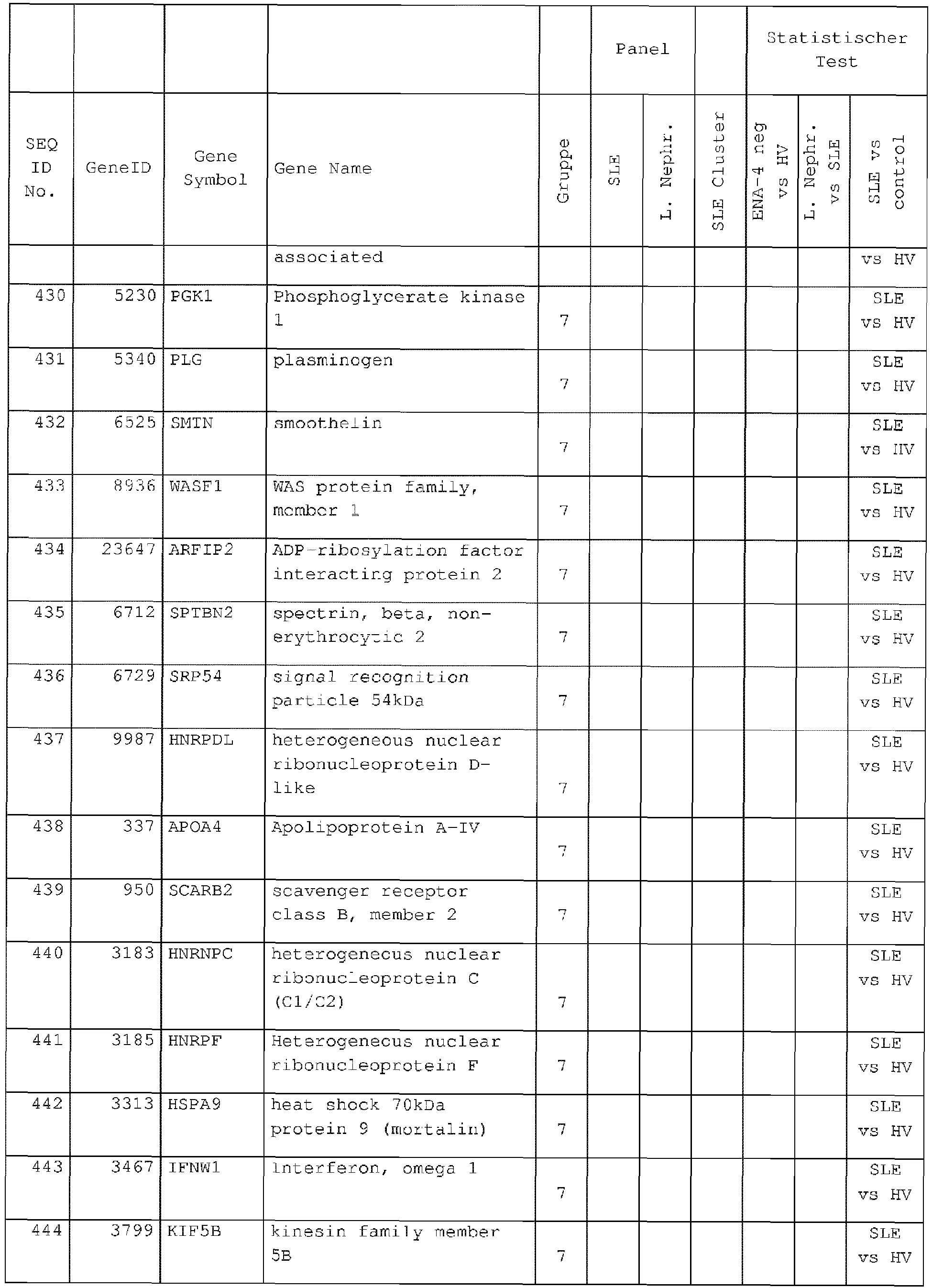

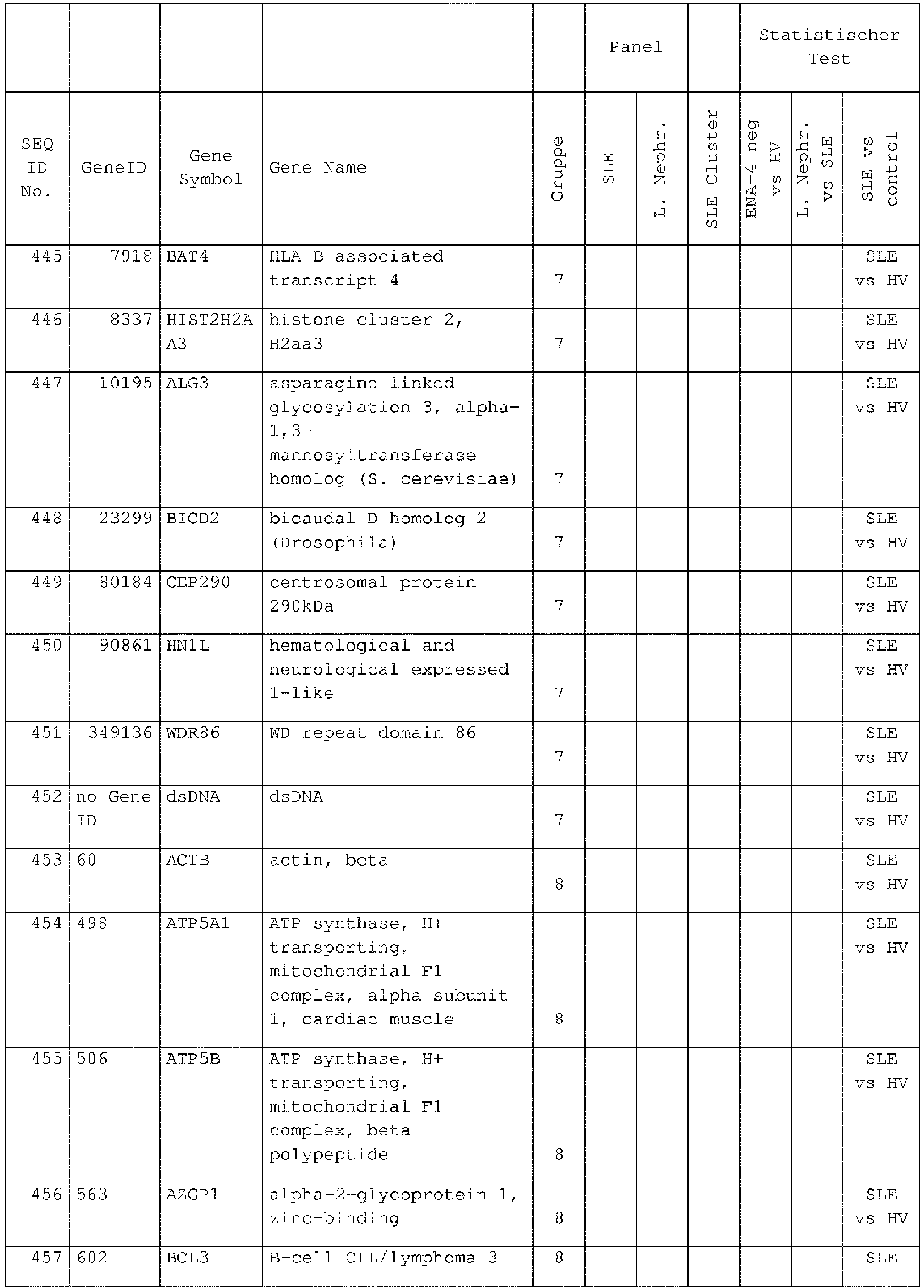

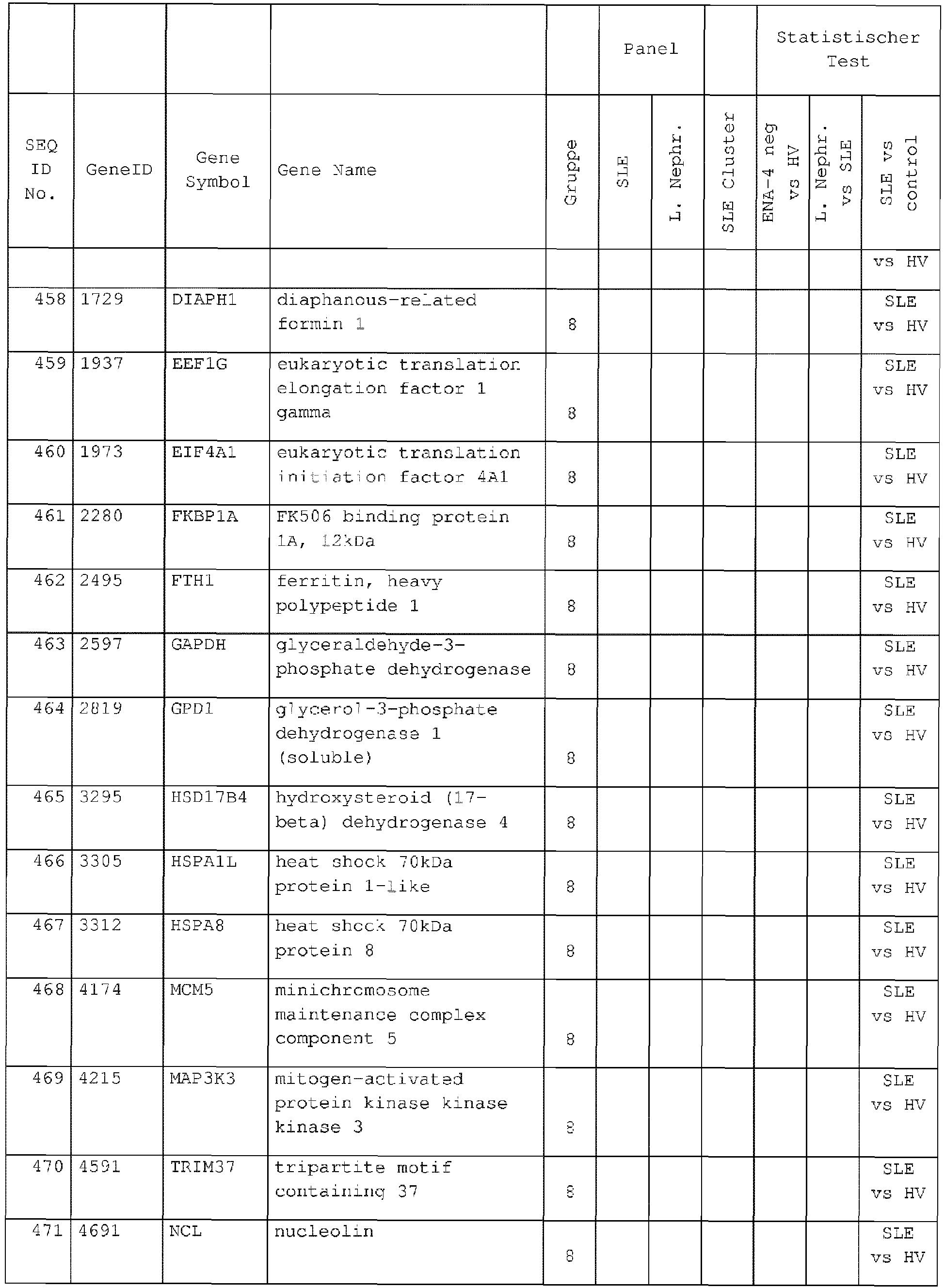

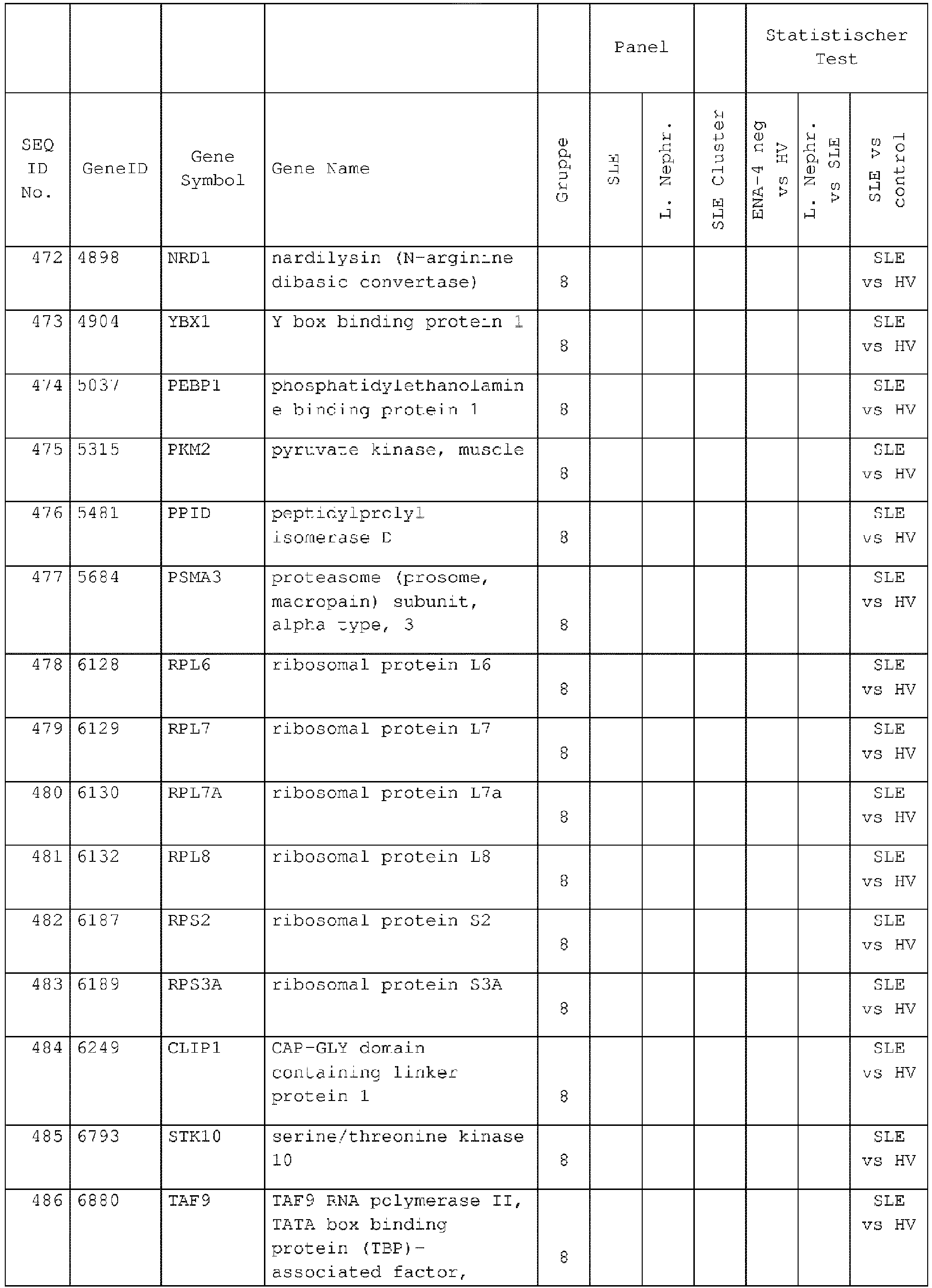

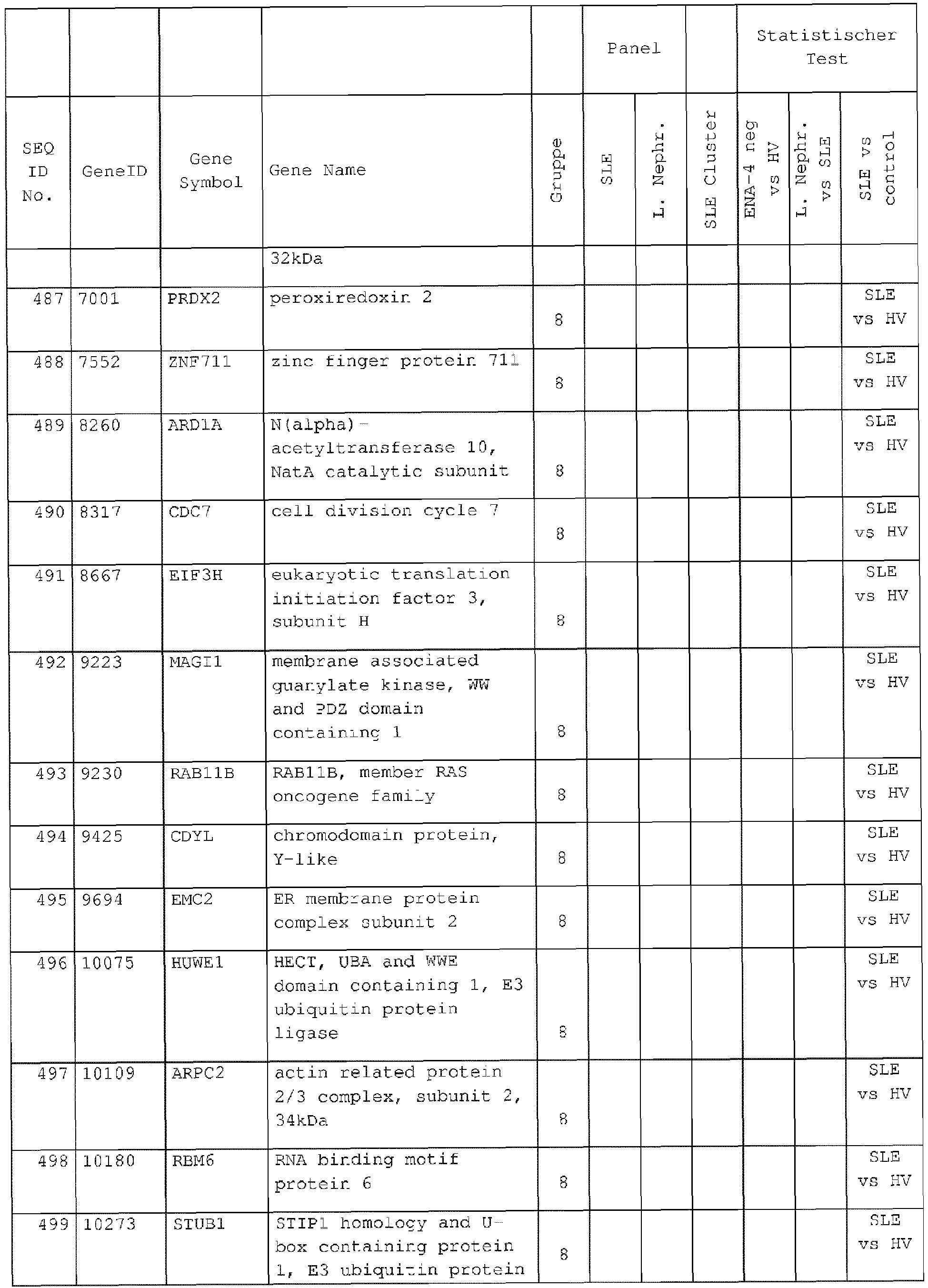

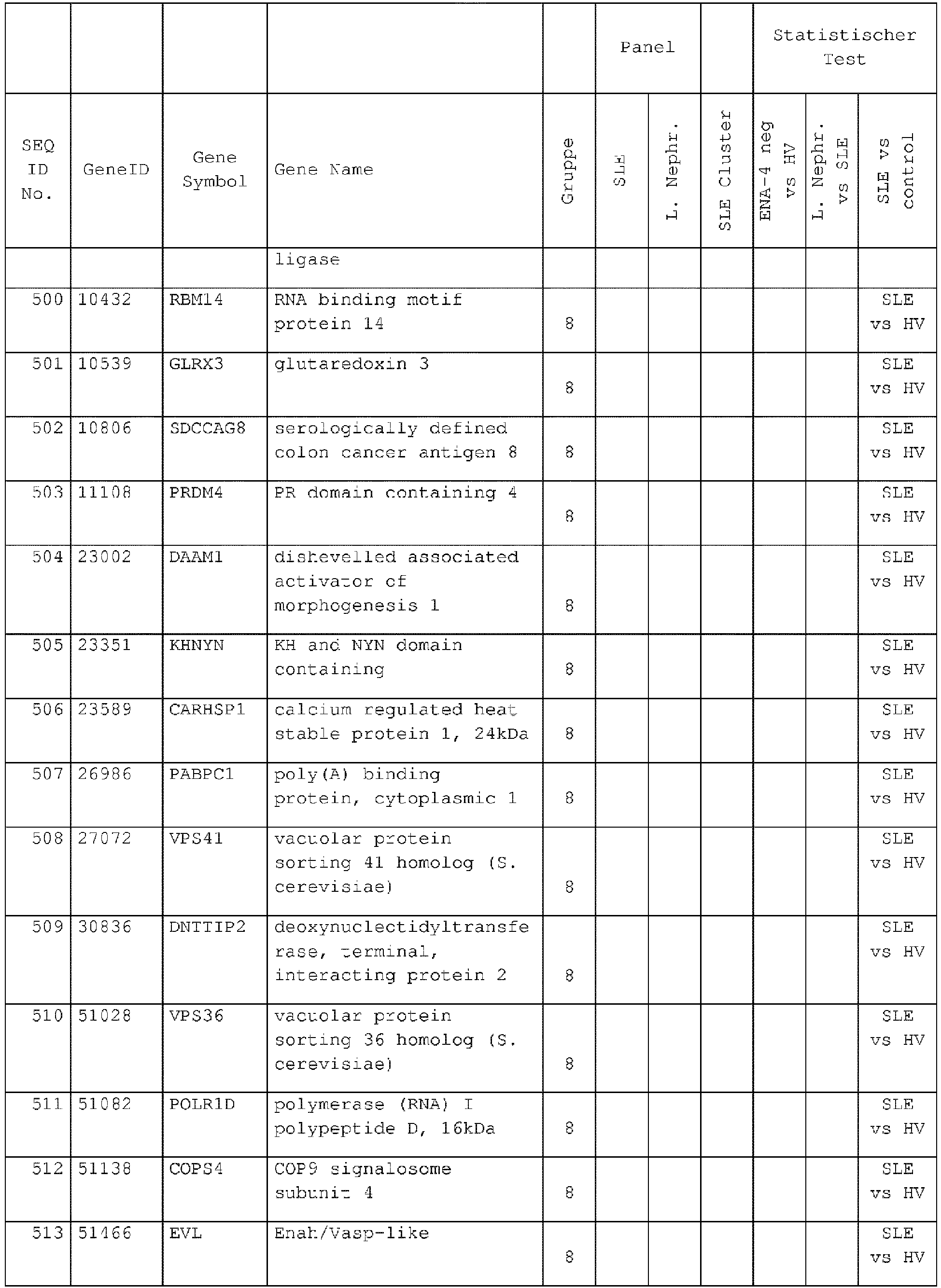

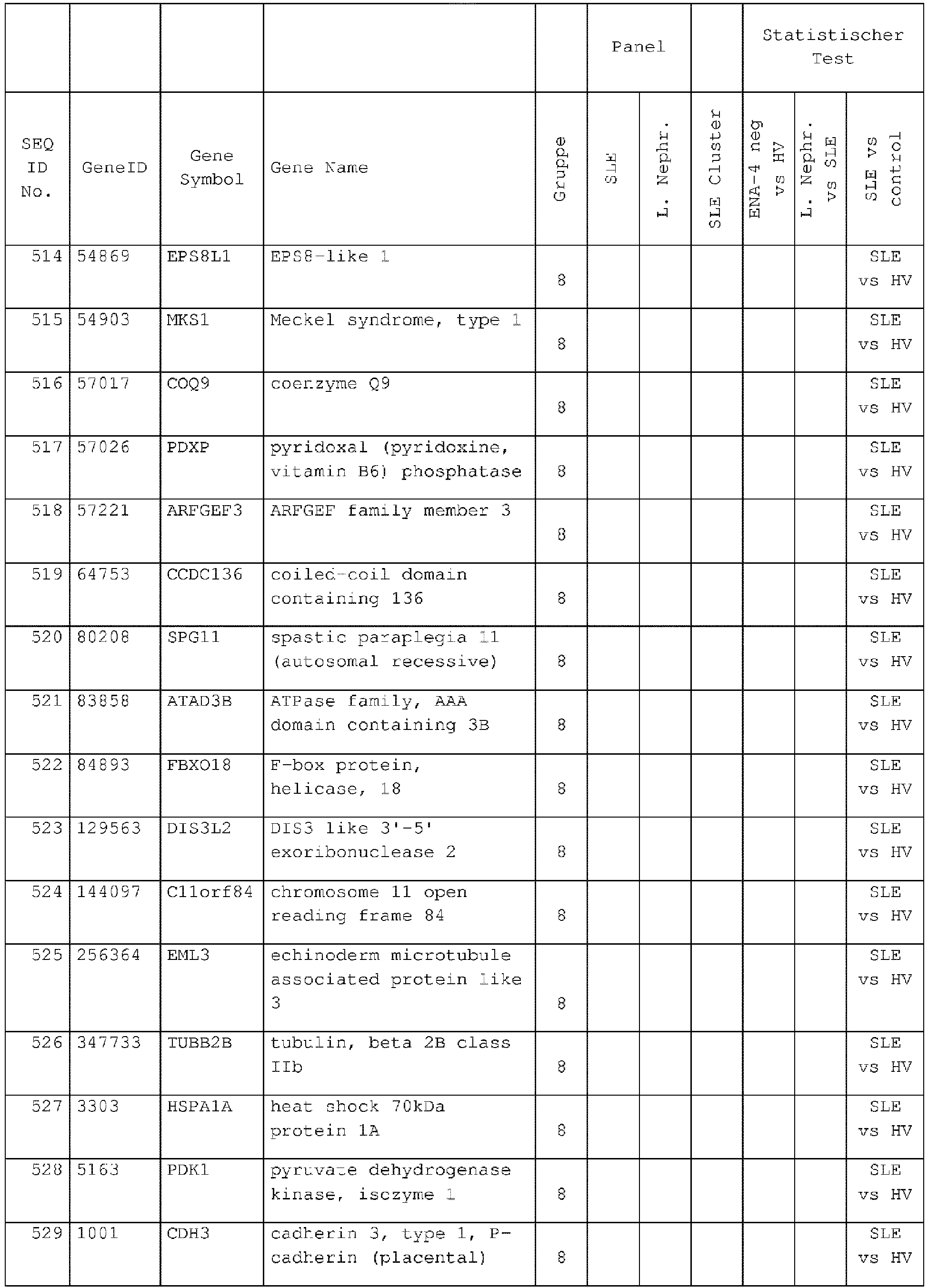

### Beispiel 9: Identifizierung von Autoantikörper Reaktivitäten in ENA-4 negativen SLE Patienten

Zur Identifizierung neuer SLE-spezifischer Autoantigene wurden die Autoantikörperprofile der Gruppe der SLE Patienten, die seropositiv für die Autoantigene Sm-Protein, U1-RNP, Rho52/SS-A und Ro60/SS-B ist, mit denen die seronegativ war verglichen. Das Ergebnis der statistischen Tests ist in Tabelle 2 zusammengefasst.

Gruppe 4 umfasst zusätzliche Antigene, die für die Identifizierung von ENA-4 negativen SLE Patienten geeignet sind.

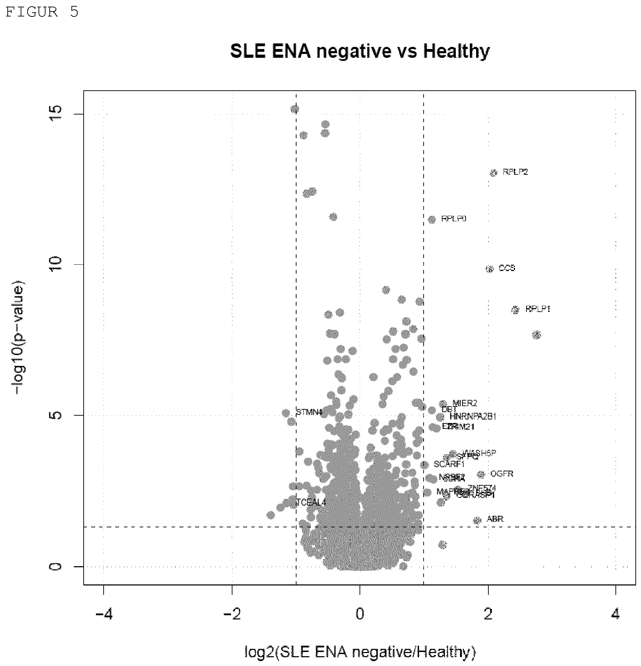

*Figur 5* zeigt den Vulcano Plot der Autoantikörperreaktivitäten von ENA-4 positiven gegen ENA-4 negativen SLE Patienten.

### Beispiel 10: Kalkulation von Antigen Panels zur verbesserten Diagnose von SLE

Aufgrund der hohen klinischen und serologischen Heterogenität der SLE Erkrankung ist es nicht möglich mit nur einem Biomarker diese zu diagnostizieren. Daher ist es erforderlich, möglichst unkorrelierte Autoantigene zu sogenannten Biomarker Panels zu kombinieren.

Gruppe 1 der Antigene in Tabelle 2 umfasst die wichtigsten 24 Antigene, die für die Berechnung von Biomarker Panels zur Diagnose von SLE verwendet werden.

Tabelle 4 zeigt unterschiedliche Kombinationen von Antigenen, die für die Berechnung der Biomarker Panels (ENA-4, ENA-4 + anti-Rib, PI, PII, PIII, PVI, PV) verwendet wurden.

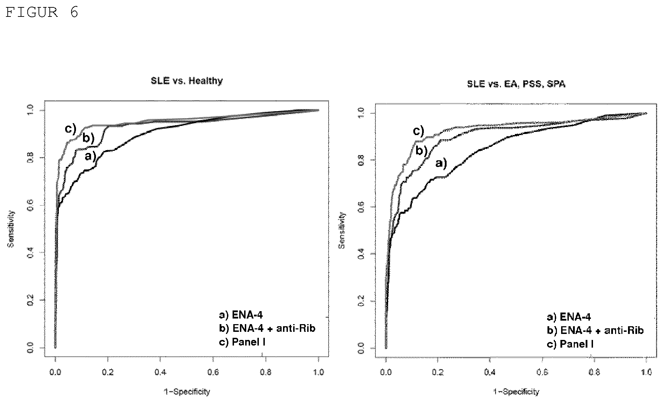

Figur 6 zeigt die Sensitivität und Spezifität, sowie die Fläche unter der Kurve (Area under the Curve, AUC) für die bekannten 4 Antigene im Vergleich zu Antigenpanels, die mit einer Kombination der Antigene aus Tabelle 2 gerechnet wurden.

Durch den Einschluss der 3 ribosomalen Antigene (anti-Rib) RPLPO, RPLP1 und RPLP2 konnte die Sensitivität bereits um 10% im Vergleich zu den bekannten 4 ENA Antigenen von 0.63 auf 0.72 gesteigert werden. Jedoch erst eine frei gewählte Kombination aus bekannten und neuen Antigenen konnte die Sensitivität um 20% gegenüber dem ENA-4 Test auf 0.8 steigern.

Für die Generierung von Panels wurden basierend auf den univariaten Ergebnisse Antigene ausgewählt, die einen adjustierten p-Wert für den nichtparametrischen Mittelwertvergleich zwischen Gruppen von <0,05 aufwiesen, gleichzeitig einen Fold Change von >1,5 und zusätzlich eine aus der ROC-Analyse resultierende AUC von >0,75. Außerdem wurden die ENA-4 Antigene ausgewählt. Für diesen Pool ausgewählter Kandidaten wurde im Rahmen einer genesteten Kreuzvalidierung ein L1-penalisiertes logistisches Regressionsmodell aufgestellt. Antigene, die im Rahmen der Modellbildung nicht berücksichtigt wurden, wurden aus der weiteren Betrachtung entfernt. Innerhalb des verbleibenden Pools wurden Panels inhaltlich definiert, zum Beispiel nach etablierten Markern und neuen Markern.

Gruppe 4 in Tabelle 2 enthält weitere statistisch signifikante Antigene, die für die Identifizierung von ENA-4 negativen Patienten und für die Definition von Biomarker-Panels verwendet werden können.

Gruppe 6 in Tabelle 2 enthält weitere statistisch signifikante Antigene, die für die Diagnose und Differentialdiagnose von SLE gegenüber Gesund und anderen Autoimmunerkrankungen herangezogen werden können.

**Tabelle 4: Zusammensetzung der diagnostischen SLE Panel**

| | | | Panels | | | | | | |
|---|---|---|---|---|---|---|---|---|---|
| Gene Symbol | Gene Name | Antigen | ENA-4 | ENA-4 + anti-Rib | PI | PII | PIII | PVI | PV |
| SNRPN | small nuclear ribonucleoprotein polypeptide N | Sm protein D | **x** | **x** | | X | | X | |
| SEQ ID NO. 14 | | | | | | | | | |
| TRIM21 | tripartite motifcontaining 21 | SSA/R0 | **X** | **x** | **X** | X | | X | |
| SEQ ID NO. 19 | | | | | | | | | |
| TROVE2 | TROVE domain family, member 2 | SSA/Ro60 | **X** | **x** | **X** | X | | X | |
| SEQ ID NO. 20 | | | | | | | | | |
| SSB | Sjogren syndrome antigen B (autoantigen La) | SSB/La | **X** | **x** | **X** | X | | X | |
| SEQ ID NO. 17 | | | | | | | | | |
| SNRNP70 | small nuclear ribonucleoprotein 70kDa (U1) | U1-RNP | **X** | **x** | **X** | X | | X | |
| SEQ ID NO. 12 | | | | | | | | | |
| SNRPB | small nuclear ribonucleoprotein polypeptides B and B1 | Sm protein B/B' | **X** | **x** | **X** | X | | X | |
| SEQ ID NO. 13 | | | | | | | | | |
| RPLPO | ribosomal protein, large, P0 | anti-Rib | | **X** | **X** | | X | X | |
| SEQ ID NO. 8 | | | | | | | | | |
| RPLP2 | ribosomal protein, large, P2 | anti-Rib | | **X** | | | X | X | |
| SEQ ID NO. 10 | | | | | | | | | |
| RPLP1 | ribosomal protein, large, P1 | anti-Rib | | **X** | | | X | X | |
| SEQ ID NO. 9 | | | | | | | | | |
| XRCC5 | X-ray repair complementing defective repair in Chinese hamster cells 5 (doublestrand-break rejoining) | Ku80 | | | | | | | X |
| SEQ ID NO. 22 | | | | | | | | | |
| VIM | vimentin | | | | | | X | | X |
| SEQ ID NO. 21 | | | | | | | | | |
| SPTB | spectrin, beta, erythrocytic | | | | | | X | | X |
| SEQ ID NO. 16 | | | | | | | | | |
| DBT | dihydrolipoamide branched chain transacylase E2 | | | | **X** | X | X | X | X |
| SEQ ID NO. 1 | | | | | | | | | |
| EZR | ezrin | | | | **X** | X | X | X | X |
| SEQ ID NO. 3 | | | | | | | | | |
| HNRNPA2B1 | heterogeneous nuclear ribonucleoprotein A2/B1 | | | | | | X | | X |
| SEQ ID NO. 6 | | | | | | | | | |
| TMPO | thymopoietin | | | | **X** | | X | X | X |
| SEQ ID NO. 18 | | | | | | | | | |
| MVP | major vault protein | | | | **X** | X | X | | X |
| SEQ ID NO. 7 | | | | | | | | | |
| ZNF574 | zinc finger protein 574 | | | | **X** | X | | | X |
| SEQ ID NO. 24 | | | | | | | | | |
| HIST1H2BD | histone cluster 1, H2bd | anti-Histone | | | | X | | | X |
| SEQ ID NO. 4 | | | | | | | | | |
| SH3KBP1 | SH3-domain kinase binding protein 1 | | | | | | | | X |
| SEQ ID NO. 11 | | | | | | | | | |
| ZNF217 | zinc finger protein 217 | | | | | | X | | X |
| SEQ ID NO. 23 | | | | | | | | | |
| SP100 | SP100 nuclear antigen | | | | **X** | X | X | X | X |
| SEQ ID NO. 15 | | | | | | | | | |

| Gene Symbol | Gene Name | Antigen | ENA-4 | ENA-4 + anti-Rib | PI | PII | PIII | PVI | PV |
|---|---|---|---|---|---|---|---|---|---|
| HNRNPA1 | heterogeneous nuclear ribonucleoprotein A1 | | | | **X** | X | X | X | X |
| SEQ ID NO. 5 | | | | | | | | | |
| DLAT | dihydrolipoamide S-acetyltransferase | PDC-E2, M2 antigen | | | **X** | | X | X | X |
| SEQ ID NO. 2 | | | | | | | | | |

**Tabelle 5: AUC, Sensitivität und Spezifität der SLE Panels**

| a) SLE versus Gesundkontrollen | | | | | | |
|---|---|---|---|---|---|---|
| **SLE vs PSS** | **AUC** | **CI (AUC)** | **Sens.** | **CI (Sens.)** | **Spec.** | **CI (Spec)** |
| Panel PI | 0.99 | [0.94,0.98] | 0.83 | [0.77,0.9] | 0.98 | [0.96,1.0] |
| Panel PII | 0.90 | [0.84,0.95] | 0.63 | [0.53,0.73] | 0.94 | [0.91,0.98] |
| Panel PIII | 0.91 | [0.87,0.95] | 0.61 | [0.5,0.72] | 0.95 | [0.92,0.98] |
| Panel PIV | 0.90 | [0.86,0.94] | 0.57 | [0.44,0.71] | 0.95 | [0.91,0.99] |
| Panel PV | 0.91 | [0.87,0.96] | 0.64 | [0.52,0.76] | 0.95 | [0.91,0.99] |
| ENA-4 | 0.89 | [0.84,0.94] | 0.63 | [0.49,0.77] | 0.96 | [0.94,0.98] |
| ENA-4 + anti-Rib | 0.93 | [0.88,0.98] | 0.72 | [0.6,0.84] | 0.97 | [0.95,0.99] |

| b) SLE versus SSc (PSS) | | | | | | |
|---|---|---|---|---|---|---|
| **SLE vs PSS** | **AUC** | **CI (AUC)** | **Sens.** | **CI (Sens.)** | **Spec.** | **CI (Spec)** |
| Panel PI | 0.9 | [0.85,0.95] | 0.78 | [0.73,0.83] | 0.83 | [0.71,0.94] |
| Panel PII | 0.83 | [0.78,0.88] | 0.72 | [0.61,0.83] | 0.76 | [0.68,0.85] |
| Panel PIII | 0.81 | [0.74,0.88] | 0.68 | [0.56,0.79] | 0.75 | [0.6,0.89] |
| panel PIV | 0.83 | [0.78,0.88] | 0.69 | [0.58,0.81] | 0.77 | [0.67,0.88] |
| Panel PV | 0.84 | [0.79,0.9] | 0.71 | [0.62,0.8] | 0.76 | [0.65,0.87] |
| ENA-4 | 0.75 | [0.66,0.85] | 0.6 | [0.5,0.7] | 0.8 | [0.71,0.88] |
| ENA-4 + anti-Rib | 0.82 | [0.74,0.9] | 0.63 | [0.51,0.75] | 0.83 | [0.74,0.93] |

| c) SLE versus alle AID (frühe RA, SSc, SPA) | | | | | | |
|---|---|---|---|---|---|---|
| **SLE vs Pool (EA, PSS, SPA)** | **AUC** | **CI (AUC)** | **Sens.** | **CI (Sens.)** | **Spec.** | **CI (Spec)** |
| Panel PI | 0.94 | [0.91,0.96] | 0.6 | [0.51,0.69] | 0.98 | [0.98,0.99] |
| Panel PII | 0.83 | [0.78,0.89] | 0.26 | [0.16,0.35] | 0.98 | [0.97,0.99] |
| Panel PIII | 0.83 | [0.74,0.92] | 0.27 | [0.16,0.37] | 0.99 | [0.98,1] |
| Panel PIV | 0.83 | [0.79,0.87] | 0.19 | [0.1,0.28] | 0.98 | [0.97,0.99] |
| Panel PV | 0.85 | [0.78,0.91] | 0.34 | [0.22,0.46] | 0.99 | [0.98,0.99] |
| ENA-4 | 0.84 | [0.79,0.9] | 0.35 | [0.22,0.47] | 0.99 | [0.98,0.99] |
| ENA-4 + anti-Rib | 0.91 | [0.88,0.93] | 0.49 | [0.41,0.58] | 0.98 | [0.97,0.99] |

### Beispiel 11: Identifizierung von Lupus Nephritis Patienten

Die Autoantikörperprofile von SLE Patienten mit Lupus Nephritis wurden mit denen von SLE Patienten ohne Lupus Nephritis verglichen. Nach univariater statistischer Auswertung wurde ein Schwellenwert von p<0.05 und eine 1,5 fach veränderte Reaktivität gegenüber der Kontrollgruppe angelegt. 85 Antigene erfüllten diese Kriterien und sind in Tabelle 2 aufgeführt.

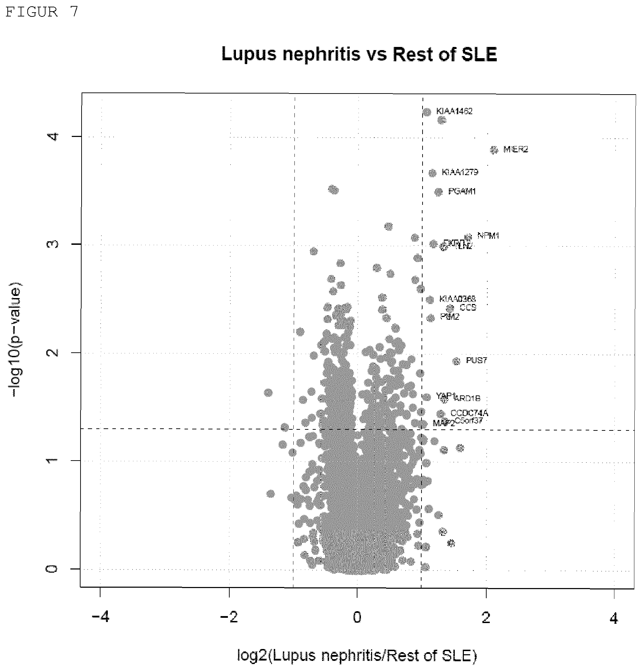

*Figur* 7 zeigt den Vulcano Plot der Reaktivität der Seren gegen ausgewählten Lupus Nephritis Antigene dar.

Gruppe 2 in Tabelle 2 beinhaltet 30 zusätzliche und wichtige Antigene, die für die Erzeugung von Lupus Nephritis Biomarker Panels verwendet werden können.

Für die Auswahl der besten Kandidaten wurde ein L1-Penalisiertes logistisches Regressionsmodell mit fünffacher Kreuzvalidierung und zwanzigfacher Wiederholung gerechnet. Die in dieser Modellrechnung am häufigsten gewählten Antigene mit einer Frequenz von über 50% stellen die besten Kandidaten für die Diagnose von Lupus Nephritis dar.

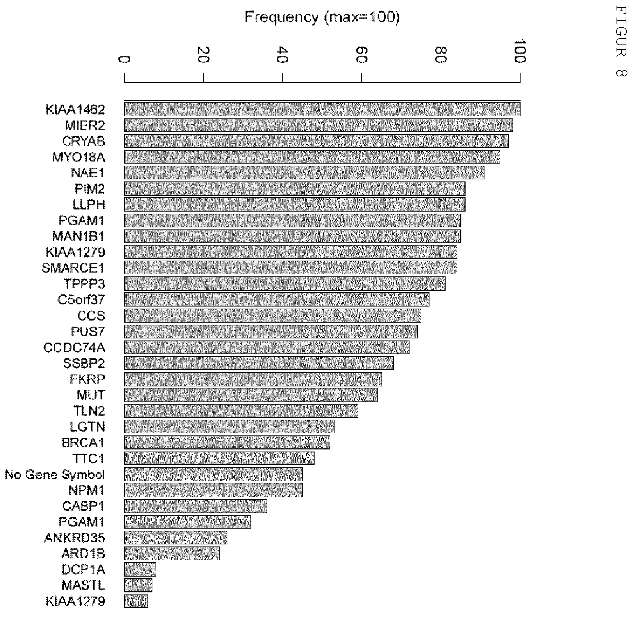

*Figur 8* zeigt die Häufigkeitsverteilung der Lupus Nephritis Antigene.

Gruppe 5 umfasst weitere statistisch signifikante Antigene, die für die Diagnose von Lupus Nephritis geeignet sind.

### Beispiel 12: Identifizierung von SLE Unterformen und Subgruppen

Die große klinische Heterogenität von SLE stellt derzeit sowohl für die Diagnostik als auch für die Wirkstoffentwicklung ein großes Problem dar.

Die Identifizierung von spezifischen Antikörpersignaturen in SLE Patienten -Subgruppen stellt daher einen wichtigen Schritt für die bessere Definition von Patientengruppen in klinischen Studien dar. So könnten beispielsweise wie unter Beispiel 9 dargestellt spezifische Autoantikörper für Lupus Nephritis dazu verwendet werden, diese Subgruppe für Medikamentenstudien zu rekrutieren.

Derzeit sind eine Vielzahl von neuen Wirkstoffen und therapeutischen Antikörpern in der klinischen Entwicklung: Unter anderem werden therapeutische Antikörper gegen Zelloberflächenrezeptoren von Immunzellen, wie z.B.anti-CD20, anti-CD22, oder gegen proinflammatorische Cytokine, wie z.B. anti-IL6 entwickelt. Daher besteht nun durch die Identifizierung serologisch-definierter Subgruppen von SLE die Möglichkeit diese mit einer targetspezifischen Antwort auf ein Medikament zu verknüpfen.

Zunächst wurde untersucht, ob sich basierend auf den typischen ENA-Antigenen und ribosomalen Antigenen bereits unterschiedliche Autoantikörpersignaturen in SLE Patienten und somit Patientensubgruppen darstellen lassen.

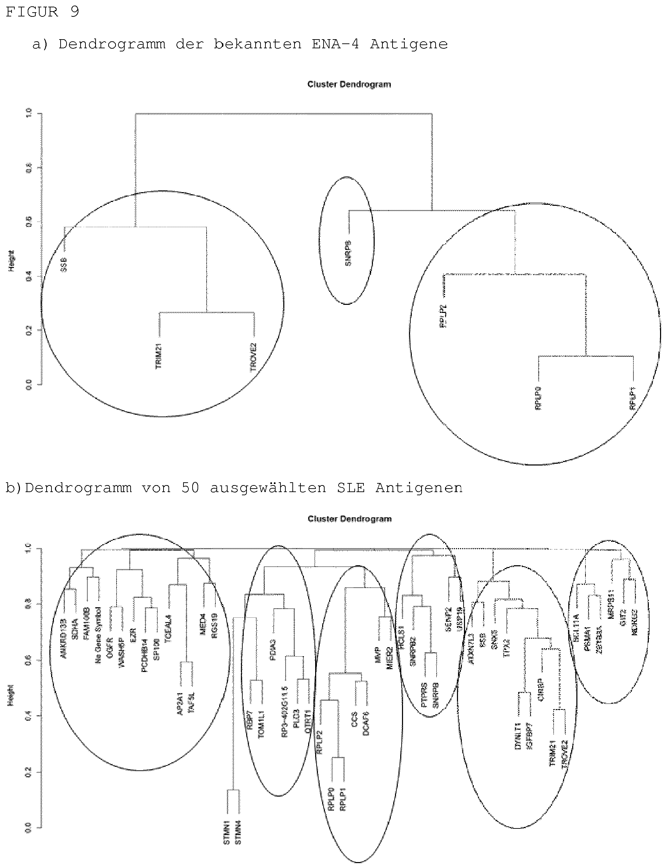

*Figur 9a, b* zeigen ein Dendogramm der SLE Antigene nach Berechnung des Spearman Rangkorrelationskoeffizienten *Figur 9a* zeigt ein Dendrogramm für die Antigene Sm, SS-B, Ro-52/SS-A, Ro60-SS-B und drei ribosomale Proteine.

Basierend auf diesen 7 Antigenen lassen sich bereits 3 Antigen-Cluster definieren.

Für eine bessere Definition von SLE-Subgruppen ist allerdings eine größere Anzahl von Antigenen notwendig. Daher wurden 50 Antigene aus Tabelle 2 ausgewählt und deren Korrelation in SLE Patienten durch Berechnung des Spearman Rangkorrelationskoeffizienten untersucht.

Gruppe 3 enthält 37 der wichtigsten Antigene, die für die Charakterisierung von SLE Subgruppen notwendig sind. Weitere Antigene sind bereits in Gruppe 1 und Gruppe 2 definiert worden.

Die Darstellung der Antigene als Dendrogramm zeigt Gruppen von Antigenen, deren Reaktivitäten in SLE Patienten miteinander korreliert sind.

Wie in *Figur 9b* dargestellt lassen sich hierdurch mindestens 6 Gruppen von korrelierten Antigenen identifizieren.

Interessanterweise beinhaltet eines der Cluster die Antigene MVP, MIER2, CCS, DCAF6, die in Tabelle als Biomarker für Lupus Nephritis identifiziert wurden.

Durch die Berechnung eines PPLS-DA basierten Regressionsmodells lässt sich visualisieren wie gut die ausgewählten Antigene zur Diskrimination der SLE Patienten von Gesundkontrollen beitragen.

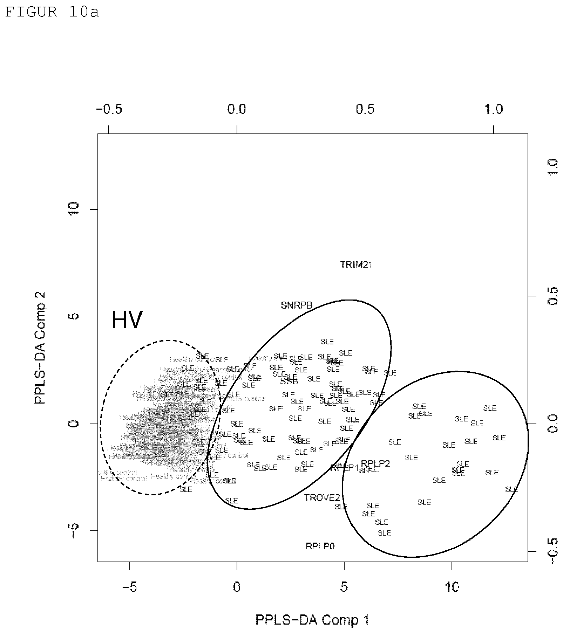

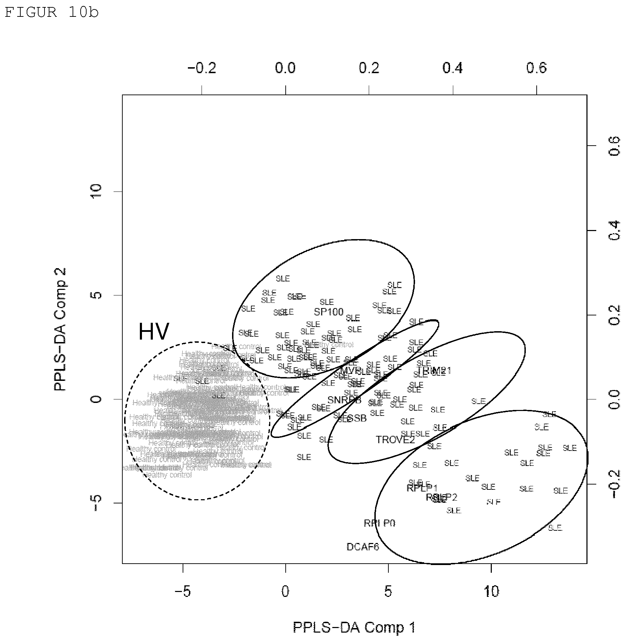

*Figur 10a**,* *b* zeigen den PPLS-DA Biplot der SLE Patienten und Gesundkontrollen unter Verwendung der SLE Antigene.

*Figur 10a* zeigt einen PPLS-DA Biplot für die ausgewählten ENA-Antigene und ribosomalen Proteine und deren Messwerte in den SLE Patienten. Figur 10a zeigt, dass die Separation von Gesund und SLE nicht vollständig ist und einige SLE Patienten mit der Gruppe der Gesundproben zusammenfallen. Allerdings ergibt sich bereits mit wenigen Antigenen eine Aufspaltung der SLE Patienten in 2 Cluster.

*Figur 10b* zeigt einen PPLS-DA Biplot für 50 Antigene, die in Tabelle 2 enthalten sind. Durch die Auswahl weiterer Antigene ergibt sich eine nahezu perfekte Separation der SLE Patienten und Gesundproben.

Durch 50 Antigene ergibt sich eine weitere Auffächerung der SLE Patienten in möglichen Untergruppen. Diese können durch spezifische Antigene definiert werden, von denen einige beispielhaft hervorgehoben worden sind.

### Beispiel 13: Validierung von SLE Antigenen in einer unabhängigen Testkohorte II

Zur Validierung der in Tabelle 2 genannten SLE-assoziierten Autoantigene wurde die Autoantikörperreaktivität in Serumproben einer weiteren unabhängigen Kohorte von 101 SLE Patienten, 105 Gesundkontrollen und 89 Proben der SLE Kohorte aus Beispiel 6 gemessen. Hierzu wurden die 529 in Tabelle 2 genannten humanen Proteine (SEQ ID No. 1059 bis 1587), sowie deren doppelsträngige DNA (dsDNA), an Luminex Beads gekoppelt und die Antigen-gekoppelten Beads in einem Multiplex Assay mit den Patientenproben vermessen. Die Bindung von Autoantikörpern wurde mittels eines PE-konjugierten Autoantikörpers in einem Luminex Instrument gemessen.

Nach univariater statistischer Auswertung wurde ein Schwellenwert von p<0.05 (Wilcoxon-Rangsummen-Test) gegenüber der Kontrollgruppe angelegt.

Eine Liste der Signifikanzwerte (p-Werte) für Autoantikörper gegen 50 Antigene in der SLE Kohorte II ist in Tabelle 6 gezeigt. Von den 50 Antigenen erreichten 43 Antigene in Kohorte I und Kohorte II einen p-Wert <0.05.

Die prozentuale Häufigkeit von Autoantikörpern gegen 50 Antigene in den drei SLE Kohorten ist in Tabelle 7 gezeigt.

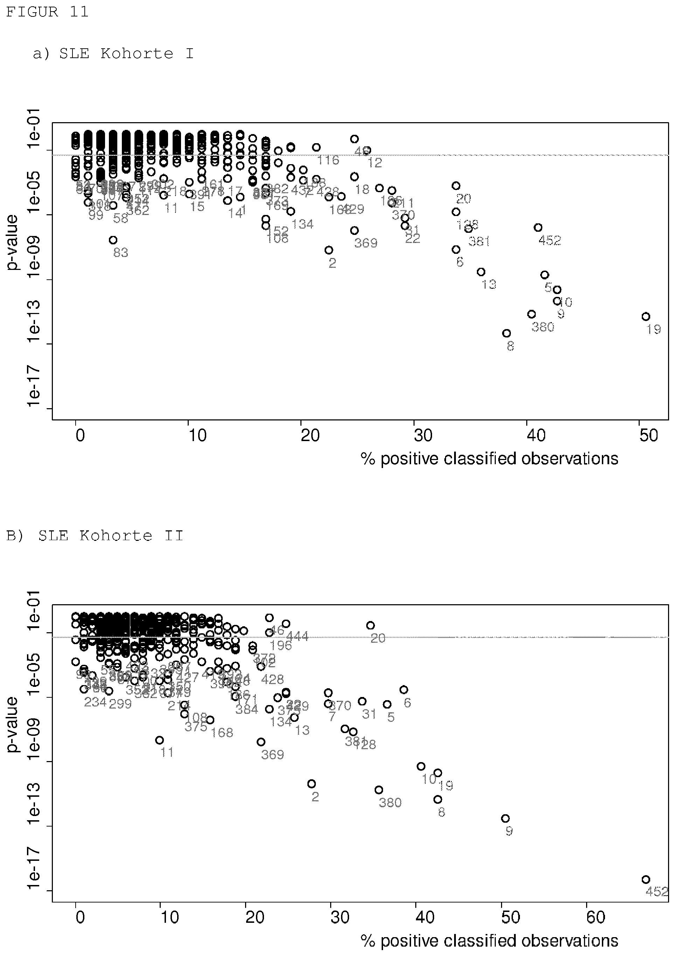

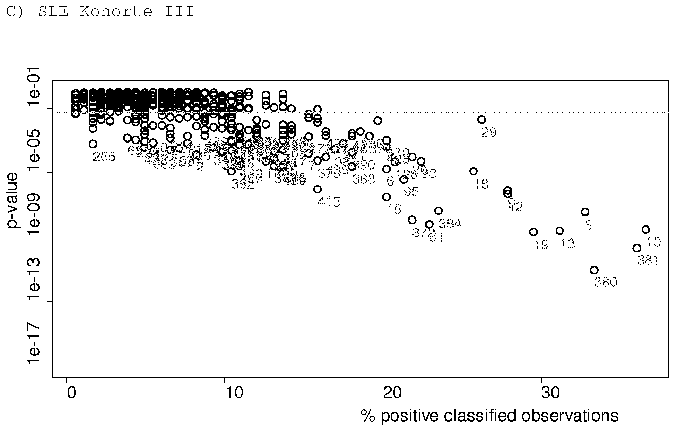

*Figur 11*: zeigt den berechneten p-Wert der Antigenen aus Tabelle 2 sowie die Häufigkeit von SLE Patienten, die als Autoantikörper-positiv für dieses Antigen klassifiziert wurden.

### Beispiel 14: Validierung von SLE Autoantigenen in einer dritten unabhängigen Testkohorte III

Zur Validierung der in Tabelle 2 genannten SLE-assoziierten Autoantigene wurde die Autoantikörperreaktivität in Serumproben einer unabhängigen Kohorte von 183 SLE Patienten und 109 Gesundkontrollen gemessen. Hierzu wurden 6.912 humane Proteine an Luminex Beads gekoppelt und die Proteingekoppelten Beads in einem Multiplex Assay mit den Patientenproben vermessen. Die Bindung von Autoantikörpern wurde mittels eines PE-konjugierten Autoantikörpers in einem Luminex Instrument gemessen.

Nach univariater statistischer Auswertung wurde ein Schwellenwert von p<0.05 und eine Effektstärke Cohen's d von größer 0.3 gegenüber der Kontrollgruppe angelegt.

Eine Liste der Signifikanzwerte ist in Tabelle 6 gezeigt. Tabelle enthält ausgewählte Marker, die Bestandteil der Panels ENA + anti-Rib, Panel I, Panel VI, Panel VII und Panel VIII sind. Die Tabelle enthält weitere Marker, die in allen drei Kohorten einen p-Wert von <0.05 erreichten.

**Tabelle 6: Signifikanzwerte (p-Werte) von 50 Antigenen in 3 SLE Kohorten**

| Seq. Nr | GeneID | Gene Symbol | SLE Kohorte I | SLE Kohorte II | SLE Kohorte III | Panel |
|---|---|---|---|---|---|---|
| 1 | 1629 | DBT | 1.28E-04 | 2.16E-03 | 3.82E-03 | Panel I; II; III; IV; V; VII |
| 2 | 1737 | DLAT | 6.33E-08 | 4.12E-11 | 1.31E-04 | Panel I; III; IV; VI; VII |
| 3 | 7430 | EZR | 1.44E-03 | 2.68E-01 | 9.79E-02 | panel I; II; III; IV |
| 4 | 3017 | HIST1H2BD | 9.43E-01 | 4.08E-01 | 4.00E-02 | Panel II, V |
| 5 | 3178 | HNRNPA1 | 1.99E-09 | 3.60E-06 | 2.81E-04 | Panel I; II, III; IV; V; VI; VII |
| 6 | 3181 | HNRNPA2B 1 | 7.31E-08 | 2.81E-05 | 1.70E-05 | Panel III; V; VI; VII |
| 7 | 9961 | MVP | 1.40E-03 | 3.90E-06 | 1.56E-04 | Panel I; II; III; V; VI; VII |
| 8 | 6175 | RPLP0 | 4.59E-13 | 4.75E-12 | 3.51E-08 | ENA-4 + anti-Rib, Panel I; III; VI; VII |
| 9 | 6176 | RPLP1 | 4.61E-11 | 3.05E-13 | 7.50E-07 | ENA-4 + anti-Rib; Pane lIII; IV; VII |
| 10 | 6181 | RPLP2 | 2.38E-10 | 5.18E-10 | 2.94E-09 | ENA-4 + anti-Rib; Panel I; VI; VII |
| 11 | 30011 | SH3KBP1 | 1.68E-04 | 2.22E-08 | 2.64E-02 | Panel V |
| 12 | 6625 | SNRNP70 | 4.87E-02 | 9.02E-02 | 1.22E-08 | ENA-4 + anti-Rib; Panel I; II; IV |
| 13 | 6628 | SNRPB | 2.95E-09 | 5.57E-07 | 2.49E-09 | ENA-4 + anti-Rib; Panel I; II; IV; VI; VII |
| 14 | 6638 | SNRPN | 7.44E-05 | 2.61E-02 | 3.64E-02 | ENA-4 + anti-Rib, Panel II; Panel IV |
| 15 | 6672 | SP100 | 1.98E-04 | 2.47E-04 | 3.04E-07 | Panel I; II; III; IV; V; VII |
| 16 | 6710 | SPTB | 7.76E-06 | 5.13E-01 | 8.37E-01 | Panel III; V |
| 17 | 6741 | SSB | 1.75E-03 | 3.20E-02 | 7.82E-02 | ENA-4 + anti-Rib; Panel I; II; IV |
| 18 | 7112 | TMPO | 2.20E-03 | 3.15E-02 | 1.15E-05 | Panel I; II; III; IV; VI |
| 19 | 6737 | TRIM21 | 5.07E-12 | 1.96E-10 | 6.35E-10 | Panel I; II; iV; VI; VII |
| 20 | 6738 | TROVE2 | 6.11E-04 | 2.59E-01 | 9.27E-05 | ENA-4 + anti-Rib; Panel I; II; IV |
| 21 | 7431 | VIM | 2.37E-01 | 2.25E-04 | 1.06E-03 | Panel III; V |
| 22 | 7520 | XRCC5 | 2.06E-06 | 2.01E-05 | 7.83E-04 | Panel V; VI; VII |
| 23 | 7764 | ZNF217 | 3.28E-01 | 1.55E-01 | 5.04E-05 | Panel III; V |
| 24 | 64763 | ZNF574 | 1.02E-02 | 8.36E-04 | 1.33E-03 | Panel I; II; V; VII |
| 31 | 9973 | CCS | 6.65E-06 | 5.28E-06 | 6.62E-09 | Panel VII |
| 95 | 6629 | SNRPB2 | 1.06E-03 | 8.68E-03 | 3.66E-06 | Panel VIII |
| 128 | 10970 | CKAP4 | 1.52E-05 | 7.26E-08 | 4.60E-05 | Panel VIII |
| 134 | 1743 | DLST | 1.63E-05 | 1.78E-06 | 4.95E-05 | Panel VII |
| 168 | 4841 | NONO | 1.27E-04 | 3.76E-07 | 5.17E-04 | Panel VI; VII |
| 169 | 29982 | NRBF2 | 2.22E-04 | 2.44E-02 | 7.44E-03 | Panel VIII |
| 171 | 4926 | NUMA1 | 1.62E-03 | 4.28E-05 | 4.31E-03 | Panel VIII |
| 214 | 7791 | ZYX | 4.54E-04 | 1.77E-05 | 2.57E-03 | Panel VII |
| 368 | 4670 | HNRNPM | 7.61E-03 | 8.98E-03 | 2.34E-05 | Panel VII |
| 369 | 10540 | DCTN2 | 1.11E-06 | 1.69E-08 | 2.49E-05 | Panel VII |
| 370 | 10938 | EHD1 | 5.60E-05 | 1.73E-05 | 1.01E-03 | Panel VII |
| 372 | 684 | BST2 | 2.51E-02 | 1.44E-02 | 1.13E-08 | Panel VIII |
| 373 | 1058 | CENPA | 4.45E-04 | 8.70E-06 | 2.51E-05 | Panel VIII |
| 375 | 3092 | HIP1 | 4.42E-02 | 8.73E-07 | 2.03E-04 | Panel VIII |
| 376 | 3336 | HSPE1 | 2.15E-02 | 1.42E-02 | 1.73E-03 | Panel VIII |
| 377 | 5455 | POU3F3 | 1.31E-03 | 1.02E-04 | 6.18E-03 | Panel VIII |
| 380 | 6626 | SNRPA | 7.16E-12 | 1.85E-11 | 2.60E-16 | Panel VIII |
| 381 | 6631 | SNRPC | 1.42E-06 | 1.06E-07 | 3.73E-15 | Panel VIII |
| 382 | 6757 | SSX2 | 2.40E-03 | 1.78E-02 | 2.09E-04 | Panel VIII |
| 384 | 10134 | BCAP31 | 4.10E-02 | 1.04E-05 | 4.34E-08 | Panel VIII |
| 390 | 55727 | BTBD7 | 3.33E-02 | 3.68E-04 | 1.79E-04 | Panel VIII |
| 428 | 4000 | LMNA | 1.59E-03 | 7.49E-04 | 3.41E-04 | Panel VIII |
| 429 | 4582 | MUC1 | 1.35E-04 | 1.66E-05 | 6.29E-04 | Panel VIII |
| 431 | 5340 | PLG | 1.22E-03 | 1.64E-02 | 5.61E-03 | Panel VIII |
| 432 | 6525 | SMTN | 2.05E-03 | 1.53E-02 | 9.78E-03 | Panel VIII |
| 452 | dsDNA | dsDNA | 1.65E-06 | 5.35E-17 | NA | dsDNA |

**Tabelle 7: Liste der Häufigkeit von Autoantikörper-positiv getesteten SLE Patienten in 3 unabhängigen Kohorten.**

| | | | | | | |
|---|---|---|---|---|---|---|
| Die prozentuale Häufigkeit von Autoantikörpern-positiven Individuen aus Tabelle 6 wurde mittels der 95% Quantile der Gesundkontrollen kalkuliert. | | | | | | |

| | | | Anteil Autoantikörperpositiver SLE Patienten (%) basierend auf der 95% Quantile der Kontrollgruppe | | | |
|---|---|---|---|---|---|---|
| Seq. Nr. | GeneID | Gene Symbol | SLE Kohorte I | SLE Kohorte II | SLE Kohorte III | Panel |
| 1 | 1629 | DBT | 1.28E-04 | 2.16E-03 | 3.82E-03 | Panel I; II; III; IV; V; VII |
| 2 | 1737 | DLAT | 6.33E-08 | 4.12E-11 | 1.31E-04 | Panel I; III; IV; VI; VII |
| 3 | 7430 | EZR | 1.44E-03 | 2.68E-01 | 9.79E-02 | panel I; II; III; IV |
| 4 | 3017 | HIST1H2BD | 9.43E-01 | 4.08E-01 | 4.00E-02 | Panel II, V |
| 5 | 3178 | HNRNPA1 | 1.99E-09 | 3.60E-06 | 2.81E-04 | Panel I; II, III; IV; V; VI; VII |
| 6 | 3181 | HNRNPA2B1 | 7.31E-08 | 2.81E-05 | 1.70E-05 | Panel III; V; VI; VII |
| 7 | 9961 | MVP | 1.40E-03 | 3.90E-06 | 1.56E-04 | Panel I; II; III; V; VI; VII |
| 8 | 6175 | RPLP0 | 4.59E-13 | 4.75E-12 | 3.51E-08 | ENA-4 + anti-Rib, Panel I; III; VI; VII |
| 9 | 6176 | RPLP1 | 4.61E-11 | 3.05E-13 | 7.50E-07 | ENA-4 + anti-Rib; Pane lIII; IV; VII |
| 10 | 6181 | RPLP2 | 2.38E-10 | 5.18E-10 | 2.94E-09 | ENA-4 + anti-Rib; Panel I; VI; VII |
| 11 | 30011 | SH3KBP1 | 1.68E-04 | 2.22E-08 | 2.64E-02 | Panel V |
| 12 | 6625 | SNRNP70 | 4.87E-02 | 9.02E-02 | 1.22E-08 | ENA-4 + anti-Rib; Panel I; II; IV |
| 13 | 6628 | SNRPB | 2.95E-09 | 5.57E-07 | 2.49E-09 | ENA-4 + anti-Rib; Panel I; II; IV; VI; VII |
| 14 | 6638 | SNRPN | 7.44E-05 | 2.61E-02 | 3.64E-02 | ENA-4 + anti-Rib, Panel II; Panel IV |
| 15 | 6672 | SP100 | 1.98E-04 | 2.47E-04 | 3.04E-07 | Panel I; II; III; IV; V; VII |
| 16 | 6710 | SPTB | 7.76E-06 | 5.13E-01 | 8.37E-01 | Panel III; V |
| 17 | 6741 | SSB | 1.75E-03 | 3.20E-02 | 7.82E-02 | ENA-4 + anti-Rib; Panel I; II; IV |
| 18 | 7112 | TMPO | 2.20E-03 | 3.15E-02 | 1.15E-05 | Panel I; II; III; IV; VI |
| 19 | 6737 | TRIM21 | 5.07E-12 | 1.96E-10 | 6.35E-10 | Panel I; II; iV; VI; VII |
| 20 | 6738 | TROVE2 | 6.11E-04 | 2.59E-01 | 9.27E-05 | ENA-4 + anti-Rib; Panel I; II; IV |
| 21 | 7431 | VIM | 2.37E-01 | 2.25E-04 | 1.06E-03 | Panel III; V |
| 22 | 7520 | XRCC5 | 2.06E-06 | 2.01E-05 | 7.83E-04 | Panel V; VI; VII |
| 23 | 7764 | ZNF217 | 3.28E-01 | 1.55E-01 | 5.04E-05 | Panel III; V |
| 24 | 64763 | ZNF574 | 1.02E-02 | 8.36E-04 | 1.33E-03 | Panel I; II; V; VII |
| 31 | 9973 | CCS | 6.65E-06 | 5.28E-06 | 6.62E-09 | Panel VII |
| 95 | 6629 | SNRPB2 | 1.06E-03 | 8.68E-03 | 3.66E-06 | Panel VIII |
| 128 | 10970 | CKAP4 | 1.52E-05 | 7.26E-08 | 4.60E-05 | Panel VIII |
| 134 | 1743 | DLST | 1.63E-05 | 1.78E-06 | 4.95E-05 | Panel VII |
| 168 | 4841 | NONO | 1.27E-04 | 3.76E-07 | 5.17E-04 | Panel VI; VII |
| 169 | 29982 | NRBE 2 | 2.22E-04 | 2.44E-02 | 7.44E-03 | Panel VIII |
| 171 | 4926 | NUMA1 | 1.62E-03 | 4.28E-05 | 4.31E-03 | Panel VIII |
| 214 | 7791 | ZYX | 4.54E-04 | 1.77E-05 | 2.57E-03 | Panel VII |
| 368 | 4670 | HNRNPM | 7.61E-03 | 8.98E-03 | 2.34E-05 | Panel VII |
| 369 | 10540 | DCTN2 | 1.11E-06 | 1.69E-08 | 2.49E-05 | Panel VII |
| 370 | 10938 | EHD1 | 5.60E-05 | 1.73E-05 | 1.01E-03 | Panel VII |
| 372 | 684 | BST2 | 2.51E-02 | 1.44E-02 | 1.13E-08 | Panel VIII |
| 373 | 1058 | CENPA | 4.45E-04 | 8.70E-06 | 2.51E-05 | Panel VIII |
| 375 | 3092 | HIP1 | 4.42E-02 | 8.73E-07 | 2.03E-04 | Panel VIII |
| 376 | 3336 | HSPE1 | 2.15E-02 | 1.92E-02 | 1.73E-03 | Panel VIII |
| 377 | 5455 | POU3F3 | 1.31E-03 | 1.02E-04 | 6.18E-03 | Panel VIII |
| 380 | 6626 | SNRPA | 7.16E-12 | 1.85E-11 | 2.60E-16 | Panel VIII |
| 381 | 6631 | SNRPC | 1.42E-06 | 1.06E-07 | 3.73E-15 | Panel VIII |
| 382 | 6757 | SSX2 | 2.40E-03 | 1.78E-02 | 2.09E-04 | Panel VIII |
| 384 | 10134 | BCAP31 | 4.10E-02 | 1.04E-05 | 4.34E-08 | Panel VIII |
| 390 | 55727 | BTBD7 | 3.33E-02 | 3.68E-04 | 1.79E-04 | Panel VIII |
| 428 | 4000 | LMNA | 1.59E-03 | 7.49E-04 | 3.41E-04 | Panel VIII |
| 429 | 4582 | MUC1 | 1.35E-04 | 1.66E-05 | 6.29E-04 | Panel VIII |
| 431 | 5340 | PLG | 1.22E-03 | 1.64E-02 | 5.61E-03 | Panel VIII |
| 432 | 6525 | SMTN | 2.05E-03 | 1.53E-02 | 9.78E-03 | Panel VIII |
| 452 | dsDNA | dsDNA | 1.65E-06 | 5.35E-17 | NA | dsDNA |

### Beispiel: Berechnung von Biomarker-Panel

Wie in Tabelle 7 gezeigt, weisen nur bis zu maximal ca. 60% der SLE Patienten Autoantikörper für ein spezifisches Autoantigen auf. Um daher die Sensitivität der diagnostischen Autoantiköper, wie z.B. anti-dsDNA, SSA-Ro (TRIM21/TROVE2) und U1-RNP (SNRNP70, SNRPNA, SNRNPC) zu erhöhen, wurden neue Verfahren mit denen sich Autoantikörper zu sogenannten Biomarkerpanels kombinieren lassen getestet.

Für diesen Pool ausgewählter Kandidaten wurde für die Panels PI bis PVII eine logistische Regression durchgeführt. Für die Panels PVIII bis PXI wurde im Rahmen einer genesteten Kreuzvalidierung ein L1-penalisiertes logistisches Regressionsmodell aufgestellt. Antigene, die im Rahmen der Modellbildung nicht berücksichtigt wurden, wurden aus der weiteren Betrachtung entfernt. Innerhalb des verbleibenden Pools wurden Panels inhaltlich definiert, zum Beispiel nach etablierten Markern und neuen Markern.

Die in Tabelle 2 genannten Antigene wurden für die Berechnung von Biomarker-Panels zur Diagnose von SLE verwendet.

Tabelle 4 zeigt unterschiedliche Kombinationen von Antigenen, die für die Berechnung der Biomarker Panels (ENA-4, ENA-4 + anti-Rib, PI, PII, PIII, PVI, PV) verwendet wurden.

Tabelle 8 zeigt weitere unterschiedliche Kombinationen von Antigenen, die für die Berechnung von Panels verwendet wurden und aufgrund ihrer Signifikanz und Reaktivität in drei SLE Kohorten ausgewählt wurden.

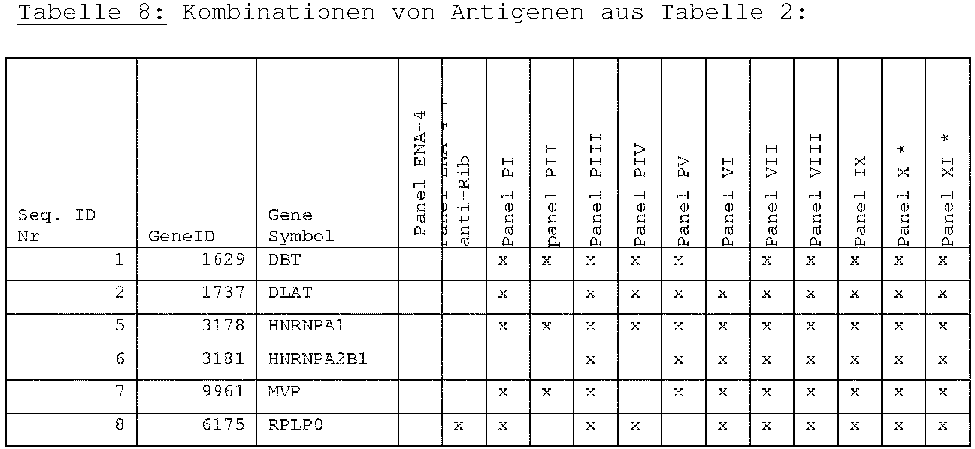

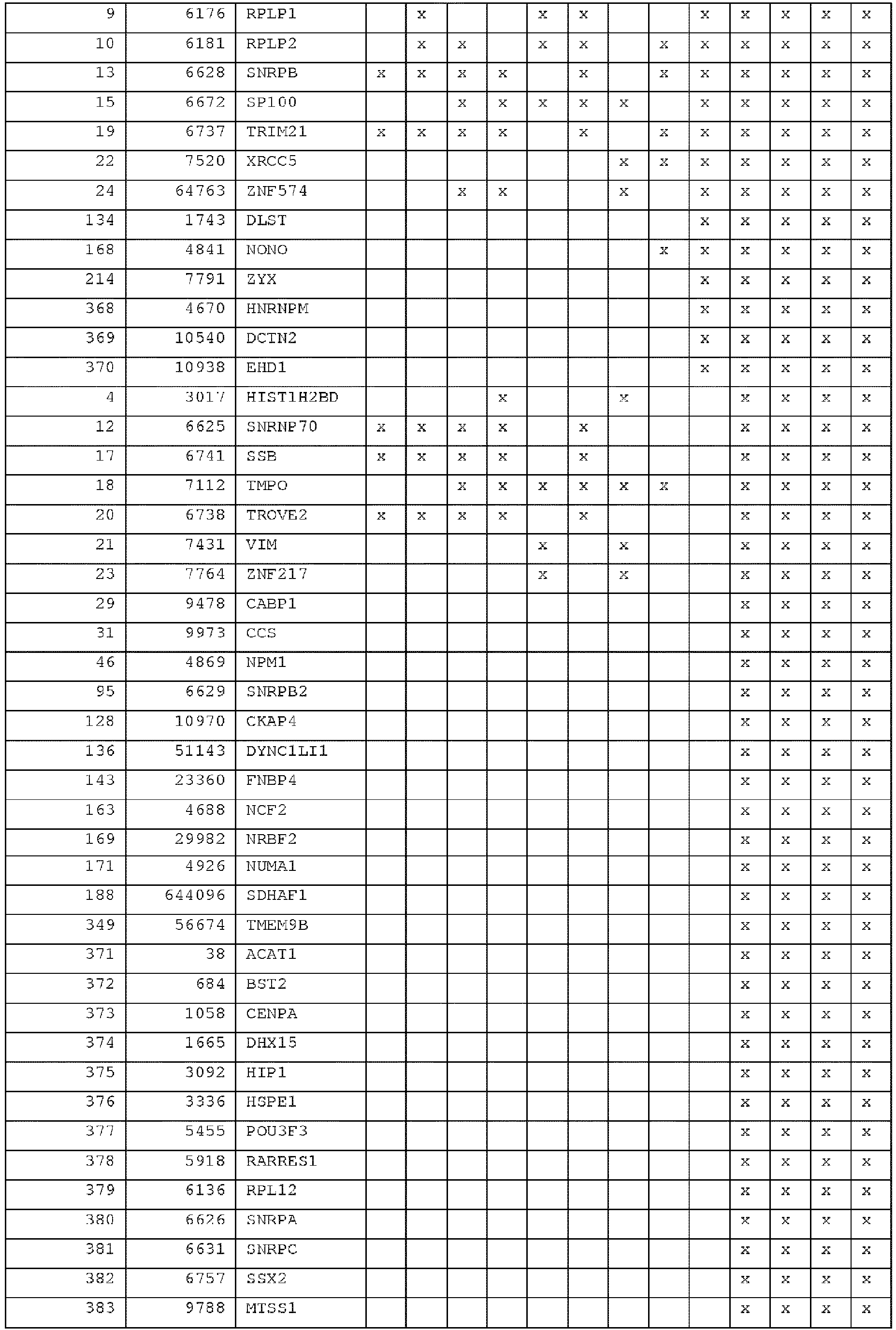

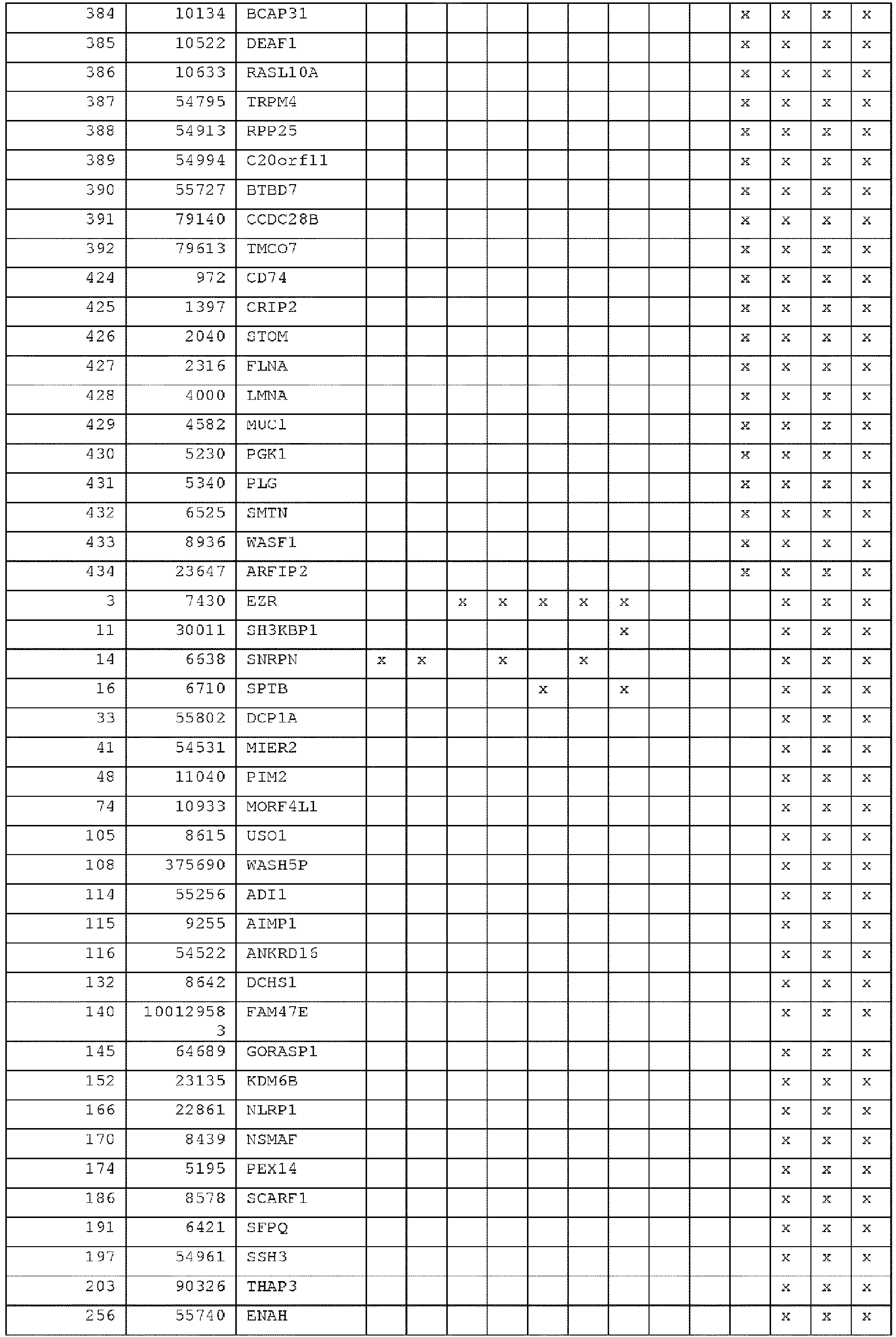

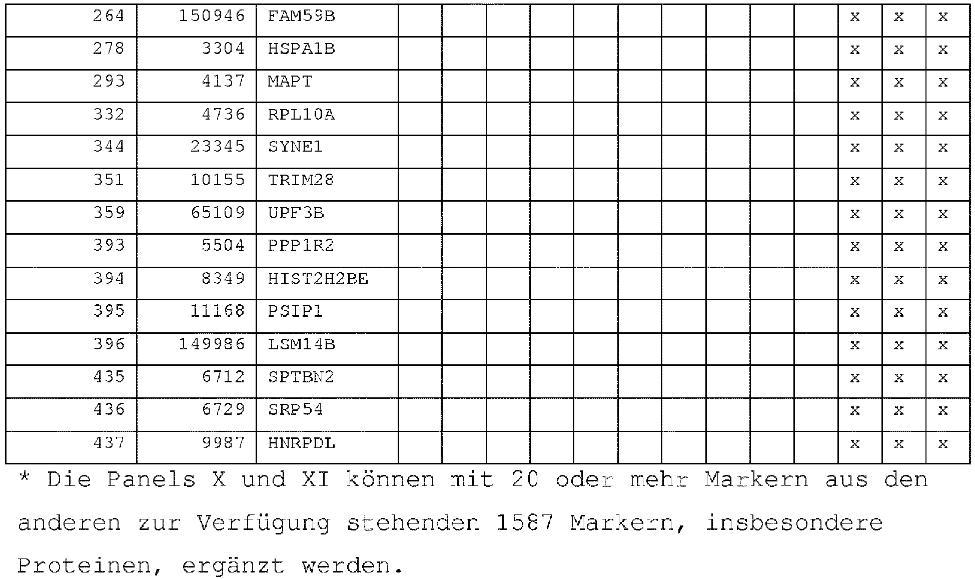

Panel VI umfasst 11 Antigene, die allen drei SLE Kohorten mit einem p-Wert <0.05 gemessen wurden.

Panel VII umfasst 19 Antigene, die in den drei SLE Kohorten mit einem p-Wert <0.05 gemessen wurden.

Panel VIII umfasst Panel VII und weitere 52 Antigene, die in Kohorte 3 und mindestens einer der anderen SLE Kohorten mit einem p-Wert <0.05 für den Vergleich SLE gegen Gesundkontrollen gefunden wurden.

Panel IX umfasst Panel VII, Panel VIII und weitere 110 Antigene, die in einer oder zwei SLE Kohorten für den Vergleich SLE gegen Gesundkontrollen einen p-Wert von 0.05 erreichten.

Panel X umfasst Panel VII, Panel VIII, Panel IX und weitere 227 Antigene, die wie in Tabelle 2 angegeben aus verschiedenen Vergleichen stammen und dort einen p-Wert <0.05 in mindestens einer SLE Kohorte erreichten.

Tabelle 9a, 9c, 9e zeigen die Fläche unterhalb der Kurve (AUC), Konfidenzintervalle, Sensitivität und Spezifität verschiedener Biomarkerkombinationen in den drei verschiedenen SLE Kohorten.

Tabelle 9b, 9d zeigen die Fläche unterhalb der Kurve (AUC), Konfidenzintervalle, Sensitivität und Spezifität der verschiedenen Panels in der drei SLE Kohorten in Kombination mit anti-dsDNA Autoantikörpern.

**Tabelle 9a: Fläche unterhalb der Kurve (AUC), Sensitivität und Spezifität der verschiedenen Panels in der SLE Kohorte I.**

| Kohorte I | AUC | | | Sensitivität | | | Spezifität | | |
|---|---|---|---|---|---|---|---|---|---|
| Panel | Mittelere | Unteres CI | Oberes CI | Mittelere | Unteres CI | Oberes CI | Mittel ere | Unteres CI | Oberes CI |
| PI | 0.86 | 0.84 | 0.87 | 0.79 | 0.76 | 0.81 | 0.84 | 0.82 | 0.86 |
| PII | 0.88 | 0.87 | 0.90 | 0.81 | 0.79 | 0.84 | 0.82 | 0.80 | 0.85 |
| PIII | 0.84 | 0.83 | 0.86 | 0.74 | 0.71 | 0.76 | 0.80 | 0.77 | 0.83 |
| PIV | 0.85 | 0.84 | 0.87 | 0.80 | 0.77 | 0.82 | 0.83 | 0.81 | 0.85 |
| PV | 0.81 | 0.79 | 0.83 | 0.73 | 0.70 | 0.75 | 0.76 | 0.73 | 0.79 |
| PVI | 0.87 | 0.86 | 0.89 | 0.79 | 0.77 | 0.82 | 0.83 | 0.81 | 0.85 |
| Panel.ENA | 0.87 | 0.85 | 0.88 | 0.73 | 0.71 | 0.76 | 0.86 | 0.84 | 0.89 |
| Panel.ENA + antiRib | 0.87 | 0.85 | 0.88 | 0.78 | 0.75 | 0.80 | 0.83 | 0.81 | 0.85 |
| PVII | 0.79 | 0.77 | 0.81 | 0.75 | 0.72 | 0.78 | 0.77 | 0.74 | 0.79 |
| PVIII | 0.85 | 0.83 | 0.86 | 0.75 | 0.72 | 0.78 | 0.82 | 0.79 | 0.84 |
| PIX | 0.83 | 0.81 | 0.84 | 0.73 | 0.71 | 0.76 | 0.81 | 0.78 | 0.83 |
| PX | 0.83 | 0.81 | 0.84 | 0.73 | 0.70 | 0.76 | 0.78 | 0.76 | 0.81 |
| PXI | 0.83 | 0.81 | 0.84 | 0.74 | 0.71 | 0.76 | 0.79 | 0.76 | 0.81 |

**Tabelle 9b: Fläche unterhalb der Kurve (AUC), oberes und unteres Konfidenzintervall (CI), Sensitivität und Spezifität der Biomarker Panels in SLE Kohorte I in Kombination mit anti-dsDNA Autoantikörpern.**

| Kohorte I | AUC | | | Sensitivität | | | Spezifität | | |
|---|---|---|---|---|---|---|---|---|---|
| Panel plus dsDNA | Mitte lere | Unter es CI | Obere s CI | Mitte lere | Unteres CI | Obere s CI | Mittel ere | Untere s CI | Oberes CI |
| PI | 0.86 | 0.84 | 0.87 | 0.79 | 0.77 | 0.81 | 0.83 | 0.81 | 0.85 |
| PII | 0.87 | 0.85 | 0.88 | 0.80 | 0.77 | 0.82 | 0.81 | 0.79 | 0.83 |
| PIII | 0.83 | 0.81 | 0.85 | 0.74 | 0.71 | 0.77 | 0.78 | 0.76 | 0.81 |
| PIV | 0.86 | 0.84 | 0.87 | 0.79 | 0.76 | 0.82 | 0.83 | 0.81 | 0.85 |
| PV | 0.80 | 0.78 | 0.82 | 0.72 | 0.70 | 0.75 | 0.76 | 0.73 | 0.78 |
| PVI | 0.86 | 0.85 | 0.88 | 0.79 | 0.77 | 0.82 | 0.82 | 0.80 | 0.85 |
| Panel.ENA | 0.86 | 0.85 | 0.88 | 0.73 | 0.71 | 0.76 | 0.86 | 0.84 | 0.89 |
| Panel.ENA + antiRib | 0.86 | 0.85 | 0.88 | 0.76 | 0.74 | 0.78 | 0.83 | 0.80 | 0.85 |
| PVII | 0.79 | 0.77 | 0.81 | 0.74 | 0.71 | 0.77 | 0.76 | 0.73 | 0.78 |
| PVIII | 0.89 | 0.88 | 0.90 | 0.80 | 0.78 | 0.83 | 0.85 | 0.83 | 0.87 |
| PIX | 0.90 | 0.89 | 0.92 | 0.81 | 0.79 | 0.83 | 0.85 | 0.83 | 0.87 |
| PX | 0.84 | 0.82 | 0.86 | 0.73 | 0.70 | 0.75 | 0.81 | 0.79 | 0.84 |
| PXI | 0.89 | 0.88 | 0.90 | 0.81 | 0.78 | 0.83 | 0.84 | 0.82 | 0.86 |

**Tabelle 9c: Fläche unterhalb der Kurve (AUC), zeigt die Sensitivität und Spezifität der verschiedenen Panels in der SLE Kohorte II.**

| Kohorte II | AUC | | | Sensitivity | | | Specificity | | |
|---|---|---|---|---|---|---|---|---|---|
| Panel | Mean | CI lower | CI upper | Mean | CI lower | CI upper | Mean | CI lower | CI upper |
| PI | 0.84 | 0.83 | 0.86 | 0.74 | 0.72 | 0.76 | 0.79 | 0.77 | 0.81 |
| PII | 0.78 | 0.77 | 0.80 | 0.68 | 0.65 | 0.70 | 0.72 | 0.70 | 0.75 |
| PIII | 0.83 | 0.81 | 0.84 | 0.73 | 0.70 | 0.75 | 0.78 | 0.76 | 0.81 |
| PIV | 0.86 | 0.84 | 0.87 | 0.76 | 0.74 | 0.78 | 0.81 | 0.79 | 0.84 |
| PV | 0.77 | 0.75 | 0.78 | 0.67 | 0.65 | 0.69 | 0.74 | 0.72 | 0.76 |
| PVI | 0.87 | 0.86 | 0.88 | 0.77 | 0.74 | 0.79 | 0.82 | 0.80 | 0.84 |
| Panel.ENA | 0.76 | 0.74 | 0.77 | 0.59 | 0.56 | 0.61 | 0.78 | 0.75 | 0.80 |
| Panel.ENA + antiRib | 0.84 | 0.83 | 0.86 | 0.73 | 0.71 | 0.76 | 0.83 | 0.82 | 0.85 |
| PVII | 0.84 | 0.82 | 0.86 | 0.76 | 0.74 | 0.78 | 0.80 | 0.77 | 0.82 |
| PVIII | 0.85 | 0.83 | 0.86 | 0.76 | 0.74 | 0.78 | 0.80 | 0.78 | 0.82 |
| PIX | 0.84 | 0.83 | 0.86 | 0.76 | 0.74 | 0.78 | 0.79 | 0.77 | 0.81 |
| PX | 0.83 | 0.81 | 0.85 | 0.76 | 0.73 | 0.78 | 0.78 | 0.76 | 0.80 |
| PXI | 0.82 | 0.81 | 0.84 | 0.74 | 0.71 | 0.76 | 0.79 | 0.76 | 0.81 |

**Tabelle 9d: Fläche unterhalb der Kurve (AUC), Sensitivität und Spezifität der Biomarker Panels in SLE Kohorte II in Kombination mit anti-dsDNA Autoantikörpern.**

| Kohorte II | AUC | | | Sensitivität | | | Spezifität | | |
|---|---|---|---|---|---|---|---|---|---|
| Panel plus dsDNA | Mittelere | Unteres CI | Oberes CI | Mittelere | Unteres CI | Oberes CI | Mittelere | Unteres CI | Oberes CI |
| PI | 0.84 | 0.82 | 0.85 | 0.73 | 0.71 | 0.76 | 0.78 | 0.76 | 0.81 |
| PII | 0.78 | 0.76 | 0.80 | 0.67 | 0.65 | 0.70 | 0.73 | 0.70 | 0.75 |
| PIII | 0.82 | 0.81 | 0.84 | 0.73 | 0.71 | 0.75 | 0.76 | 0.74 | 0.79 |
| PIV | 0.85 | 0.84 | 0.87 | 0.76 | 0.73 | 0.78 | 0.80 | 0.78 | 0.83 |
| PV | 0.77 | 0.75 | 0.78 | 0.67 | 0.65 | 0.69 | 0.71 | 0.69 | 0.74 |
| PVI | 0.87 | 0.85 | 0.88 | 0.77 | 0.75 | 0.79 | 0.82 | 0.80 | 0.84 |
| Panel.ENA | 0.77 | 0.76 | 0.79 | 0.60 | 0.58 | 0.63 | 0.77 | 0.75 | 0.80 |
| Panel.ENA + antiRib | 0.85 | 0.84 | 0.86 | 0.73 | 0.71 | 0.76 | 0.83 | 0.81 | 0.85 |
| PVII | 0.84 | 0.82 | 0.85 | 0.75 | 0.73 | 0.78 | 0.79 | 0.77 | 0.82 |
| PVIII | 0.85 | 0.83 | 0.86 | 0.72 | 0.70 | 0.75 | 0.83 | 0.80 | 0.85 |
| PIX | 0.78 | 0.77 | 0.80 | 0.64 | 0.62 | 0.67 | 0.78 | 0.75 | 0.80 |
| PX | 0.84 | 0.83 | 0.85 | 0.72 | 0.70 | 0.75 | 0.83 | 0.81 | 0.85 |
| PXI | 0.87 | 0.85 | 0.88 | 0.75 | 0.73 | 0.77 | 0.84 | 0.82 | 0.86 |

**Tabelle 9e: Fläche unterhalb der Kurve (AUC), Sensitivität und Spezifität der verschiedenen Panels in der SLE Kohorte III.**

| Kohorte III | AUC | | | Sensitivität | | | Spezifität | | |
|---|---|---|---|---|---|---|---|---|---|
| Panel | Mittelere | Unteres CI | Oberes CI | Mittelere | Unteres CI | Oberes CI | Mittelere | Unteres CI | Oberes CI |
| PI | 0.83 | 0.82 | 0.84 | 0.71 | 0.69 | 0.73 | 0.80 | 0.78 | 0.81 |
| PII | 0.82 | 0.81 | 0.84 | 0.71 | 0.69 | 0.72 | 0.80 | 0.79 | 0.81 |
| PIII | 0.79 | 0.78 | 0.80 | 0.65 | 0.63 | 0.67 | 0.76 | 0.74 | 0.78 |
| PIV | 0.82 | 0.81 | 0.83 | 0.71 | 0.69 | 0.73 | 0.79 | 0.78 | 0.81 |
| PV | 0.77 | 0.76 | 0.78 | 0.66 | 0.64 | 0.67 | 0.76 | 0.74 | 0.78 |
| PVI | 0.84 | 0.83 | 0.85 | 0.71 | 0.70 | 0.73 | 0.81 | 0.79 | 0.82 |
| Panel.ENA | 0.78 | 0.77 | 0.80 | 0.65 | 0.63 | 0.67 | 0.82 | 0.81 | 0.84 |
| Panel.ENA + antiRib | 0.79 | 0.78 | 0.80 | 0.67 | 0.66 | 0.69 | 0.83 | 0.82 | 0.85 |
| PVII | 0.83 | 0.82 | 0.84 | 0.72 | 0.71 | 0.74 | 0.79 | 0.77 | 0.81 |
| PVIII | 0.83 | 0.82 | 0.84 | 0.73 | 0.72 | 0.75 | 0.77 | 0.76 | 0.79 |
| PIX | 0.81 | 0.79 | 0.82 | 0.72 | 0.70 | 0.74 | 0.76 | 0.74 | 0.77 |
| PX | 0.79 | 0.78 | 0.81 | 0.73 | 0.71 | 0.75 | 0.76 | 0.74 | 0.78 |
| PXI | 0.78 | 0.77 | 0.80 | 0.70 | 0.69 | 0.72 | 0.75 | 0.73 | 0.77 |

*Figur 11*: Die Figur zeigt den Vergleich der berechneten p-Werte (p-value) und Autoantikörperhäufigkeiten (% positive classified observations) für die Antigene aus Tabelle 2 in den drei SLE Kohorten. Die Antigene sind als Kreise mit der laufenden Nummer dargestellt. Die waagerechte Linie markiert den Schwellenwert von p<0.05 für den Vergleich SLE gegen Gesundkontrollen.

### Literatur:

Li PH, Wong WH, Lee TL, Lau CS, Chan TM, Leung AM, Tong KL, Tse NK, Mok CC, Wong SN, Lee KW, Ho MH, Lee PP, Chong CY, Wong RW, Mok MY, Ying SK, Fung SK, Lai WM, Yang W, Lau YL. Relationship between autoantibody clustering and clinical subsets in SLE: cluster and association analyses in Hong Kong Chinese. Rheumatology (Oxford). 2013 Feb;52(2):337-45. doi: 10.1093/rheumatology/kes261.Epub 2012 Oct 4. PubMed PMID: 23038697.
Liu CC, Kao AH, Manzi S, Ahearn JM. Biomarkers in systemic lupus erythematosus: challenges and prospects for the future. Ther Adv Musculoskelet Dis. 2013 Aug;5(4):210-33.
Ching KH, Burbelo PD, Tipton C, Wei C, Petri M, Sanz I, Iadarola MJ. Two major autoantibody clusters in systemic lupus erythematosus. PLoS One. 2012;7(2):e32001. doi: 10.1371/journal.pone.0032001. Epub 2012 Feb 21. PubMed PMID: 22363785; PubMed Central PMCID: PMC3283706.
Stohl W. Future prospects in biologic therapy for systemic lupus erythematosus. Nat Rev Rheumatol. 2013 Sep 10. doi: 10.1038/nrrheum.2013.136. [Epub ahead of print] PubMed PMID: 24018550.
Thanou A, Merrill JT. Treatment of systemic lupus erythematosus: new therapeutic avenues and blind alleys. Nat Rev Rheumatol. 2013 Oct 8. doi:10.1038/nrrheum.2013.145. [Epub ahead of print] PubMed PMID: 24100460.
Sherer Y, Gorstein A, Fritzler MJ, Shoenfeld Y. Autoantibody explosion in systemic lupus erythematosus: more than 100 different antibodies found in SLE patients. Semin Arthritis Rheum. 2004 Oct;34(2):501-37. Review. PubMed PMID: 15505768.

Weitere Aspekte und Ausführungsformen der Erfindung sind in den folgenden Klauseln offenbahrt:
1. Verfahren zur Identifizierung von Markern für Systemischer Lupus erythematodes (SLE) umfassend die Schritte
   a) Serumproben von SLE Patienten werden mit mehr als 5000 an Beads gekoppelten Antigenen in Kontakt gebracht, die Bindung der einzelnen Antigene an Proteine, Autoantikörper im Serum der SLE Patienten durch Immunfluoreszenzassay gemessen und die mittlere Fluoreszenzintensität (MFI = median fluorescence intensity) für jedes einzelne Antigen bestimmt;
   b) Serumproben von Patienten mit rheumatoider Arthritis (RA) werden mit denselben an Beads gekoppelten Antigenen in Kontakt gebracht, die Bindung der einzelnen Antigene an Proteine, Autoantikörper im Serum der RA Patienten durch Immunfluoreszenzassay gemessen und daraus die mittlere Fluoreszenzintensität (MFI) für jedes einzelne Antigen bestimmt;
   c) Serumproben von Gesunden werden mit denselben an Beads gekoppelten Antigenen in Kontakt gebracht, die Bindung der einzelnen Antigene an Proteine, Autoantikörper im Serum von Gesunden durch Immunfluoreszenzassay gemessen und daraus die mittlere Fluoreszenzintensität (MFI) für jedes einzelne Antigen bestimmt;
   d) die MFI Daten aus a), b) und c) werden statistisch mittels univariater Analyse ausgewertet und dadurch Markerkandidat Antigene identifiziert, mit denen SLE Patienten von RA Patienten und von Gesunden differenziert werden können;
   e) Serumproben von Patienten mit früher RA werden mit den an Beads gekoppelten Markerkandidat Antigenen, die in d) identifiziert wurden, in Kontakt gebracht, die Bindung von Markerkandidat Antigenen an Proteine, Autoantikörper im Serum von Patienten mit früher RA durch Immunfluoreszenzassay gemessen und daraus die mittlere Fluoreszenzintensität (MFI) für jedes Markerkandidat Antigen bestimmt;
   f) Serumproben von SSc Patienten (SSc = Systemische Sklerodermie) werden mit den an Beads gekoppelten Markerkandidat Antigenen, die in d) identifiziert wurden, in Kontakt gebracht, die Bindung von Markerkandidat Antigenen an Proteine, Autoantikörper im Serum von SSc Patienten durch Immunfluoreszenzassay gemessen und daraus die mittlere Fluoreszenzintensität (MFI) für jedes Markerkandidat Antigen bestimmt;
   g) Serumproben von SPA Patienten (SPA = Spondylitis ankylosans oder Morbus Bechterew) werden mit den an Beads gekoppelten Markerkandidat Antigenen, die in d) identifiziert wurden, in Kontakt gebracht, die Bindung von Markerkandidat Antigenen an Proteine, Autoantikörper im Serum von SPA Patienten durch Immunfluoreszenzassay gemessen und daraus die mittlere Fluoreszenzintensität (MFI) für jedes Markerkandidat Antigen bestimmt;
   h) die MFI Daten aus e), f) und g) werden statistisch mittels univariater Analyse ausgewertet und wenn ein Schwellenwert von 3 Standardabweichungen über dem Mittelwert der Gesundproben nicht erreicht wird, wird dadurch ein spezifischer Marker für SLE identifiziert, wobei die Marker ausgewählt werden aus den Sequenzen SEQ ID No. 1 bis 1587, Homologen der Sequenzen SEQ ID No. 1 bis 1587 mit mindestens 95 % Homologie, Teilsequenzen von SEQ ID No. 1 bis 1587, Teilsequenzen der Homologen von SEQ ID No. 1 bis 1587 mit mindestens 95 % Homologie, den durch die Sequenzen SEQ ID No. 1 bis 1058 kodierten Proteinen, den durch Homologe von SEQ ID No. 1 bis 1058 kodierten Proteinen, den durch Teilsequenzen von SEQ ID No 1 bis 1058 kodierten Proteinen, den durch Homologe von Teilsequenzen von SEQ ID No. 1 bis 1058 kodierten Proteinen.
2. Verfahren nach Klausel 1 zur Identifizierung von Markern für SLE umfassend die Auswahl von Markern für SLE, die in der univariate Analyse einen adjustierten p-Wert für den nichtparametrischen Mittelwertvergleich zwischen den Gruppen von kleiner 0,05 aufweisen, gleichzeitig einen Fold Change von größer 1,5 und eine aus der ROC-Analyse resultierende AUC von größer 0,75 haben und wobei die Marker für SLE ausgewählt werden aus den Sequenzen SEQ ID No. 1 bis 24, 134, 168, 214, 368 bis 370 SEQ ID No. 530 bis 553, 663, 697, 743, 897 bis 899 und SEQ ID No. 1059 bis 1082, 1192, 1226, 1272, 1426 bis 1428, Homologen von SEQ ID No. 1 bis 24, 134, 168, 214, 368 bis 370 SEQ ID No. 530 bis 553, 663, 697, 743, 897 bis 899 und SEQ ID No. 1059 bis 1082, 1192, 1226, 1272, 1426 bis 1428 mit mindestens 95 % Homologie, Teilsequenzen von SEQ ID No. 1 bis 24, 134, 168, 214, 368 bis 370 SEQ ID No. 530 bis 553, 663, 697, 743, 897 bis 899 und SEQ ID No. 1059 bis 1082, 1192, 1226, 1272, 1426 bis 1428 und Teilsequenzen der Homologen von SEQ ID No. 1 bis 24, 134, 168, 214, 368 bis 370 SEQ ID No. 530 bis 553, 663, 697, 743, 897 bis 899 und SEQ ID No. 1059 bis 1082, 1192, 1226, 1272, 1426 bis 1428 mit mindestens 95 % Homologie und den durch die Sequenzen kodierten Proteinen.
3. Verfahren nach Klausel 1 zur Identifizierung von Markern für die Subgruppe der SLE Patienten mit der Komplikation Lupus Nephritis umfassend den Vergleich der Autoantikörperprofile von SLE Patienten mit Lupus Nephritis mit denen von SLE Patienten ohne Lupus Nephritis und wobei die Marker für die Subgruppe der SLE Patienten mit der Komplikation Lupus Nephritis ausgewählt werden aus den Sequenzen SEQ ID No. 25 bis 54, 215, 216, 217, 218, 228, 233, 241, 245, 247, 249, 258, 288, 289, 301, 309, 315, 316, 324, 330, 331, 337, 339, 348, 350, 359, 362, 363, SEQ ID No. 554 bis 583, 744, 745, 746, 747, 757, 762, 770, 774, 776, 778, 787, 817, 818, 830, 838, 844, 845, 853, 859, 860, 866, 868, 877, 879, 888, 891, 892 und SEQ ID No. 1083 bis 1112, 1273, 1274, 1275, 1276, 1286, 1291, 1299, 1303, 1305, 1307, 1316, 1346, 1347, 1359, 1367, 1373, 1374, 1382, 1388, 1389, 1395, 1397, 1406, 1408, 1417, 1420, 1421, Homologen von SEQ ID No. 2 SEQ ID No. 25 bis 54, 215, 216, 217, 218, 228, 233, 241, 245, 247, 249, 258, 288, 289, 301, 309, 315, 316, 324, 330, 331, 337, 339, 348, 350, 359, 362, 363, SEQ ID No. 554 bis 583, 744, 745, 746, 747, 757, 762, 770, 774, 776, 778, 787, 817, 818, 830, 838, 844, 845, 853, 859, 860, 866, 868, 877, 879, 888, 891, 892 und SEQ ID No. 1083 bis 1112, 1273, 1274, 1275, 1276, 1286, 1291, 1299, 1303, 1305, 1307, 1316, 1346, 1347, 1359, 1367, 1373, 1374, 1382, 1388, 1389, 1395, 1397, 1406, 1408, 1417, 1420, 1421 mit mindestens 95 % Homologie, Teilsequenzen von SEQ ID No. 25 bis 54, 215, 216, 217, 218, 228, 233, 241, 245, 247, 249, 258, 288, 289, 301, 309, 315, 316, 324, 330, 331, 337, 339, 348, 350, 359, 362, 363, SEQ ID No. 554 bis 583, 744, 745, 746, 747, 757, 762, 770, 774, 776, 778, 787, 817, 818, 830, 838, 844, 845, 853, 859, 860, 866, 868, 877, 879, 888, 891, 892 und SEQ ID No. 1083 bis 1112, 1273, 1274, 1275, 1276, 1286, 1291, 1299, 1303, 1305, 1307, 1316, 1346, 1347, 1359, 1367, 1373, 1374, 1382, 1388, 1389, 1395, 1397, 1406, 1408, 1417, 1420, 1421 und Teilsequenzen der Homologen von SEQ ID No. 25 bis 54, 215, 216, 217, 218, 228, 233, 241, 245, 247, 249, 258, 288, 289, 301, 309, 315, 316, 324, 330, 331, 337, 339, 348, 350, 359, 362, 363, SEQ ID No. 554 bis 583, 744, 745, 746, 747, 757, 762, 770, 774, 776, 778, 787, 817, 818, 830, 838, 844, 845, 853, 859, 860, 866, 868, 877, 879, 888, 891, 892 und SEQ ID No. 1083 bis 1112, 1273, 1274, 1275, 1276, 1286, 1291, 1299, 1303, 1305, 1307, 1316, 1346, 1347, 1359, 1367, 1373, 1374, 1382, 1388, 1389, 1395, 1397, 1406, 1408, 1417, 1420, 1421 mit mindestens 95 % Homologie und den durch die Sequenzen kodierten Proteinen.
4. Verfahren nach einem der Klausel 1 oder 3 umfassend die statistische Auswertung mittels eines L1-Penalisierten logistischen Regressionsmodels mit fünffacher Kreuzvalidierung und zwanzigfacher Wiederholung und Auswahl der Marker, die mit einer Frequenz von 50 % oder mehr auftreten und wobei die Marker für die Subgruppe der SLE Patienten mit der Komplikation Lupus Nephritis ausgewählt werden aus den Sequenzen SEQ ID No. 25 bis 54, SEQ ID No. 554 bis 583 und SEQ ID No. 1083 bis 1112, Homologen von SEQ ID No. 25 bis 54, SEQ ID No. 554 bis 583 und SEQ ID No. 1083 bis 1112 mit mindestens 95 % Homologie, Teilsequenzen von SEQ ID No. 25 bis 54, SEQ ID No. 554 bis 583 und SEQ ID No. 1083 bis 1112 und Teilsequenzen der Homologen von SEQ ID No. 25 bis 54, SEQ ID No. 554 bis 583 und SEQ ID No. 1083 bis 1112 mit mindestens 95 % Homologie und den durch die Sequenzen kodierten Proteinen.
5. Verfahren nach Klausel 1 zur Identifizierung von Markern für definierte Subgruppen von SLE Patienten umfassend Korrelation der Sequenzen SEQ ID No. 1 bis 529 (Klonsequenzen) durch Berechnung des Spearman Rangkorrelationskoeffizienten für den jeweiligen Marker mit einer der Sequenzen SEQ ID No. 1 bis 529 und wobei die Marker, die eine Korrelation der Reaktivitäten in SLE Patienten miteinander zeigen, ausgewählt werden aus den Sequenzen SEQ ID No. 55 bis 111, SEQ ID No. 584 bis 1007 und SEQ ID No. 1113 bis 1169, Homologen von SEQ ID No. 55 bis 111, SEQ ID No. 584 bis 1007 und SEQ ID No. 1113 bis 1169 mit mindestens 95 % Homologie, Teilsequenzen von SEQ ID No. 55 bis 111, SEQ ID No. 584 bis 1007 und SEQ ID No. 1113 bis 1169 und Teilsequenzen der Homologen von SEQ ID No. 55 bis 111, SEQ ID No. 584 bis 1007 und SEQ ID No. 1113 bis 1169 mit mindestens 95 % Homologie und den durch die Sequenzen kodierten Proteinen.
6. Verfahren nach Klausel 1 zur Identifizierung von Markern für die Subgruppe der ENA-4 negativen SLE Patienten umfassend das Testen der Serumproben von SLE Patienten auf die Abwesenheit von Autoantikörpern gegen die extrahierbaren nukleären Antigene Sm-Protein, U1-RNP, Rho52/SS-A und Ro60/SS-B und wobei die Marker für ENA-4 negative SLE Patienten ausgewählt werden aus den Sequenzen SEQ ID No. 112 bis 214 und 279, SEQ ID No. 641 bis 743 und 808 und SEQ ID No. 1170 bis 1272 und 1337, Homologen von SEQ ID No. 112 bis 214 und 279, SEQ ID No. 641 bis 743 und 808 und SEQ ID No. 1170 bis 1272 und 1337 mit mindestens 95 % Homologie, Teilsequenzen von SEQ ID No. 112 bis 214 und 279, SEQ ID No. 641 bis 743 und 808 und SEQ ID No. 1170 bis 1272 und 1337 und Teilsequenzen der Homologen von SEQ ID No. 112 bis 214 und 279, SEQ ID No. 641 bis 743 und 808 und SEQ ID No. 1170 bis 1272 und 1337 mit mindestens 95 % Homologie und den durch die Sequenzen kodierten Proteinen.
7. Verfahren nach Klausel 1 umfassend die Auswahl von Markern, die einen adjustierten p-Wert für den nichtparametrischen Mittelwertvergleich zwischen Gruppen von kleiner 0,05 aufweisen, gleichzeitig einen Fold Change von größer 1,5 und eine aus der ROC-Analyse resultierende AUC von größer 0,75 haben und wobei die Marker für SLE ausgewählt werden aus den Sequenzen SEQ ID No. 219 bis 227, 229 bis 232, 234 bis 240, 242, 243, 244, 246, 248, 250 bis 257, 259 bis 278, 280 bis 287, 290 bis 300, 302 bis 308, 310 bis 314, 317 bis 323, 325 bis 329, 332 bis 336, 338, 340 bis 347, 349, 351 bis 358, 360, 361, 364 bis 367, SEQ ID No. 748 bis 756, 758 bis 761, 763 bis 769, 771, 772, 773, 775, 777, 779 bis 786, 788 bis 807, 809 bis 816, 819 bis 829, 831 bis 837, 839 bis 843, 846 bis 852, 853 bis 857, 861 bis 865, 867, 869 bis 876, 878, 880 bis 887, 889, 890, 893 bis 896, SEQ ID No. 1277 bis 1285, 1287 bis 1290, 1292 bis 1298, 1300, 1301, 1302, 1304, 1306, 1308 bis 1315, 1317 bis 1336, 1338 bis 1345, 1348 bis 1358, 1360 bis 1366, 1368 bis 1372, 1375 bis 1381, 1382 bis 1386, 1390 bis 1394, 1396, 1398 bis 1405, 1407, 1409 bis 1416, 1418, 1420, 1422 bis 1425, Homologen von SEQ ID No. 219 bis 227, 229 bis 232, 234 bis 240, 242, 243, 244, 246, 248, 250 bis 257, 259 bis 278, 280 bis 287, 290 bis 300, 302 bis 308, 310 bis 314, 317 bis 323, 325 bis 329, 332 bis 336, 338, 340 bis 347, 349, 351 bis 358, 360, 361, 364 bis 367, SEQ ID No. 748 bis 756, 758 bis 761, 763 bis 769, 771, 772, 773, 775, 777, 779 bis 786, 788 bis 807, 809 bis 816, 819 bis 829, 831 bis 837, 839 bis 843, 846 bis 852, 853 bis 857, 861 bis 865, 867, 869 bis 876, 878, 880 bis 887, 889, 890, 893 bis 896, SEQ ID No. 1277 bis 1285, 1287 bis 1290, 1292 bis 1298, 1300, 1301, 1302, 1304, 1306, 1308 bis 1315, 1317 bis 1336, 1338 bis 1345, 1348 bis 1358, 1360 bis 1366, 1368 bis 1372, 1375 bis 1381, 1382 bis 1386, 1390 bis 1394, 1396, 1398 bis 1405, 1407, 1409 bis 1416, 1418, 1420, 1422 bis 1425 mit mindestens 95 % Homologie, Teilsequenzen von SEQ ID No. 219 bis 227, 229 bis 232, 234 bis 240, 242, 243, 244, 246, 248, 250 bis 257, 259 bis 278, 280 bis 287, 290 bis 300, 302 bis 308, 310 bis 314, 317 bis 323, 325 bis 329, 332 bis 336, 338, 340 bis 347, 349, 351 bis 358, 360, 361, 364 bis 367, SEQ ID No. 748 bis 756, 758 bis 761, 763 bis 769, 771, 772, 773, 775, 777, 779 bis 786, 788 bis 807, 809 bis 816, 819 bis 829, 831 bis 837, 839 bis 843, 846 bis 852, 853 bis 857, 861 bis 865, 867, 869 bis 876, 878, 880 bis 887, 889, 890, 893 bis 896, SEQ ID No. 1277 bis 1285, 1287 bis 1290, 1292 bis 1298, 1300, 1301, 1302, 1304, 1306, 1308 bis 1315, 1317 bis 1336, 1338 bis 1345, 1348 bis 1358, 1360 bis 1366, 1368 bis 1372, 1375 bis 1381, 1382 bis 1386, 1390 bis 1394, 1396, 1398 bis 1405, 1407, 1409 bis 1416, 1418, 1420, 1422 bis 1425 und Teilsequenzen der Homologen von SEQ ID No. 219 bis 227, 229 bis 232, 234 bis 240, 242, 243, 244, 246, 248, 250 bis 257, 259 bis 278, 280 bis 287, 290 bis 300, 302 bis 308, 310 bis 314, 317 bis 323, 325 bis 329, 332 bis 336, 338, 340 bis 347, 349, 351 bis 358, 360, 361, 364 bis 367, SEQ ID No. 748 bis 756, 758 bis 761, 763 bis 769, 771, 772, 773, 775, 777, 779 bis 786, 788 bis 807, 809 bis 816, 819 bis 829, 831 bis 837, 839 bis 843, 846 bis 852, 853 bis 857, 861 bis 865, 867, 869 bis 876, 878, 880 bis 887, 889, 890, 893 bis 896, SEQ ID No. 1277 bis 1285, 1287 bis 1290, 1292 bis 1298, 1300, 1301, 1302, 1304, 1306, 1308 bis 1315, 1317 bis 1336, 1338 bis 1345, 1348 bis 1358, 1360 bis 1366, 1368 bis 1372, 1375 bis 1381, 1382 bis 1386, 1390 bis 1394, 1396, 1398 bis 1405, 1407, 1409 bis 1416, 1418, 1420, 1422 bis 1425 mit mindestens 95 % Homologie und den durch die Sequenzen kodierten Proteine.
8. Marker für SLE oder SLE Subgruppen erhältlich durch ein Verfahren nach einem der Klausel 1 bis 7.
9. Marker für SLE oder SLE Subgruppen ausgewählt aus den Markern mit den Sequenzen SEQ ID No. 1 bis 1587, Homologen der Sequenzen SEQ ID No. 1 bis 1587 mit mindestens 95 % Homologie, Teilsequenzen von SEQ ID No. 1 bis 1587, Teilsequenzen der Homologen von SEQ ID No. 1 bis 1587 mit mindestens 95 % Homologie, den durch die Sequenzen SEQ ID No. 1 bis 1058 kodierten Proteinen, den durch Homologe von SEQ ID No. 1 bis 1058 kodierten Proteinen, den durch Teilsequenzen von SEQ ID No 1 bis 1058 kodierten Proteinen, den durch Homologe von Teilsequenzen von SEQ ID No. 1 bis 1058 kodierten Proteinen.
10. Panel von Markern für die Diagnose von SLE umfassend mindestens zwei verschiedene Marker unabhängig voneinander ausgewählt aus Markern mit den Sequenzen SEQ ID No. 1 bis 1587, Homologen der Sequenzen SEQ ID No. 1 bis 1587 mit mindestens 95 % Homologie, Teilsequenzen von SEQ ID No. 1 bis 1587, Teilsequenzen der Homologen von SEQ ID No. 1 bis 1587 mit mindestens 95 % Homologie, den durch die Sequenzen SEQ ID No. 1 bis 1058 kodierten Proteinen, den durch Homologe von SEQ ID No. 1 bis 1058 kodierten Proteinen, den durch Teilsequenzen von SEQ ID No 1 bis 1058 kodierten Proteinen, den durch Homologe von Teilsequenzen von SEQ ID No. 1 bis 1058 kodierten Proteinen.
11. Panel von Markern für die Diagnose von SLE nach Klausel 10 umfassend mindestens zwei Marker, fünf Marker oder 10 Marker oder mehr ausgewählt aus
   Panel I (P1)
      SEQ ID No. 1, 2, 3, 5, 7, 8, 10, 12, 13, 15, 17, 18, 19, 20, 24,
      SEQ ID No. 530, 531, 532, 534, 536, 537, 539, 541, 542, 544, 546, 547, 548, 549, 553, vorzugsweise
      SEQ ID No. 1059, 1060, 1061, 1063, 1065, 1066, 1068, 1070, 1071, 1073, 1075, 1076, 1077, 1078, 1082
      oder
   Panel II (P2)
      SEQ ID No. 1, 3, 4, 5, 7, 12, 13, 14, 15, 17, 18, 19, 20, 24
      SEQ ID No. 530, 532, 533, 534, 536, 541, 542, 543, 544, 546, 547, 548, 549, 553, vorzugsweise
      SEQ ID No. 1059, 1061, 1062, 1063, 1065, 1070, 1071, 1072, 1073, 1075, 1076, 1077, 1078, 1082
      oder
   Panel III (P3)
      SEQ ID No. 1, 2, 3, 5, 6, 7, 8, 9, 10, 15, 16, 18, 21, 23
      SEQ ID No. 530, 531, 532, 534, 535, 536, 537, 538, 539, 544, 545, 547, 550, 552, vorzugsweise
      SEQ ID No. 1059, 1060, 1061, 1063, 1064, 1065, 1066, 1067, 1068, 1073, 1074, 1076, 1079, 1081
      oder
   Panel IV (P4)
      SEQ ID No. 1, 2, 3, 4, 5, 8, 9, 10, 12, 13, 14, 15, 17, 19, 20
      SEQ ID No. 530, 531, 532, 533, 534, 537, 538, 539, 541, 542, 543, 544, 546, 548, 549, vorzugsweise
      SEQ ID No. 1059, 1060, 1061, 1062, 1063, 1066, 1067, 1068, 1070, 1071, 1072, 1073, 1075, 1077, 1078
      oder
   Panel V
      SEQ ID No. 1, 2, 3, 4, 5, 6, 7, 11, 15, 16, 18, 21, 22, 23, 24
      SEQ ID No. 530, 531, 532, 533, 534, 535, 536, 540, 544, 545, 547, 550, 551, 552, 553, vorzugsweise
      SEQ ID No. 1059, 1060, 1061, 1062, 1063, 1064, 1065, 1069, 1073, 1074, 1076, 1079, 1080, 1081, 1082
      oder
   Panel VI
      SEQ ID No. 2, 5, 6, 7, 8, 10, 13, 18, 19, 22, 168
      SEQ ID No. 531, 534, 535, 536, 537, 539, 542, 547, 548, 551, 697, vorzugsweise
      SEQ ID No. 1060, 1063, 1064, 1065, 1066, 1068, 1071, 1076, 1077, 1080, 1226
      oder
   Panel VII
      SEQ ID No. 1, 2, 5, 6, 7, 8, 9, 10, 13, 15, 19, 22, 24, 134, 168, 214, 368, 369, 370
      SEQ ID No. 530, 531, 534, 535, 536, 537, 538, 539, 542, 544, 548, 551, 553, 663, 697, 743, 897, 898, 899, vorzugsweise
      SEQ ID No. 1059, 1060, 1063, 1064, 1065, 1066, 1067, 1068, 1071, 1073, 1077, 1080, 1082, 1192, 1226, 1272, 1426, 1427, 1428
      oder
   Panel VIII (P8)
      SEQ ID No. 1, 2, 4, 5, 6, 7, 8, 9, 10, 12, 13, 15, 17, 18, 19, 20, 21, 22, 23, 24, 29, 31, 46, 95, 128, 134, 136, 143, 163, 168, 169, 171, 188, 214, 349, 368, 369, 370, 371-392, 424-434
      SEQ ID No. 530, 531, 533, 534, 535, 536, 537, 538, 539, 541, 542, 544, 546, 547, 548, 549, 550, 551, 552, 553, 558, 560, 575, 624, 657, 663, 665, 692, 697, 698, 700, 717, 743, 878, 897, 898, 899, 900-921, 953-963, vorzugsweise
      SEQ ID No. 1059, 1060, 1062, 1063, 1064, 1065, 1066, 1067, 1068, 1070, 1071, 1073, 1075, 1076, 1077, 1078, 1079, 1080, 1081, 1082, 1087, 1089, 1104, 1153, 1186, 1192, 1194, 1221, 1222, 1226, 1227, 1229, 1246, 1272, 1407, 1426, 1427, 1428, 1429-1450, 1482-1492
      oder
   Panel IX (P9)
      SEQ ID No. 1, 2, 3, 4, 5, 6, 7, 8, 9, 10, 11, 12, 13, 14, 15, 16, 17, 18, 19, 20, 21, 22, 23, 24, 29, 31, 33, 41, 46, 48, 74, 95, 105, 108, 114, 115, 116, 128, 132, 134, 136, 143, 163, 168, 169, 171, 188, 214, 349, 368, 369, 370, 371-392, 424-434
      SEQ ID No. 530, 531, 532, 533, 534, 535, 536, 537, 538, 539, 540, 541, 542, 543, 544, 545, 546, 547, 548, 549, 550, 551, 552, 553, 558, 560, 562, 570, 575, 577, 603, 624, 634, 637, 643, 644, 645, 657, 661, 663, 665, 672, 692, 697, 698, 700, 717, 743, 878, 897, 898, 899, 900-921, 953-963, vorzugsweise
      SEQ ID No. 1059, 1060, 1061, 1062, 1063, 1064, 1065, 1066, 1067, 1068, 1069, 1070, 1071, 1072, 1073, 1074, 1075, 1076, 1077, 1078, 1079, 1080, 1081, 1082, 1087, 1089, 1091, 1099, 1104, 1106, 1132, 1153, 1163, 1166, 1172, 1173, 1174, 1186, 1190, 1192, 1194, 1201, 1221, 1226, 1227, 1229, 1246, 1272, 1407, 1426, 1427, 1428, 1429-1450, 1482-1492,
   einschließlich jeweilger Homologen oder Teilsequenzen.
12. Diagnostikum oder Test Kit umfassend mindestens einen Marker für SLE ausgewählt aus den Sequenzen SEQ ID No. 1 bis 1587, Homologen der Sequenzen SEQ ID No. 1 bis 1587 mit mindestens 95 % Homologie, Teilsequenzen von SEQ ID No. 1 bis 1587, Teilsequenzen der Homologen von SEQ ID No. 1 bis 1587 mit mindestens 95 % Homologie, den durch die Sequenzen SEQ ID No. 1 bis 1058 kodierten Proteinen, den durch Homologe von SEQ ID No. 1 bis 1058 kodierten Proteinen, den durch Teilsequenzen von SEQ ID No 1 bis 1058 kodierten Proteinen, den durch Homologe von Teilsequenzen von SEQ ID No. 1 bis 1058 kodierten Proteinen oder umfassend ein Panel von Markern nach Klausel 10 oder Klausel 11.
13. Verwendung mindestens eines Markers ausgewählt aus den Sequenzen SEQ ID No. 1 bis 1587, Homologen der Sequenzen SEQ ID No. 1 bis 1587 mit mindestens 95 % Homologie, Teilsequenzen von SEQ ID No. 1 bis 1587, Teilsequenzen der Homologen von SEQ ID No. 1 bis 1587 mit mindestens 95 % Homologie, den durch die Sequenzen SEQ ID No. 1 bis 1058 kodierten Proteinen, den durch Homologe von SEQ ID No. 1 bis 1058 kodierten Proteinen, den durch Teilsequenzen von SEQ ID No 1 bis 1058 kodierten Proteinen, den durch Homologe von Teilsequenzen von SEQ ID No. 1 bis 1058 kodierten Proteinen oder mindestens eines Panels von Markern für SLE nach Klausel 10 oder nach Klausel . 11 oder eines Diagnostikums oder Test Kits nach Klausel 12 zur Identifizierung von Subgruppen von SLE Patienten, zur Diagnose von SLE, zur Differentialdiagnose, insbesondere zur Unterscheidung von SLE von anderen rheumatischen Erkrankungen oder anderen Autoimmunerkrankungen, zur Prognose bei SLE, zur Therapiesteuerung bei SLE, zur Wirkstoffauswahl bei SLE, zur Therapieüberwachung bei SLE, zur Nachsorge bei SLE, oder Apherese von SLE Autoantikörpern.
14. Verwendung mindestens eines Markers ausgewählt aus den Sequenzen SEQ ID No. 112 bis 214 und 279, SEQ ID No. 479 bis 581 und 646 und SEQ ID No. 846 bis 948 und 1013, Homologen von SEQ ID No. 112 bis 214 und 279, SEQ ID No. 479 bis 581 und 646 und SEQ ID No. 846 bis 948 und 1013 mit mindestens 95 % Homologie, Teilsequenzen von SEQ ID No. 112 bis 214 und 279, SEQ ID No. 479 bis 581 und 646 und SEQ ID No. 846 bis 948 und 1013 und Teilsequenzen der Homologen von SEQ ID No. 112 bis 214 und 279, SEQ ID No. 479 bis 581 und 646 und SEQ ID No. 846 bis 948 und 1013 mit mindestens 95 % Homologie und den durch die Sequenzen kodierten Proteine zur Diagnose von SLE bei ENA-4 negativen SLE Patienten
   oder Verwendung mindestens eines Markers ausgewählt aus den Sequenzen SEQ ID No. 25 bis 54, SEQ ID No. 392 bis 421 und SEQ ID No. 759 bis 788, Homologen von SEQ ID No. 25 bis 54, SEQ ID No. 392 bis 421 und SEQ ID No. 759 bis 788 mit mindestens 95 % Homologie, Teilsequenzen von SEQ ID No. 25 bis 54, SEQ ID No. 392 bis 421 und SEQ ID No. 759 bis 788 und Teilsequenzen der Homologen von SEQ ID No. 25 bis 54, SEQ ID No. 392 bis 421 und SEQ ID No. 759 bis 788 mit mindestens 95 % Homologie und den durch die Sequenzer kodierten Proteine zur Diagnose von Lupus Nephritis bei SLE Patienten.
15. Verfahren zur Früherkennung, Diagnose, Differentialdiagnose, Prognose, Therapiesteuerung und/oder Nachsorge bei SLE, wobei
   a. mindestens einer der Marker ausgewählt aus den Sequenzen SEQ ID No. 1 bis 1587, den Homologen der Sequenzen SEQ ID No. 1 bis 1587 mit mindestens 95 % Homologie, den Teilsequenzen von SEQ ID No. 1 bis 1587 oder den Teilsequenzen der Homologen von SEQ ID No. 1 bis 1587 mit mindestens 95 % Homologie, den durch die Sequenzen SEQ ID No. 1 bis 1058 kodierten Proteinen, den durch Homologe von SEQ ID No. 1 bis 1058 kodierten Proteinen, den durch Teilsequenzen von SEQ ID No 1 bis 1058 kodierten Proteinen, den durch Homologe von Teilsequenzen von SEQ ID No. 1 bis 1058 kodierten Proteinen, oder umfassend ein Panel von Markern nach Klausel 10 oder Klausel 11,
   b. mit Körperflüssigkeit oder Gewebeauszug eines zu untersuchenden Individuums in Kontakt gebracht wird, und
   c. der Nachweis einer Wechselwirkung der Körperflüssigkeit oder des Gewebeauszugs mit dem oder den Markern aus a. erfolgt.
16. Verfahren zum Screening von Wirkstoffen für SLE, wobei
   a. mindestens einer der Marker ausgewählt aus den Sequenzen SEQ ID No. 1 bis 1587, den Homologen der Sequenzen SEQ ID No. 1 bis 1587 mit mindestens 95 % Homologie, den Teilsequenzen von SEQ ID No. 1 bis 1587 oder den Teilsequenzen der Homologen von SEQ ID No. 1 bis 1587 mit mindestens 95 % Homologie, den durch die Sequenzen SEQ ID No. 1 bis 1058 kodierten Proteinen, den durch Homologe von SEQ ID No. 1 bis 1058 kodierten Proteinen, den durch Teilsequenzen von SEQ ID No 1 bis 1058 kodierten Proteinen, den durch Homologe von Teilsequenzen von SEQ ID No. 1 bis 1058 kodierten Proteinen, oder umfassend ein Panel von Markern nach Klausel 10 oder Klausel 11,
   b. mit einer zu untersuchenden Substanz in Kontakt gebracht wird und
   c. der Nachweis einer Wechselwirkung der zu untersuchenden Substanz mit dem oder den Markern a. erfolgt.

## Patentansprüche

1. Verwendung von Markers bei der ex vivo oder in vitro Differentialdiagnose bei SLE, wobei mindestens 4 der Marker des Panels 1 (P1) mit Körperflüssigkeit oder Gewebeauszug eines zu untersuchenden Individuums in Kontakt gebracht werden und der Nachweis einer Wechselwirkung der Körperflüssigkeit oder des Gewebeauszugs mit den Markern erfolgt, wobei die mindestens 4 Marker aus dem Panel 1 (P1) der Marker HNRNPA1 (SEQ ID NO: 1063; gen SEQ ID NO: 5), MVP (SEQ ID NO: 1065; gen SEQ ID NO: 7), TMPO (SEQ ID NO: 1076; gen SEQ ID NO: 18), ZNF574 (SEQ ID NO: 1082; gen SEQ ID NO: 24), RPLPO (SEQ ID NO: 1066; gen SEQ ID NO: 8), RPLP2 (SEQ ID NO: 1068; gen SEQ ID NO: 10), SNRNP70 (SEQ ID NO: 1070; gen SEQ ID NO: 12), SNRPB (SEQ ID NO: 1071; gen SEQ ID NO: 13), SP100 (SEQ ID NO: 1073; gen SEQ ID NO: 15), SSB (SEQ ID NO: 1075; gen SEQ ID NO: 17), TRIM21 (SEQ ID NO: 1077; gen SEQ ID NO: 19), TROVE2 (SEQ ID NO: 1078; gen SEQ ID NO: 20), DBT (SEQ ID NO: 1059; gen SEQ ID NO: 1), DLAT (SEQ ID NO: 1060; gen SEQ ID NO: 2), und EZR (SEQ ID NO: 1061; gen SEQ ID NO: 3). ausgewahlt sind.

2. Verwendung nach dem Anspruch 1, wobei mindestens 5 oder 10 Marker des Panels 1 (P1) verwendet werden.

3. Verwendung nach dem Anspruch 1, wobei das gesamte Panel 1 verwendet wird.

4. Diagnostikum oder Test Kit umfassend mindestens 4 Marker aus dem Panel 1 (P1) der Marker
HNRNPA1 (SEQ ID NO: 1063; gen SEQ ID NO: 5), MVP (SEQ ID NO: 1065; gen SEQ ID NO: 7), TMPO (SEQ ID NO: 1076; gen SEQ ID NO: 18), ZNF574 (SEQ ID NO: 1082; gen SEQ ID NO: 24), RPLPO (SEQ ID NO: 1066; gen SEQ ID NO: 8), RPLP2 (SEQ ID NO: 1068; gen SEQ ID NO: 10), SNRNP70 (SEQ ID NO: 1070; gen SEQ ID NO: 12), SNRPB (SEQ ID NO: 1071; gen SEQ ID NO: 13), SP100 (SEQ ID NO: 1073; gen SEQ ID NO: 15), SSB (SEQ ID NO: 1075; gen SEQ ID NO: 17), TRIM21 (SEQ ID NO: 1077; gen SEQ ID NO: 19), TROVE2 (SEQ ID NO: 1078; gen SEQ ID NO: 20), DBT (SEQ ID NO: 1059; gen SEQ ID NO: 1), DLAT (SEQ ID NO: 1060; gen SEQ ID NO: 2), und EZR (SEQ ID NO: 1061; gen SEQ ID NO: 3).

5. Diagnostikum oder Test Kit nach dem Anspruch 4, wobei das Diagnostikum oder Test Kit mindestens 5 oder 10 Marker des Panels 1 (P1) umfasst.

6. Diagnostikum oder Test Kit nach dem Anspruch 4, wobei das Diagnostikum oder Test Kit das gesamte Panel 1 umfasst.

7. Ein fester Träger auf dem mindestens 4 Marker aus dem Panel 1 (P1) der Marker
HNRNPA1 (SEQ ID NO: 1063; gen SEQ ID NO: 5), MVP (SEQ ID NO: 1065; gen SEQ ID NO: 7), TMPO (SEQ ID NO: 1076; gen SEQ ID NO: 18), ZNF574 (SEQ ID NO: 1082; gen SEQ ID NO: 24), RPLP0 (SEQ ID NO: 1066; gen SEQ ID NO: 8), RPLP2 (SEQ ID NO: 1068; gen SEQ ID NO: 10), SNRNP70 (SEQ ID NO: 1070; gen SEQ ID NO: 12), SNRPB (SEQ ID NO: 1071; gen SEQ ID NO: 13), SP100 (SEQ ID NO: 1073; gen SEQ ID NO: 15), SSB (SEQ ID NO: 1075; gen SEQ ID NO: 17), TRIM21 (SEQ ID NO: 1077; gen SEQ ID NO: 19), TROVE2 (SEQ ID NO: 1078; gen SEQ ID NO: 20), DBT (SEQ ID NO: 1059; gen SEQ ID NO: 1), DLAT (SEQ ID NO: 1060; gen SEQ ID NO: 2), und EZR (SEQ ID NO: 1061; gen SEQ ID NO: 3) immobilisiert sind.

8. Ein fester Träger nach dem Anspruch 7, wobei mindestens 5 oder 10 Marker des Panels 1 (P1) auf dem Träger immobilisiert sind.

9. Ein fester Träger nach dem Anspruch 7, wobei das gesamte Panel 1 auf dem Träger immobilisiert ist.
